# EUROPEAN PATENT SPECIFICATION

(11) **EP 3 204 018 B1**
(45) Date of publication and mention of the grant of the patent: **25.08.2021**
(21) Application number: 15849252.0
(22) Date of filing: 05.10.2015
(51) Int. Cl.: A61K 47/68, A61K 45/06, A61K 31/4745, A61K 31/704, A61K 31/166, A61K 35/00, A61P 35/00, A61P 35/02, A61P 35/04

(54) **NEOADJUVANT USE OF ANTIBODY-DRUG CONJUGATES**
NEOADJUVANTE VERWENDUNG VON ANTIKÖRPER-WIRKSTOFF-KONJUGATEN
UTILISATION NÉOADJUVANTE DE CONJUGUÉS ANTICORPS-MÉDICAMENTS

(30) Priority: 07.10.2014 US 201462060858 P
(43) Date of publication of application: 16.08.2017
(73) Proprietor: Immunomedics, Inc., Morris Plains, NJ 07950 (US)
(72) Inventor: GOLDENBERG, David M., Morris Plains, NJ 07950 (US)
(74) Representative: Bohmann, Armin K.
(86) International application number: PCT/US2015/054011
(87) International publication number: WO 2016/057398

(56) References cited:
- WO-A1-2012/012454
- WO-A1-2014/124227
- WO-A2-2005/070026
- WO-A2-2008/076278
- US-A1- 2014 099 254
- US-A1- 2014 099 254
- US-A1- 2014 227 180
- Anonymous: "Phase II Study of IMMU-132 Alone or in Combination With Carboplatin in Patients With Triple-Negative Breast Cancer Study NCT02161679 on Date: June 11, 2014 (v1)", ClinicalTrials.gov , 11 June 2014 (2014-06-11), Retrieved from the Internet: URL:https://www.clinicaltrials.gov/ct2/his tory/NCT02161679?V_1=View#StudyPageTop [retrieved on 2018-12-20]

### SEQUENCE LISTING

<110> IMMUNOMEDICS, INC.
<120> NEOADJUVANT USE OF ANTIBODY-DRUG CONJUGATES
<130> IMM350WO1
<140>
   <141>
<150> 62/060,858
   <151> 2014-10-07
<160> 74
<170> PatentIn version 3.5
<210> 1
   <211> 330
   <212> PRT
   <213> Artificial Sequence
<220>
   <223> Description of Artificial Sequence: Synthetic polypeptide
<400> 1
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Ala Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly Lys
                   325                 330
<210> 2
   <211> 330
   <212> PRT
   <213> Artificial Sequence
<220>
   <223> Description of Artificial Sequence: Synthetic polypeptide
<400> 2
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Arg Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Glu Glu
   225                 230                 235                 240
   Met Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
   275 280 285
   Leu Tyr Ser Lys Leu Thr Val Asp Lys Ser Arg Trp Gln Gln Gly Asn
   290 295 300
   Val Phe Ser Cys Ser Val Met His Glu Ala Leu His Asn His Tyr Thr
   305 310 315 320
   Gln Lys Ser Leu Ser Leu Ser Pro Gly Lys
   325 330
<210> 3
   <211> 11
   <212> PRT
   <213> Artificial Sequence
<220>
   <223> Description of Artificial Sequence: Synthetic peptide
<400> 3
   Lys Ala Ser Gln Asp Val Ser Ile Ala Val Ala
   1 5 10
<210> 4
   <211> 7
   <212> PRT
   <213> Artificial Sequence
<220>
   <223> Description of Artificial Sequence: Synthetic peptide
<400> 4
   Ser Ala Ser Tyr Arg Tyr Thr
   1 5
<210> 5
   <211> 9
   <212> PRT
   <213> Artificial Sequence
<220>
   <223> Description of Artificial Sequence: Synthetic peptide
<400> 5
   Gln Gln His Tyr Ile Thr Pro Leu Thr
   1 5
<210> 6
   <211> 5
   <212> PRT
   <213> Artificial Sequence
<220>
   <223> Description of Artificial Sequence: Synthetic peptide
<400> 6
   Asn Tyr Gly Met Asn
   1               5
<210> 7
   <211> 17
   <212> PRT
   <213> Artificial Sequence
<220>
   <223> Description of Artificial Sequence: Synthetic peptide
<400> 7
   Trp Ile Asn Thr Tyr Thr Gly Glu Pro Thr Tyr Thr Asp Asp Phe Lys
   1               5                   10                  15
   Gly
<210> 8
   <211> 12
   <212> PRT
   <213> Artificial Sequence
<220>
   <223> Description of Artificial Sequence: Synthetic peptide
<400> 8
   Gly Gly Phe Gly Ser Ser Tyr Trp Tyr Phe Asp Val
   1               5                   10
<210> 9
   <211> 11
   <212> PRT
   <213> Artificial Sequence
<220>
   <223> Description of Artificial Sequence: Synthetic peptide
<400> 9
   Lys Ala Ser Gln Asp Val Gly Thr Ser Val Ala
   1               5                   10
<210> 10
   <211> 7
   <212> PRT
   <213> Artificial Sequence
<220>
   <223> Description of Artificial Sequence: Synthetic peptide
<400> 10
   Trp Thr Ser Thr Arg His Thr
   1 5
<210> 11
   <211> 8
   <212> PRT
   <213> Artificial Sequence
<220>
   <223> Description of Artificial Sequence: Synthetic peptide
<400> 11
   Gln Gln Tyr Ser Leu Tyr Arg Ser
   1 5
<210> 12
   <211> 5
   <212> PRT
   <213> Artificial Sequence
<220>
   <223> Description of Artificial Sequence: Synthetic peptide
<400> 12
   Thr Tyr Trp Met Ser
   1 5
<210> 13
   <211> 17
   <212> PRT
   <213> Artificial Sequence
<220>
   <223> Description of Artificial Sequence: Synthetic peptide
<400> 13
   Glu Ile His Pro Asp Ser Ser Thr Ile Asn Tyr Ala Pro Ser Leu Lys
   1 5 10 15
   Asp
<210> 14
   <211> 10
   <212> PRT
   <213> Artificial Sequence
<220>
   <223> Description of Artificial Sequence: Synthetic peptide
<400> 14
   Leu Tyr Phe Gly Phe Pro Trp Phe Ala Tyr
   1 5 10
<210> 15
   <211> 16
   <212> PRT
   <213> Artificial Sequence
<220>
   <223> Description of Artificial Sequence: Synthetic peptide
<400> 15
   Arg Ser Ser Gln Ser Ile Val His Ser Asn Gly Asn Thr Tyr Leu Glu
   1 5 10 15
<210> 16
   <211> 7
   <212> PRT
   <213> Artificial Sequence
<220>
   <223> Description of Artificial Sequence: Synthetic peptide
<400> 16
   Lys Val Ser Asn Arg Phe Ser
   1 5
<210> 17
   <211> 9
   <212> PRT
   <213> Artificial Sequence
<220>
   <223> Description of Artificial Sequence: Synthetic peptide
<400> 17
   Phe Gln Gly Ser His Val Pro Pro Thr
   1 5
<210> 18
   <211> 5
   <212> PRT
   <213> Artificial Sequence
<220>
   <223> Description of Artificial Sequence: Synthetic peptide
<400> 18
   Asn Tyr Gly Met Asn
   1 5
<210> 19
   <211> 17
   <212> PRT
   <213> Artificial Sequence
<220>
   <223> Description of Artificial Sequence: Synthetic peptide
<400> 19
   Trp Ile Asn Thr Tyr Thr Gly Glu Pro Thr Tyr Ala Asp Asp Phe Lys
   1               5                   10                  15
   Gly
<210> 20
   <211> 12
   <212> PRT
   <213> Artificial Sequence
<220>
   <223> Description of Artificial Sequence: Synthetic peptide
<400> 20
   Lys Gly Trp Met Asp Phe Asn Ser Ser Leu Asp Tyr
   1               5                   10
<210> 21
   <211> 10
   <212> PRT
   <213> Artificial Sequence
<220>
   <223> Description of Artificial Sequence: Synthetic peptide
<400> 21
   Ser Ala Ser Ser Arg Val Ser Tyr Ile His
   1               5                   10
<210> 22
   <211> 7
   <212> PRT
   <213> Artificial Sequence
<220>
   <223> Description of Artificial Sequence: Synthetic peptide
<400> 22
   Gly Thr Ser Thr Leu Ala Ser
   1               5
<210> 23
   <211> 9
   <212> PRT
   <213> Artificial Sequence
<220>
   <223> Description of Artificial Sequence: Synthetic peptide
<400> 23
   Gln Gln Trp Ser Tyr Asn Pro Pro Thr
   1               5
<210> 24
   <211> 5
   <212> PRT
   <213> Artificial Sequence
<220>
   <223> Description of Artificial Sequence: Synthetic peptide
<400> 24
   Asp Tyr Tyr Met Ser
   1               5
<210> 25
   <211> 19
   <212> PRT
   <213> Artificial Sequence
<220>
   <223> Description of Artificial Sequence: Synthetic peptide
<400> 25
   Phe Ile Ala Asn Lys Ala Asn Gly His Thr Thr Asp Tyr Ser Pro Ser
   1               5                   10                  15
   Val Lys Gly
<210> 26
   <211> 10
   <212> PRT
   <213> Artificial Sequence
<220>
   <223> Description of Artificial Sequence: Synthetic peptide
<400> 26
   Asp Met Gly Ile Arg Trp Asn Phe Asp Val
   1               5                   10
<210> 27
   <211> 16
   <212> PRT
   <213> Artificial Sequence
<220>
   <223> Description of Artificial Sequence: Synthetic peptide
<400> 27
   Arg Ser Ser Gln Ser Leu Val His Arg Asn Gly Asn Thr Tyr Leu His
   1 5 10 15
<210> 28
   <211> 7
   <212> PRT
   <213> Artificial Sequence
<220>
   <223> Description of Artificial Sequence: Synthetic peptide
<400> 28
   Thr Val Ser Asn Arg Phe Ser
   1 5
<210> 29
   <211> 9
   <212> PRT
   <213> Artificial Sequence
<220>
   <223> Description of Artificial Sequence: Synthetic peptide
<400> 29
   Ser Gln Ser Ser His Val Pro Pro Thr
   1 5
<210> 30
   <211> 5
   <212> PRT
   <213> Artificial Sequence
<220>
   <223> Description of Artificial Sequence: Synthetic peptide
<400> 30
   Asn Tyr Gly Val Asn
   1 5
<210> 31
   <211> 17
   <212> PRT
   <213> Artificial Sequence
<220>
   <223> Description of Artificial Sequence: Synthetic peptide
<400> 31
   Trp Ile Asn Pro Asn Thr Gly Glu Pro Thr Phe Asp Asp Asp Phe Lys
   1 5 10 15
   Gly
<210> 32
   <211> 11
   <212> PRT
   <213> Artificial Sequence
<220>
   <223> Description of Artificial Sequence: Synthetic peptide
<400> 32
   Ser Arg Gly Lys Asn Glu Ala Trp Phe Ala Tyr
   1 5 10
<210> 33
   <211> 16
   <212> PRT
   <213> Artificial Sequence
<220>
   <223> Description of Artificial Sequence: Synthetic peptide
<400> 33
   Lys Ser Ser Gln Ser Val Leu Tyr Ser Ala Asn His Lys Tyr Leu Ala
   1 5 10 15
<210> 34
   <211> 7
   <212> PRT
   <213> Artificial Sequence
<220>
   <223> Description of Artificial Sequence: Synthetic peptide
<400> 34
   Trp Ala Ser Thr Arg Glu Ser
   1 5
<210> 35
   <211> 9
   <212> PRT
   <213> Artificial Sequence
<220>
   <223> Description of Artificial Sequence: Synthetic peptide
<400> 35
   His Gln Tyr Leu Ser Ser Trp Thr Phe
   1 5
<210> 36
   <211> 5
   <212> PRT
   <213> Artificial Sequence
<220>
   <223> Description of Artificial Sequence: Synthetic peptide
<400> 36
   Ser Tyr Trp Leu His
   1               5
<210> 37
   <211> 17
   <212> PRT
   <213> Artificial Sequence
<220>
   <223> Description of Artificial Sequence: Synthetic peptide
<400> 37
   Tyr Ile Asn Pro Arg Asn Asp Tyr Thr Glu Tyr Asn Gln Asn Phe Lys
   1               5                   10                  15
   Asp
<210> 38
   <211> 7
   <212> PRT
   <213> Artificial Sequence
<220>
   <223> Description of Artificial Sequence: Synthetic peptide
<400> 38
   Arg Asp Ile Thr Thr Phe Tyr
   1               5
<210> 39
   <211> 16
   <212> PRT
   <213> Artificial Sequence
<220>
   <223> Description of Artificial Sequence: Synthetic peptide
<400> 39
   Arg Ser Ser Gln Ser Ile Val His Ser Asn Gly Asn Thr Tyr Leu Glu
   1               5                   10                  15
<210> 40
   <211> 7
   <212> PRT
   <213> Artificial Sequence
<220>
   <223> Description of Artificial Sequence: Synthetic peptide
<400> 40
   Lys Val Ser Asn Arg Phe Ser
   1               5
<210> 41
   <211> 9
   <212> PRT
   <213> Artificial Sequence
<220>
   <223> Description of Artificial Sequence: Synthetic peptide
<400> 41
   Phe Gln Gly Ser Arg Val Pro Tyr Thr
   1               5
<210> 42
   <211> 5
   <212> PRT
   <213> Artificial Sequence
<220>
   <223> Description of Artificial Sequence: Synthetic peptide
<400> 42
   Glu Tyr Val Ile Thr
   1               5
<210> 43
   <211> 16
   <212> PRT
   <213> Artificial Sequence
<220>
   <223> Description of Artificial Sequence: Synthetic peptide
<400> 43
   Glu Ile Tyr Pro Gly Ser Gly Ser Thr Ser Tyr Asn Glu Lys Phe Lys
   1               5                   10                  15
<210> 44
   <211> 3
   <212> PRT
   <213> Artificial Sequence
<220>
   <223> Description of Artificial Sequence: Synthetic peptide
<400> 44
   Glu Asp Leu
   1
<210> 45
   <211> 12
   <212> PRT
   <213> Artificial Sequence
<220>
   <223> Description of Artificial Sequence: Synthetic peptide
<400> 45
   Ser Ala Ser Ser Ser Val Ser Ser Ser Tyr Leu Tyr
   1 5 10
<210> 46
   <211> 7
   <212> PRT
   <213> Artificial Sequence
<220>
   <223> Description of Artificial Sequence: Synthetic peptide
<400> 46
   Ser Thr Ser Asn Leu Ala Ser
   1 5
<210> 47
   <211> 9
   <212> PRT
   <213> Artificial Sequence
<220>
   <223> Description of Artificial Sequence: Synthetic peptide
<400> 47
   His Gln Trp Asn Arg Tyr Pro Tyr Thr
   1 5
<210> 48
   <211> 5
   <212> PRT
   <213> Artificial Sequence
<220>
   <223> Description of Artificial Sequence: Synthetic peptide
<400> 48
   Ser Tyr Val Leu His
   1 5
<210> 49
   <211> 17
   <212> PRT
   <213> Artificial Sequence
<220>
   <223> Description of Artificial Sequence: Synthetic peptide
<400> 49
   Tyr Ile Asn Pro Tyr Asn Asp Gly Thr Gln Tyr Asn Glu Lys Phe Lys
   1               5                   10                  15
   Gly
<210> 50
   <211> 10
   <212> PRT
   <213> Artificial Sequence
<220>
   <223> Description of Artificial Sequence: Synthetic peptide
<400> 50
   Gly Phe Gly Gly Ser Tyr Gly Phe Ala Tyr
   1               5                   10
<210> 51
   <211> 5
   <212> PRT
   <213> Artificial Sequence
<220>
   <223> Description of Artificial Sequence: Synthetic peptide
<400> 51
   Ser Tyr Val Ile His
   1               5
<210> 52
   <211> 17
   <212> PRT
   <213> Artificial Sequence
<220>
   <223> Description of Artificial Sequence: Synthetic peptide
<400> 52
   Tyr Ile His Pro Tyr Asn Gly Gly Thr Lys Tyr Asn Glu Lys Phe Lys
   1               5                   10                  15
   Gly
<210> 53
   <211> 9
   <212> PRT
   <213> Artificial Sequence
<220>
   <223> Description of Artificial Sequence: Synthetic peptide
<400> 53
   Ser Gly Gly Gly Asp Pro Phe Ala Tyr
   1               5
<210> 54
   <211> 11
   <212> PRT
   <213> Artificial Sequence
<220>
   <223> Description of Artificial Sequence: Synthetic peptide
<400> 54
   Lys Ala Ser Gln Asp Ile Asn Lys Tyr Ile Gly
   1               5                   10
<210> 55
   <211> 7
   <212> PRT
   <213> Artificial Sequence
<220>
   <223> Description of Artificial Sequence: Synthetic peptide
<400> 55
   Tyr Thr Ser Ala Leu Leu Pro
   1               5
<210> 56
   <211> 8
   <212> PRT
   <213> Artificial Sequence
<220>
   <223> Description of Artificial Sequence: Synthetic peptide
<400> 56
   Leu Gln Tyr Asp Asp Leu Trp Thr
   1               5
<210> 57
   <211> 5
   <212> PRT
   <213> Artificial Sequence
<220>
   <223> Description of Artificial Sequence: Synthetic peptide
<400> 57
   Asn Tyr Gly Met Asn
   1 5
<210> 58
   <211> 17
   <212> PRT
   <213> Artificial Sequence
<220>
   <223> Description of Artificial Sequence: Synthetic peptide
<400> 58
   Trp Ile Asn Thr Tyr Thr Arg Glu Pro Thr Tyr Ala Asp Asp Phe Lys
   1 5 10 15
   Gly
<210> 59
   <211> 12
   <212> PRT
   <213> Artificial Sequence
<220>
   <223> Description of Artificial Sequence: Synthetic peptide
<400> 59
   Asp Ile Thr Ala Val Val Pro Thr Gly Phe Asp Tyr
   1 5 10
<210> 60
   <211> 11
   <212> PRT
   <213> Artificial Sequence
<220>
   <223> Description of Artificial Sequence: Synthetic peptide
<400> 60
   Arg Ala Ser Glu Asn Ile Tyr Ser Asn Leu Ala
   1 5 10
<210> 61
   <211> 7
   <212> PRT
   <213> Artificial Sequence
<220>
   <223> Description of Artificial Sequence: Synthetic peptide
<400> 61
   Ala Ala Ser Asn Leu Ala Asp
   1 5
<210> 62
   <211> 9
   <212> PRT
   <213> Artificial Sequence
<220>
   <223> Description of Artificial Sequence: Synthetic peptide
<400> 62
   Gln His Phe Trp Thr Thr Pro Trp Ala
   1 5
<210> 63
   <211> 10
   <212> PRT
   <213> Artificial Sequence
<220>
   <223> Description of Artificial Sequence: Synthetic peptide
<400> 63
   Arg Ala Ser Ser Ser Val Ser Tyr Ile His
   1 5 10
<210> 64
   <211> 7
   <212> PRT
   <213> Artificial Sequence
<220>
   <223> Description of Artificial Sequence: Synthetic peptide
<400> 64
   Ala Thr Ser Asn Leu Ala Ser
   1 5
<210> 65
   <211> 9
   <212> PRT
   <213> Artificial Sequence
<220>
   <223> Description of Artificial Sequence: Synthetic peptide
<400> 65
   Gln Gln Trp Thr Ser Asn Pro Pro Thr
   1 5
<210> 66
   <211> 5
   <212> PRT
   <213> Artificial Sequence
<220>
   <223> Description of Artificial Sequence: Synthetic peptide
<400> 66
   Ser Tyr Asn Met His
   1               5
<210> 67
   <211> 17
   <212> PRT
   <213> Artificial Sequence
<220>
   <223> Description of Artificial Sequence: Synthetic peptide
<400> 67
   Ala Ile Tyr Pro Gly Asn Gly Asp Thr Ser Tyr Asn Gln Lys Phe Lys
   1               5                   10                  15
   Gly
<210> 68
   <211> 12
   <212> PRT
   <213> Artificial Sequence
<220>
   <223> Description of Artificial Sequence: Synthetic peptide
<400> 68
   Ser Thr Tyr Tyr Gly Gly Asp Trp Tyr Phe Asp Val
   1               5                   10
<210> 69
   <211> 15
   <212> PRT
   <213> Artificial Sequence
<220>
   <223> Description of Artificial Sequence: Synthetic peptide
<400> 69
   Lys Ala Ser Gln Ser Val Asp Tyr Asp Gly Asp Ser Tyr Leu Asn
   1               5                   10                  15
<210> 70
   <211> 7
   <212> PRT
   <213> Artificial Sequence
<220>
   <223> Description of Artificial Sequence: Synthetic peptide
<400> 70
   Asp Ala Ser Asn Leu Val Ser
   1 5
<210> 71
   <211> 9
   <212> PRT
   <213> Artificial Sequence
<220>
   <223> Description of Artificial Sequence: Synthetic peptide
<400> 71
   Gln Gln Ser Thr Glu Asp Pro Trp Thr
   1 5
<210> 72
   <211> 5
   <212> PRT
   <213> Artificial Sequence
<220>
   <223> Description of Artificial Sequence: Synthetic peptide
<400> 72
   Ser Tyr Trp Met Asn
   1 5
<210> 73
   <211> 17
   <212> PRT
   <213> Artificial Sequence
<220>
   <223> Description of Artificial Sequence: Synthetic peptide
<400> 73
   Gln Ile Trp Pro Gly Asp Gly Asp Thr Asn Tyr Asn Gly Lys Phe Lys
   1 5 10 15
   Gly
<210> 74
   <211> 15
   <212> PRT
   <213> Artificial Sequence
<220>
   <223> Description of Artificial Sequence: Synthetic peptide
<400> 74
   Arg Glu Thr Thr Thr Val Gly Arg Tyr Tyr Tyr Ala Met Asp Tyr
   1               5                   10                  15

## Description

### FIELD OF THE INVENTION

The present invention relates to an antibody-drug conjugate (ADC) for use in a method for neoadjuvant treatment of cancer and a standard anti-cancer therapy for use in a method for neoadjuvant treatment of cancer, as defined in the claims.

### BACKGROUND OF THE INVENTION

Neoadjuvant agents are administered to a patient prior to treatment with a primary therapy, such as surgery or radiation therapy (see, e.g., Wikipedia - Neoadjuvant therapy). The object of neoadjuvant cancer therapy is to reduce the size or extent of the patient's tumor(s) before the primary therapy, preferably improving the likelihood of successful outcome and/or decreasing the adverse effects of more extensive treatment that would be required in the absence of neoadjuvant therapy (*Id.*). Neoadjuvant treatment may also target micrometasteses that may be unaffected by the primary therapy (*Id.*)*.* Recently, neoadjuvant therapy has been gaining a role as a means to test novel chemotherapies more expeditiously, because responses to neoadjuvant therapy can be assessed rapidly in a relatively small number of patients, and can be predictive of longer-term outcome (Rastagi et al., 2008, J Clin Oncol 26:778-85; Bardia & Baselga, 2013, Clin Cancer Res 19:6360-70). Indeed, evidence from neoadjuvant studies indicates that determination of pathologic complete response (pCR) at surgery (i.e., no residual disease in the breast and axilla) is predictive of long-term clinical response, even after two cycles of neoadjuvant chemotherapy (Rastagi et al., 2008, J Clin Oncol 26:778-85; von Mickwitz et al., 2012, J Clin Oncol 30:1796-1804; Huober et al., 2010, Breast Cancer Res Treat 124:133-40).

The history of neoadjuvant treatment in cancer is extensive. Much of the earlier work in this field related to use of neoadjuvant chemotherapy prior to surgerical excision or radiation therapy. Ervin et al. (1984, Arch Otolaryngol 110:241-5) reported neoadjuvant chemotherapy of advanced head and neck cancer with cisplatin, bleomycin and methotrexate, before surgery plus radiotherapy or high-dose radiotherapy alone. Although some improvement in outcome was seen, particularly where neoadjuvant therapy resulted in substantial tumor reduction, relapse of disease was common (Ervin et al, 1984). Neoadjuvant chemotherapy and/or radiation therapy has also been reported in osteogenic sarcoma (Rosen & Nirenberg, 1985, Prog Clin Biol Res 201:39-51), breast cancer (Ragaz et al., 1985, Prog Clin Biol Res 201:77-87), esophageal cancer (Kelsen et al., 1986, Semin Surg Oncol 2:170-6), anal and rectal cancer (Smith et al., 1986, Am J Surg 151:577-80), lung cancer (Cox et al., 1986, Cancer Treat Rep 70:1219-20) and many other forms of cancer. While improved outcome is often reported with neoadjuvant therapy, the degree to which neoadjuvant chemotherapy and/or radiation therapy improves long-term patient survival in cancer has generally not yet been confirmed by prospective studies (see, e.g., Bittoni et al., 2014, Gastroenterol Res Pract 2014:183852; Doval et al., 2013, J Indian Med Assoc 111:629-31).

Recently, neoadjuvant use of antibodies or antibody-drug conjugates (ADCs) has been attempted in breast cancer. Pertuzumab (anti-HER2) has been investigated and received FDA approval in combination with trastuzumab and docetaxel in neoadjuvant treatment of HER2-positive metastatic breast cancer (Sabatier & Goncalves, 2014, Bull Cancer 101:765-71; Esserman & DeMichele, 2014, Clin Cancer Res 20:3632-36). Ado-trastuzumab emtansine (T-DM1), comprising an anti-HER2 antibody conjugated to the potent microtubule inhibitor emtansine, has been approved for use in HER2-positive metastatic breast cancer for patients who have failed previous therapy and is being investigated for neoadjuvant use (Corrigan et al., 2014, Ann Pharmacother [Epub ahead of print, July 31, 2014]).

While these results are promising, anti-HER2 antibodies are of little use in, for example, triple-negative breast cancer (TNBC), which lacks expression of estrogen receptors, progesterone receptors and HER2 (e.g., Gogia et al., 2014, Indian J Cancer 51:163-6). TNBC accounts for about 10 to 20% of breast cancers and is more aggressive and lethal than other forms of this disease, with virtually all women with metastatic TNBC ultimately dying of the disease, despite systemic therapy. A need exists in the field for more effective forms of immunoconjugate-based neoadjuvant cancer therapy, particularly for forms of cancer that are resistant to standard anti-cancer treatments, such as TNBC.

US 2014/0099254 A1 is related to a combination of two or more agents for inducing an immune response to cancer or infectious disease, wherein agents include leukocyte redirecting complexes, antibody-drug conjugates, interferons and/or checkpoint inhibitor antibodies.

US 2014/0227180 A1 is related to methods and compositions for preparing SN-38 conjugates of proteins or peptides, preferably immunoconjugates of antibodies or antigen-binding antibody fragments.

### SUMMARY OF THE INVENTION

The problem underlying the present invention is solved by the subject matter of the attached independent claims; preferred embodiments may be taken from the attached dependent claims.

More specifically, the problem underlying the present invention is solved in a first aspect by an antibody-drug conjugate (ADC) for use in a method for neoadjuvant treatment of cancer comprising:
a) administering the antibody-drug conjugate (ADC) comprising an antibody moiety conjugated to a drug, to a subject with cancer, wherein the antibody moiety is an anti-Trop-2 hRS7 antibody and the drug is SN-38; and
b) treating the subject with a standard anti-cancer therapy selected from the group consisting of surgery, and radiation therapy, and
wherein the ADC is administered before the standard anti-cancer therapy.

More specifically, the problem underlying the present invention is solved in a second aspect by a standard anti-cancer therapy for use in a method for neoadjuvant treatment of cancer comprising:
a) administering an antibody-drug conjugate (ADC) comprising an antibody moiety conjugated to a drug, to a subject with cancer, wherein the antibody moiety is an anti-Trop-2 hRS7 antibody and the drug is SN-38; and
b) treating the subject with the standard anti-cancer therapy, wherein the standard anti-cancer therapy is selected from the group consisting of surgery, and radiation therapy, and
wherein the ADC is administered before the standard anti-cancer therapy.

In an embodiment of the first and the second aspect, the antibody moiety comprises human constant regions selected from the group consisting of IgG1, IgG2, IgG3 and IgG4.

In an embodiment of the first and the second aspect, the antibody moiety has an allotype selected from the group consisting of non-Glml (nG1m1), G1m3, and Km3.

In an embodiment of the first and the second aspect, the ADC comprises a linker that attaches the drug to the antibody moiety.

In an embodiment of the first and the second aspect, the drug is SN-38 and the linker is CL2A.

In an embodiment of the first and the second aspect, the method further comprises administering at least one therapeutic agent to said subject.

In a preferred embodiment of the first and the second aspect, the therapeutic agent is selected from the group consisting of a toxin, an enzyme, a hormone, a second antibody, a second antibody fragment, an immunoconjugate, a radionuclide, an antisense oligonucleotide, and an RNAi.

In a more preferred embodiment of the first and the second aspect, the radionuclide is attached to an antibody or antigen-binding antibody fragment and is selected from the group consisting of ¹¹¹In, ¹¹¹At, ¹⁷⁷Lu, ²¹¹Bi, ²¹²Bi, ²¹³Bi, ²¹¹At, ⁶²Cu , ⁶⁷Cu, ⁹⁰Y, ¹²⁵I, ¹³¹I, ¹³³I, ³²P, ³³P, ⁴⁷Sc, ¹¹¹Ag, ⁶⁷Ga, ¹⁵³Sm, ¹⁶¹Tb, ¹⁵²Dy, ¹⁶⁶Dy, ¹⁶¹Ho, ¹⁶⁶Ho, ¹⁸⁶Re, ¹⁸⁸Re, ¹⁸⁹Re, ²¹¹Pb, ²¹²Pb, ²²³Ra, ²²⁵Ac, ⁷⁷As, ⁸⁹Sr, ⁹⁹Mo, ¹⁰⁵Rh , ¹⁴⁹Pm, ¹⁶⁹Er, ¹⁹⁴Ir, ⁵⁸Co, ^{80m}Br, ^{99m}Tc, ^{103m}Rh, ¹⁰⁹Pt, ¹¹⁹Sb, ^{189m}Os, ¹⁹²Ir ²¹⁹Rn, ²¹⁵Po, ²²¹Fr, ²⁵⁵Fm, ¹¹C, ¹³N, ¹⁵O, ⁷⁵Br, ¹⁹⁸Au, ¹⁹⁹Au, ²²⁴Ac, ⁷⁷Br, ^{113m}In, ⁹⁵Ru, ⁹⁷Ru, ¹⁰³Ru, ¹⁰⁵Ru, ¹⁰⁷Hg, ²⁰³Hg, ^{121m}Te, ^{122m}Te, ^{125m}Te, ²²⁷Th, ¹⁶⁵Tm, ¹⁶⁷Tm, ¹⁶⁸Tm, ¹⁹⁷Pt, ¹⁰⁹Pd, ¹⁴²Pr, ¹⁴³Pr, ¹⁶¹Tb , ⁵⁷Co, ⁵⁸Co, ⁵¹Cr, ⁵⁹Fe, ⁷⁵Se, ²⁰¹Tl, ⁷⁶Br and ¹⁶⁹Yb.

In an embodiment of the first and the second aspect, the cancer is selected from the group consisting of colon cancer, stomach cancer, esophageal cancer, medullary thyroid cancer, kidney cancer, breast cancer, lung cancer, pancreatic cancer, urinary bladder cancer, ovarian cancer, uterine cancer, cervical cancer, testicular cancer, prostate cancer, liver cancer, skin cancer, bone cancer, brain cancer, rectal cancer, and melanoma.

In an embodiment of the first and the second aspect, the cancer is selected from the group consisting of triple negative breast cancer (TNBC), metastatic colon cancer, metastatic non-small-cell lung cancer (NSCLC), metastatic pancreatic cancer, metastatic renal cell carcinoma, metastatic gastric cancer, metastatic prostate cancer, and metastatic small-cell lung cancer.

In an embodiment of the first and the second aspect, the cancer is refractory to other therapies but responds to therapy with neoadjuvant ADC.

The present invention as defined in the claims makes use of antibody conjugates of SN-38 that, preferably, have nanomolar toxicities *in vitro,* compared to the sub-nanomolar to picomolar toxicities of ultratoxic chemotherapeutic agents like calicheamicin, maytansinoids or MMAE. Use of drugs that are not ultratoxic allows the use of antibody-drug linkers that do not require cell internalization for the release of free drugs, but rather allow some extracellular release of drug. With the CL2A linker described below, 50% of the conjugated drug is released in 24 hr, thereby augmenting the bioavailability of the drug by liberating it both extracellularly and intracellularly. In addition, the use of relatively non-toxic drugs allows the administration of higher dosages of ADCs, leading to better therapeutic effects.

The present invention as defined in the claims resolves an unfulfilled need in the art. The disclosed methods and compositions are of use for the neoadjuvant treatment of cancers which are refractory or less responsive to other forms of therapy. Refractory cancers may include, but are not limited to, triple-negative breast cancer, metastatic colon cancer, metastatic non-small-cell lung cancer (NSCLC), metastatic pancreatic cancer, metastatic renal cell carcinoma, metastatic gastric cancer, metastatic prostate cancer, or metastatic small-cell lung cancer.

The antibody can be of various isotypes, preferably human IgG1, IgG2, IgG3 or IgG4, more preferably comprising human IgG1 hinge and constant region sequences. The antibody or fragment thereof can be a chimeric, humanized, or fully human antibody or antigen-binding fragment thereof, such as half-IgG4 antibodies, as described by van der Neut Kolfschoten et al. (Science 2007; 317:1554-1557), or single domain antibodies (e.g., nanobodies) as commercially available (e.g., ABLYNX®, Ghent, Belgium). More preferably, the antibody or fragment thereof may be designed or selected to comprise human constant region sequences that belong to specific allotypes, which may result in reduced immunogenicity when the immunoconjugate is administered to a human subject. Preferred allotypes for administration include a non-Glml allotype (nG1m1), such as G1m3, G1m3,1, G1m3,2 or G1m3,1,2. More preferably, the allotype is selected from the group consisting of the nG1m1, G1m3, nG1m1,2 and Km3 allotypes.

For neoadjuvant treatment of cancer, many antigens expressed by or otherwise associated with tumor cells are known in the art, including but not limited to, carbonic anhydrase IX, alpha-fetoprotein (AFP), α-actinin-4, A3, antigen specific for A33 antibody, ART-4, B7, Ba 733, BAGE, BrE3-antigen, CA125, CAMEL, CAP-1, CASP-8/m, CCL19, CCL21, CD1, CD1a, CD2, CD3, CD4, CD5, CD8, CD11A, CD14, CD15, CD16, CD18, CD19, CD20, CD21, CD22, CD23, CD25, CD29, CD30, CD32b, CD33, CD37, CD38, CD40, CD40L, CD44, CD45, CD46, CD52, CD54, CD55, CD59, CD64, CD66a-e, CD67, CD70, CD70L, CD74, CD79a, CD80, CD83, CD95, CD126, CD132, CD133, CD138, CD147, CD154, CDC27, CDK-4/m, CDKN2A, CTLA-4, CXCR4, CXCR7, CXCL12, HIF-1α, colon-specific antigen-p (CSAp), CEACAM5, CEACAM6, c-Met, DAM, EGFR, EGFRvIII, EGP-1 (TROP-2), EGP-2, ELF2-M, Ep-CAM, fibroblast growth factor (FGF), Flt-1, Flt-3, folate receptor, G250 antigen, GAGE, gp100, GRO-β, HLA-DR, HM1.24, human chorionic gonadotropin (HCG) and its subunits, HER2/neu, histone H2B, histone H3, histone H4, HMGB-1, hypoxia inducible factor (HIF-1), HSP70-2M, HST-2, la, IGF-1R, IFN-γ, IFN-α, IFN-β, IFN-λ, IL-4R, IL-6R, IL-13R, IL-15R, IL-17R, IL-18R, IL-2, IL-6, IL-8, IL-12, IL-15, IL-17, IL-18, IL-23, IL-25, insulin-like growth factor-1 (IGF-1), KC4-antigen, KS-1-antigen, KS1-4, Le-Y, LDR/FUT, macrophage migration inhibitory factor (MIF), MAGE, MAGE-3, MART-1, MART-2, NY-ESO-1, TRAG-3, mCRP, MCP-1, MIP-1A, MIP-1B, MIF, MUC1, MUC2, MUC3, MUC4, MUC5ac, MUC13, MUC16, MUM-1/2, MUM-3, NCA66, NCA95, NCA90, PAM4 antigen, pancreatic cancer mucin, PD-1, PD-L1, PD-1 receptor, placental growth factor, p53, PLAGL2, prostatic acid phosphatase, PSA, PRAME, PSMA, PlGF, ILGF, ILGF-1R, IL-6, IL-25, RS5, RANTES, T101, SAGE, S100, survivin, survivin-2B, TAC, TAG-72, tenascin, TRAIL receptors, TNF-α, Tn antigen, Thomson-Friedenreich antigens, tumor necrosis antigens, VEGFR, ED-B fibronectin, WT-1, 17-1A-antigen, complement factors C3, C3a, C3b, C5a, C5, an angiogenesis marker, bcl-2, bcl-6, Kras, an oncogene marker and an oncogene product (see, e.g., Sensi et al., Clin Cancer Res 2006, 12:5023-32; Parmiani et al., J Immunol 2007, 178:1975-79; Novellino et al. Cancer Immunol Immunother 2005, 54:187-207). Preferably, the antibody binds to AFP, CEACAM5, CEACAM6, CSAp, EGP-1 (TROP-2), AFP, MUC5ac, PAM4 antigen, CD74, CD19, CD20, CD22 or HLA-DR.

Exemplary antibodies that may be utilized include, but are not limited to, hR1 (anti-IGF-1R, U.S. Patent Application Serial No. 12/722,645, filed 3/12/10), hPAM4 (anti-MUC5ac, U.S. Patent No. 7,282,567), hA20 (anti-CD20, U.S. Patent No. 7,151,164), hA19 (anti-CD19, U.S. Patent No. 7,109,304), hIMMU31 (anti-AFP, U.S. Patent No. 7,300,655), hLL1 (anti-CD74, U.S. Patent No. 7,312,318), hLL2 (anti-CD22, U.S. Patent No. 5,789,554), hRFB4 (anti-CD22, U.S. Prov. Pat. Appl. Serial No. 61/944,295, filed 2/25/14), hMu-9 (anti-CSAp, U.S. Patent No. 7,387,772), hL243 (anti-HLA-DR, U.S. Patent No. 7,612,180), hMN-14 (anti-CEACAM5, U.S. Patent No. 6,676,924), hMN-15 (anti-CEACAM6, U.S. Patent No. 8,287,865), hRS7 (anti-TROP-2, U.S. Patent No. 7,238,785), hMN-3 (anti-CEACAM6, U.S. Patent No. 7,541,440), Ab124 and Ab125 (anti-CXCR4, U.S. Patent No. 7,138,496). More preferably, the antibody is IMMU-31 (anti-AFP), hRS7 (anti-TROP-2), hMN-14 (anti-CEACAM5), hMN-3 (anti-CEACAM6), hMN-15 (anti-CEACAM6), hLL1 (anti-CD74), hLL2 (anti-CD22), hL243 or IMMU-114 (anti-HLA-DR), hA19 (anti-CD19) or hA20 (anti-CD20). As used herein, the terms epratuzumab and hLL2 are interchangeable, as are the terms veltuzumab and hA20, and hL243g4P, hL243gamma4P and IMMU-114.

Alternative antibodies of use include, but are not limited to, abciximab (antiglycoprotein IIb/IIIa), alemtuzumab (anti-CD52), bevacizumab (anti-VEGF), cetuximab (anti-EGFR), gemtuzumab (anti-CD33), ibritumomab (anti-CD20), panitumumab (anti-EGFR), rituximab (anti-CD20), tositumomab (anti-CD20), trastuzumab (anti-ErbB2), lambrolizumab (anti-PD-1 receptor), nivolumab (anti-PD-1 receptor), ipilimumab (anti-CTLA-4), abagovomab (anti-CA-125), adecatumumab (anti-EpCAM), atlizumab (anti-IL-6 receptor), benralizumab (anti-CD125), obinutuzumab (GA101, anti-CD20), CC49 (anti-TAG-72), AB-PG1-XG1-026 (anti-PSMA, U.S. Patent Application 11/983,372, deposited as ATCC PTA-4405 and PTA-4406), D2/B (anti-PSMA, WO 2009/130575), tocilizumab (anti-IL-6 receptor), basiliximab (anti-CD25), daclizumab (anti-CD25), efalizumab (anti-CD11a), GA101 (anti-CD20; Glycart Roche), muromonab-CD3 (anti-CD3 receptor), natalizumab (anti-α4 integrin), omalizumab (anti-IgE); anti-TNF-a antibodies such as CDP571 (Ofei et al., 2011, Diabetes 45:881-85), MTNFAI, M2TNFAI, M3TNFAI, M3TNFABI, M302B, M303 (Thermo Scientific, Rockford, IL), infliximab (Centocor, Malvern, PA), certolizumab pegol (UCB, Brussels, Belgium), anti-CD40L (UCB, Brussels, Belgium), adalimumab (Abbott, Abbott Park, IL), and belimumab (Human Genome Sciences). Recently, humanized antibodies against human histones H2B, H3 and H4 have been disclosed (U.S. Patent Application Serial No. 14/180,646) that may be utilized in the disclosed methods and compositions.

In an embodiment of the invention as defined in the claims, the antibody moiety links to at least one drug moiety, more preferably 1 to about 5 drug moieties, alternatively about 6 to 12 drug moieties. In various embodiments, the antibody moiety may be attached to 4 or 6 drug moieties, or to 5 or less drug moieties. The number of drug moieties per antibody moiety may be 1, 2, 3, 4, 5, 6, 7, or more.

An exemplary camptothecin is CPT-11. Extensive clinical data are available concerning CPT-11's pharmacology and its *in vivo* conversion to the active SN-38 (Iyer and Ratain, Cancer Chemother Pharmacol. 42: S31-43 (1998); Mathijssen et al., Clin Cancer Res. 7:2182-2194 (2002); Rivory, Ann NY Acad Sci. 922:205-215, 2000)). The active form SN-38 is about 2 to 3 orders of magnitude more potent than CPT-11. In specific examples , the immunoconjugate may be an hMN-14-SN-38, hMN-3-SN-38, hMN-15-SN-38, hIMMU-31-SN-38, hR1-SN-38, hA20-SN-38, hPAM4-SN-38, hL243-SN-38, hLL1-SN-38, hRFB4-SN-38, hMu-9-SN-38 or hLL2-SN-38 conjugate. In an embodiment of the invention as defined in the claims, a CL2A linker is used to conjugate the SN-38 to the antibody moiety.

An exemplary anthracycline is a prodrug form of 2-pyrrolinodoxorubicin (P2PDox), such as N-(4,4-diacetoxybutyl)doxorubicin, disclosed in U.S. Patent Application Serial No. 14/175,089. Surprisingly, P2PDox has been found to be tightly bound to conjugated antibody, due to the formation of cross-links with antibody peptide chains. The cross-linking assists in minimizing toxicity, for example cardiotoxicity, that would result from release of free drug in circulation. Preferably, the P2PDox is attached to interchain disulfide thiol groups while in the prodrug form. The prodrug protection is rapidly removed *in vivo* soon after injection and the resulting 2-PDox portion of the conjugate cross-links the peptide chains of the antibody, forming intramolecular cross-linking within the antibody molecule. This both stabilizes the ADC and prevents cross-linking to other molecules in circulation. In specific preferred embodiments, the immunoconjugate may be an hMN-14-P2PDox, hMN-3-P2PDox, hMN-15-P2PDox, hIMMU-31-P2PDox, hRS7-P2PDox, hR1-P2PDox, hA20-P2PDox, hPAM4-P2PDox, hL243-P2PDox, hLL1-P2PDox, hRFB4-P2PDox, hMu-9-P2PDox or hLL2-P2PDox conjugate.

The instant disclosure concerns the possible use of the subject methods and compositions to treat a cancer, including but not limited to non-Hodgkin's lymphomas, B-cell acute and chronic lymphoid leukemias, Burkitt lymphoma, Hodgkin's lymphoma, acute large B-cell lymphoma, hairy cell leukemia, acute myeloid leukemia, chronic myeloid leukemia, acute lymphocytic leukemia, chronic lymphocytic leukemia, T-cell lymphomas and leukemias, multiple myeloma, Waldenstrom's macroglobulinemia.

According to the invention as defined in the claims, the cancer may by one of carcinomas, melanomas, sarcomas, gliomas, bone, and skin cancers. The carcinomas may include carcinomas of the oral cavity, esophagus, gastrointestinal tract, pulmonary tract, lung, stomach, colon, breast, ovary, prostate, uterus, endometrium, cervix, urinary bladder, pancreas, bone, brain, connective tissue, liver, gall bladder, urinary bladder, kidney, skin, central nervous system and testes.

In certain embodiments of the invention as defined in the claims, the drug conjugates may be used as neoadjuvants prior to treatment with surgery, radiation therapy, chemotherapy, immunotherapy with naked antibodies, radioimmunotherapy, immunomodulators, and the like. These neoadjuvant therapies can allow lower doses of each therapeutic to be given, thus reducing certain severe side effects, or improving the efficacy of other treatments such as surgery.

Preferred optimal dosing of immunoconjugates may include a dosage of between 3 mg/kg and 18 mg/kg, preferably given either weekly, twice weekly, every other week or every third week. The optimal dosing schedule may include treatment cycles of two consecutive weeks of therapy followed by one, two, three or four weeks of rest, or alternating weeks of therapy and rest, or one week of therapy followed by two, three or four weeks of rest, or three weeks of therapy followed by one, two, three or four weeks of rest, or four weeks of therapy followed by one, two, three or four weeks of rest, or five weeks of therapy followed by one, two, three, four or five weeks of rest, or administration once every two weeks, once every three weeks or once a month. Treatment may be extended for any number of cycles, preferably at least 2, at least 4, at least 6, at least 8, at least 10, at least 12, at least 14, or at least 16 cycles. The dosage may be up to 24 mg/kg. Exemplary dosages of use may include 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, 20 mg/kg, 22 mg/kg and 24 mg/kg. Preferred dosages are 4, 6, 8, 9, 10, 12, 14, 16 or 18 mg/kg. The person of ordinary skill will realize that a variety of factors, such as age, general health, specific organ function or weight, as well as effects of prior therapy on specific organ systems (e.g., bone marrow) may be considered in selecting an optimal dosage of immunoconjugate, and that the dosage and/or frequency of administration may be increased or decreased during the course of therapy. The dosage may be repeated as needed, with evidence of tumor shrinkage observed after as few as 4 to 8 doses. The optimized dosages and schedules of administration for neoadjuvant use disclosed herein show unexpected superior efficacy and reduced toxicity in human subjects, which could not have been predicted from animal model studies. Surprisingly, the superior efficacy allows treatment of tumors that were previously found to be resistant to one or more standard anticancer therapies.

A surprising result with the invention as defined in the claims is the unexpected tolerability of high doses of antibody-drug conjugate, even with repeated infusions, with only relatively low-grade toxicities of nausea and vomiting observed, or manageable neutropenia. A further surprising result is the lack of accumulation of the antibody-drug conjugate, unlike other products that have conjugated chemotherapeutic drugs to albumin, PEG or other carriers. The lack of accumulation is associated with improved tolerability and lack of serious toxicity even after repeated or increased dosing. These surprising results allow optimization of dosage and delivery schedule, with unexpectedly high efficacies and low toxicities. In an embodiment, the invention as defined in the claims provides for shrinkage of solid tumors, in individuals with previously resistant cancers, of 15% or more, preferably 20% or more, preferably 30% or more, more preferably 40% or more in size (as measured by longest diameter). The person of ordinary skill will realize that tumor size may be measured by a variety of different techniques, such as total tumor volume, maximal tumor size in any dimension or a combination of size measurements in several dimensions. This may be with standard radiological procedures, such as computed tomography, ultrasonography, and/or positron-emission tomography. The means of measuring size is less important than observing a trend of decreasing tumor size with immunoconjugate treatment, preferably resulting in elimination of the tumor.

While the immunoconjugate may be administered as a periodic bolus injection, in alternative embodiments the immunoconjugate may be administered by continuous infusion of antibody-drug conjugates. In order to increase the Cmax and extend the PK of the immunoconjugate in the blood, a continuous infusion may be administered for example by indwelling catheter. Such devices are known in the art, such as HICKMAN®, BROVIAC® or PORT-A-CATH® catheters (see, e.g., Skolnik et al., Ther Drug Monit 32:741-48, 2010) and any such known indwelling catheter may be used. A variety of continuous infusion pumps are also known in the art and any such known infusion pump may be used. The dosage range for continuous infusion may be between 0.1 and 3.0 mg/kg per day. More preferably, these immunoconjugates can be administered by intravenous infusions over relatively short periods of 2 to 5 hours, more preferably 2-3 hours.

In particularly preferred embodiments of the invention as defined in the claims, the immunoconjugates and dosing schedules may be efficacious in patients resistant to standard therapies. For example, an hMN-14-SN-38 immunoconjugate may be administered to a patient who has not responded to prior therapy with irinotecan, the parent agent of SN-38. Surprisingly, the irinotecan-resistant patient may show a partial or even a complete response to hMN-14-SN-38. The ability of the immunoconjugate to specifically target the tumor tissue may overcome tumor resistance by improved targeting and enhanced delivery of the therapeutic agent. Alternatively, an anti-CEACAM5 immunoconjugate, such as hMN-14, may be co-administered with an anti-CEACAM6 immunoconjugate, such as hMN-3 or hMN-15. Other antibody-SN-38 or antibody-P2PDox immunoconjugates may show similar improved efficacy and/or decreased toxicity, compared to alternative standard therapeutic treatments, and combinations of different immunoconjugates, or ADCs in combination with an antibody conjugated to a radionuclide, toxin or other drug, may provide even more improved efficacy and/or reduced toxicity. A specific preferred subject may be a metastatic colon cancer patient, a triple-negative breast cancer patient, a HER+, ER+, progesterone+ breast cancer patient, a metastatic non-small-cell lung cancer (NSCLC) patient, a metastatic pancreatic cancer patient, a metastatic renal cell carcinoma patient, a metastatic gastric cancer patient, a metastatic prostate cancer patient, or a metastatic small-cell lung cancer patient.

### BRIEF DESCRIPTION OF THE FIGURES

**FIG. 1****.** Exemplary antibody-drug conjugate, showing the hRS7 anti-TROP-2 antibody conjugated via an intracellularly cleavable CL2A linker to the SN-38 camptothecin drug.
**FIG. 2A****.** Structure of doxorubicin. "Me" is a methyl group.
**FIG. 2B****.** Structure of 2-pyrrolinodoxorubicin,(2-PDox). "Me" is a methyl group.
**FIG. 2C****.** Structure of a prodrug form of 2-pyrrolinodoxorubicin,(P2PDox). "Me" is a methyl group and "Ac" is an acetyl group.
**FIG. 2D****.** Structure of a maleimide-activated form of P2PDox, for antibody coupling. "Me" is a methyl group and "Ac" is an acetyl group.
**FIG. 3A****.** *In vivo* efficacy of IMMU-132 in Calu-3 human NSCLC xenografts.
**FIG. 3B****.** *In vivo* efficacy of IMMU-132 in COLO 205 human colon cancer xenografts.
**FIG. 3C****.** *In vivo* efficacy of IMMU-132 in Capan-1 human pancreatic cancer xenografts.
***FIG. 3D******.** In vivo* efficacy of IMMU-132 in BxPC-3 human pancreatic cancer xenografts.
**FIG. 3E****.** *In vivo* efficacy of IMMU-132 in SK-MES-1 human squamous cell lung cancer xenografts.
**FIG. 3F****.** *In vivo* efficacy of IMMU-132 in NCI-N87 human gastric cancer xenografts.
**FIG. 4A****.** *In vivo* efficacy of IMMU-132 in MDA-MB-468 human TNBC xenografts.
**FIG. 4B****.** *In vivo* efficacy of IMMU-132 in MDA-MB-468 human TNBC xenografts. Tumor bearing mice were initially treated with control (non-targeting) ADC. After allowing the tumors to grow, the mice were administered the indicated dosages of IMMU-132, starting on day 78. Even after allowing the tumors to grow to large size, the IMMU-132 was effective to induce tumor regression.
**FIG. 4C****.** *In vivo* efficacy of IMMU-132 in MDA-MB-231 human TNBC xenografts.
**FIG. 5A****.** Best response for 14 TNBC patients enrolled in IMMU-132-01 trial.
**FIG. 5B****.** Time to progression for 14 TNBC patients enrolled in IMMU-132-01 trial.
**FIG. 6A****.** IMMU-132 peak serum concentrations of IgG and ADC, as a function of dose level.
**FIG. 6B****.** IMMU-132 peak serum concentrations of IgG and ADC, as a function of dose level when normalized to patient weight.
**FIG. 7A****.** Pharmacokinetics of IMMU-132 total IgG vs. total IMMU-132.
**FIG. 7B****.** Pharmacokinetics of IMMU-132 total SN-38 vs. free SN-38.
**FIG. 7C****.** Clearance of IMMU-132 based on ELISA or on SN-38 concentration in the serum.
**FIG. 8A****.** Different IMMU-132 dosing regimens in NCI-N87 human gastric carcinoma xenografts.
**FIG. 8B****.** Different IMMU-132 dosing regimens in BxPC-3 human pancreatic adenocarcinoma xenografts.
**FIG. 9A****.** IMMU-132 mediated apoptotic signaling in NCI-N87 human gastric carcinoma exposed to 1 µM of free SN-38 or the equivalent amount of IMMU-132.
**FIG. 9B****.** IMMU-132 mediated PARP cleavage in SK-BR-3 and MDA-MB 486 breast carcinoma cells.
**FIG.10****.** Pharmacotoxicology studies of IMMU-132 vs. irinotecan in a pancreatic cancer xenograft model.
**FIG. 11****.** Neoadjuvant treatment regimen for paclitaxel +/- IMMU-132 in TNBC.
**FIG. 12****.** Therapy in nude mice bearing s.c. human tumor xenografts using 2.25 mg/kg protein dose (0.064 mg/kg of drug dose) of MAb-P2PDox conjugates twice weekly x 2 weeks in nude mice with Capan-1 human pancreatic adenocarcinoma xenografts (n = 5).
**FIG. 13A****.** MTD study of hLL1-P2PDox conjugates with multiple injections. Mice administered hLL1-P2PDox (q4dx4) at 25 µg i.v. per dose.
**FIG. 13B****.** MTD study of hLL1-P2PDox conjugates with multiple injections. Mice administered hLL1-P2PDox (q4dx4) at 50 µg i.v. per dose.
**FIG. 13C****.** MTD study of hLL1-P2PDox conjugates with multiple injections. Mice administered hLL1-P2PDox (q4dx4) at 100 µg i.v. per dose.
**FIG. 13D****.** MTD study of hLL1-P2PDox conjugates with multiple injections. Mice administered hLL1-P2PDox (q4dx4) at 150 µg i.v. per dose.
**FIG. 14A****.** *In vivo* efficacy of P2PDox conjugates in nude mice with NCI-N87 human gastric cancer xenografts. Mice were administered a saline control.
**FIG. 14B****.** *In vivo* efficacy of P2PDox conjugates in nude mice with NCI-N87 human gastric cancer xenografts. Mice were administered 45 µg of hA20-P2PDox as indicated by arrows.
**FIG. 14C****.** *In vivo* efficacy of P2PDox conjugates in nude mice with NCI-N87 human gastric cancer xenografts. Mice were administered 45 µg of hMN-15-P2PDox as indicated by arrows.
**FIG. 14D****.** *In vivo* efficacy of P2PDox conjugates in nude mice with NCI-N87 human gastric cancer xenografts. Mice were administered 45 µg of hRS7-P2PDox as indicated by arrows.
**FIG. 14E****.** *In vivo* efficacy of P2PDox conjugates in nude mice with NCI-N87 human gastric cancer xenografts. Mice were administered 45 µg of hLLl-P2PDox as indicated by arrows.
**FIG. 14F****.** *In vivo* efficacy of P2PDox conjugates in nude mice with NCI-N87 human gastric cancer xenografts. Mice were administered 45 µg of hMN-14-P2PDox as indicated by arrows.
**FIG. 15****.** Effect of different dosing schedules of hRS7-P2PDox on survival in nude mice with NCI-N87 human gastric carcinoma xenografts.
**FIG. 16****.** Effect of different single doses of hRS7-P2PDox on growth of human gastric carcinoma xenografts.
**FIG. 17****.** Effect of different single doses of hRS7-P2PDox on survival of mice bearing human gastric carcinoma xenografts.
**FIG. 18****.** ADCC of various hRS7-ADCs vs. hRS7 IgG.

### DETAILED DESCRIPTION OF THE INVENTION

### Definitions

In the description that follows, a number of terms are used and the following definitions are provided to facilitate understanding of the claimed subject matter. Terms that are not expressly defined herein are used in accordance with their plain and ordinary meanings.

Unless otherwise specified, a or an means "one or more."

The term about is used herein to mean plus or minus ten percent (10%) of a value. For example, "about 100" refers to any number between 90 and 110.

An antibody, as used herein, refers to a full-length (i.e., naturally occurring or formed by normal immunoglobulin gene fragment recombinatorial processes) immunoglobulin molecule *(e.g.,* an IgG antibody) or an antigen-binding portion of an immunoglobulin molecule, such as an antibody fragment. An antibody or antibody fragment may be conjugated or otherwise derivatized within the scope of the claimed subject matter. Such antibodies include but are not limited to IgG1, IgG2, IgG3, IgG4 (and IgG4 subforms), as well as IgA isotypes.

An antibody fragment is a portion of an antibody such as F(ab')₂, F(ab)₂, Fab', Fab, Fv, scFv (single chain Fv), single domain antibodies (DABs or VHHs) and the like, including the half-molecules of IgG4 cited above (van der Neut Kolfschoten et al. (Science 2007; 317(14 Sept): 1554-1557). A commercially available form of single domain antibody, referred to as a nanobody (ABLYNX®, Ghent, Belgium), is discussed in further detail below. Regardless of structure, an antibody fragment of use binds with the same antigen that is recognized by the intact antibody. The term "antibody fragment" also includes synthetic or genetically engineered proteins that act like an antibody by binding to a specific antigen to form a complex. For example, antibody fragments include isolated fragments consisting of the variable regions, such as the "Fv" fragments consisting of the variable regions of the heavy and light chains, recombinant single chain polypeptide molecules in which light and heavy variable regions are connected by a peptide linker ("scFv proteins"), and minimal recognition units consisting of the amino acid residues that mimic the hypervariable region, such as CDRs. The Fv fragments may be constructed in different ways to yield multivalent and/or multispecific binding forms. In the case of multivalent, they have more than one binding site against the specific epitope, whereas with multispecific forms, more than one epitope (either of the same antigen or against one antigen and a different antigen) is bound.

A naked antibody is generally an entire (full-length) antibody that is not conjugated to a therapeutic agent. This is so because the Fc portion of the antibody molecule provides effector or immunological functions, such as complement fixation and ADCC (antibodydependent cell cytotoxicity), which set mechanisms into action that may result in cell lysis. However, the Fc portion may not be required for therapeutic function of the antibody, but rather other mechanisms, such as apoptosis, anti-angiogenesis, anti-metastatic activity, antiadhesion activity, such as inhibition of heterotypic or homotypic adhesion, and interference in signaling pathways, may come into play and interfere with disease progression. Naked antibodies include both polyclonal and monoclonal antibodies, and fragments thereof, that include murine antibodies, as well as certain recombinant antibodies, such as chimeric, humanized or human antibodies and fragments thereof. As used herein, "naked" is synonymous with "unconjugated," and means not linked or conjugated to a therapeutic agent.

A chimeric antibody is a recombinant protein that contains the variable domains of both the heavy and light antibody chains, including the complementarity determining regions (CDRs) of an antibody derived from one species, preferably a rodent antibody, more preferably a murine antibody, while the constant domains of the antibody molecule are derived from those of a human antibody. For veterinary applications, the constant domains of the chimeric antibody may be derived from that of other species, such as a primate, cat or dog.

A humanized antibody is a recombinant protein in which the CDRs from an antibody from one species; e.g., a murine antibody, are transferred from the heavy and light variable chains of the murine antibody into human heavy and light variable domains (framework regions). The constant domains of the antibody molecule are derived from those of a human antibody. In some cases, specific residues of the framework region of the humanized antibody, particularly those that are touching or close to the CDR sequences, may be modified, for example replaced with the corresponding residues from the original murine, rodent, subhuman primate, or other antibody.

A human antibody is an antibody obtained, for example, from transgenic mice that have been "engineered" to produce human antibodies in response to antigenic challenge. In this technique, elements of the human heavy and light chain loci are introduced into strains of mice derived from embryonic stem cell lines that contain targeted disruptions of the endogenous heavy chain and light chain loci. The transgenic mice can synthesize human antibodies specific for various antigens, and the mice can be used to produce human antibody-secreting hybridomas. Methods for obtaining human antibodies from transgenic mice are described by Green et al., Nature Genet. 7:13 (1994), Lonberg et al., Nature 368:856 (1994), and Taylor et al., Int. Immun. 6:579 (1994). A fully human antibody also can be constructed by genetic or chromosomal transfection methods, as well as phage display technology, all of which are known in the art. See for example, McCafferty et al., Nature 348:552-553 (1990) for the production of human antibodies and fragments thereof *in vitro,* from immunoglobulin variable domain gene repertoires from unimmunized donors. In this technique, antibody variable domain genes are cloned in-frame into either a major or minor coat protein gene of a filamentous bacteriophage, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. In this way, the phage mimics some of the properties of the B cell. Phage display can be performed in a variety of formats, for their review, see e.g. Johnson and Chiswell, Current Opinion in Structural Biology 3:5564-571 (1993). Human antibodies may also be generated by *in vitro* activated B cells. See U.S. Patent Nos. 5,567,610 and 5,229,275.

A therapeutic agent is a molecule or atom that is administered separately, concurrently or sequentially with a binding moiety, e.g., an antibody or antibody fragment, and is useful in the treatment of a disease. Examples of therapeutic agents include, but are not limited to, antibodies, antibody fragments, conjugates, drugs, cytotoxic agents, proapoptotic agents, toxins, nucleases (including DNAses and RNAses), hormones, immunomodulators, chelators, boron compounds, photoactive agents or dyes, radioisotopes or radionuclides, oligonucleotides, interference RNA, peptides, anti-angiogenic agents, chemotherapeutic agents, cyokines, chemokines, prodrugs, enzymes, binding proteins or peptides or combinations thereof.

An immunoconjugate is an antibody, antibody fragment or other antibody moiety conjugated to a therapeutic agent. As used herein, the terms "conjugate" and "immunoconjugate" are used interchangeably.

As used herein, the term antibody fusion protein is a recombinantly-produced antigen-binding molecule in which one or more natural antibodies, single-chain antibodies or antibody fragments are linked to another moiety, such as a protein or peptide, a toxin, a cytokine, a hormone, etc. In certain preferred embodiments, the fusion protein may comprise two or more of the same or different antibodies, antibody fragments or single-chain antibodies fused together, which may bind to the same epitope, different epitopes on the same antigen, or different antigens.

An immunomodulator is a therapeutic agent that when present, alters, suppresses or stimulates the body's immune system. Typically, an immunomodulator of use stimulates immune cells to proliferate or become activated in an immune response cascade, such as macrophages, dendritic cells, B-cells, and/or T-cells. An example of an immunomodulator as described herein is a cytokine, which is a soluble small protein of approximately 5-20 kDa that is released by one cell population (e.g., primed T-lymphocytes) on contact with specific antigens, and which acts as an intercellular mediator between cells. As the skilled artisan will understand, examples of cytokines include lymphokines, monokines, interleukins, and several related signaling molecules, such as tumor necrosis factor (TNF) and interferons. Chemokines are a subset of cytokines. Certain interleukins and interferons are examples of cytokines that stimulate T cell or other immune cell proliferation.

CPT is an abbreviation for camptothecin. As used in the present application, CPT represents camptothecin itself or an analog or derivative of camptothecin. The structures of camptothecin and some of its analogs, with the numbering indicated and the rings labeled with letters A-E, are given in formula 1 in Chart 1 below.

### Chart 1

| | |
|---|---|
| 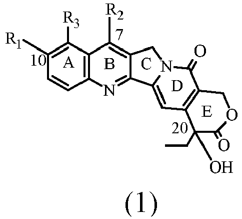 | CPT: R₁=R₂=R₃=H |
| | 10-Hydroxy-CPT: R₁ = OH; R₂ = R₃ = H |
| | CPT-11: R₁ = 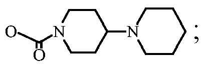 R₂ = ethyl; R₃ = H |
| | SN-38: R₁ = OH; R₂ = ethyl; R₃ = H |
| | Topotecan: R₁ = OH; R₂ = H; R₃ = CH₂-N(CH₃)₂ |

### Camptothecin Conjugates

Non-limiting methods and compositions for preparing immunoconjugates comprising a camptothecin therapeutic agent attached to an antibody or antigen-binding antibody fragment are described below. In preferred embodiments, the solubility of the drug is enhanced by placing a defined polyethyleneglycol (PEG) moiety (i.e., a PEG containing a defined number of monomeric units) between the drug and the antibody, wherein the defined PEG is a low molecular weight PEG, preferably containing 1-30 monomeric units, more preferably containing 1-12 monomeric units.

Preferably, a first linker connects the drug at one end and may terminate with an acetylene or an azide group at the other end. This first linker may comprise a defined PEG moiety with an azide or acetylene group at one end and a different reactive group, such as carboxylic acid or hydroxyl group, at the other end. Said bifunctional defined PEG may be attached to the amine group of an amino alcohol, and the hydroxyl group of the latter may be attached to the hydroxyl group on the drug in the form of a carbonate. Alternatively, the nonazide(or acetylene) moiety of said defined bifunctional PEG may be attached to the N-terminus of an L-amino acid or a polypeptide, with the C-terminus attached to the amino group of amino alcohol, and the hydroxy group of the latter may be attached to the hydroxyl group of the drug in the form of carbonate or carbamate, respectively.

A second linker, comprising an antibody-coupling group and a reactive group complementary to the azide (or acetylene) group of the first linker, namely acetylene (or azide), may react with the drug-(first linker) conjugate via acetylene-azide cycloaddition reaction to furnish a final bifunctional drug product that is useful for conjugating to diseasetargeting antibodies. The antibody-coupling group is preferably either a thiol or a thiolreactive group.

In the acetylene-azide 'click chemistry' coupling, a copper (+1)-catalyzed cycloaddition reaction occurs between an acetylene moiety and an azide moiety (Kolb HC and Sharpless KB, Drug Discov Today 2003; 8: 1128-37), although alternative forms of click chemistry are known and may be used. The reaction uses a mixture of cuprous bromide and triphenylphosphine to enable highly efficient coupling in non-polar organic solvents, such as dichloromethane. The advantage of click chemistry is that it is chemoselective, and complements other well-known conjugation chemistries such as the thiol-maleimide reaction. In the following discussion, where a conjugate comprises an antibody or antibody fragment, another type of binding moiety, such as a targeting peptide, may be substituted.

Methods for selective regeneration of the 10-hydroxyl group in the presence of the C-20 carbonate in preparations of drug-linker precursor involving CPT analogs such as SN-38 are provided below. Other protecting groups for reactive hydroxyl groups in drugs such as the phenolic hydroxyl in SN-38, for example t-butyldimethylsilyl or t-butyldiphenylsilyl, may also be used, and these may be deprotected by tetrabutylammonium fluoride prior to linking of the derivatized drug to an antibody-coupling moiety. The 10-hydroxyl group of CPT analogs is alternatively protected as an ester or carbonate, other than 'BOC', such that the bifunctional CPT is conjugated to an antibody without prior deprotection of this protecting group. The protecting group may be readily deprotected under physiological pH conditions after the bioconjugate is administered.

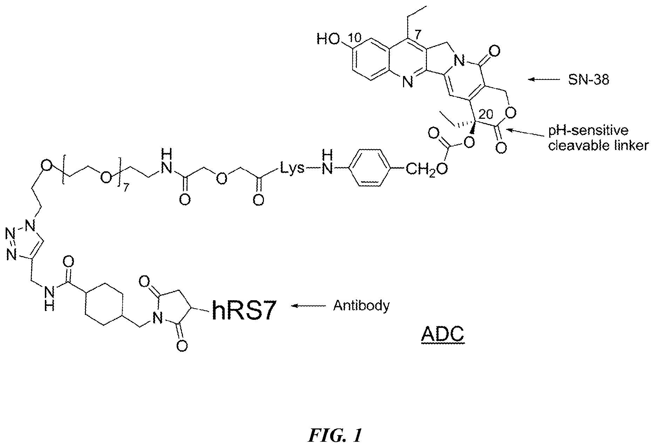

An exemplary embodiment of an ADC is shown in **FIG. 1****,** which illustrates the structure of hRS7 (anti-TROP-2) conjugated via the intracellularly cleavable CL2A linker to the SN-38 camptothecin.

The conjugates of antibodies and drugs may be purified by tangential flow filtration (TFF) method using a 50,000 Da molecular weight cut-off membrane using 25 to 30 diafiltration volumes of the conjugate formulation buffer for purifying hundreds of grams of the conjugates. This method obviates a need to employ expensive and cumbersome chromatographic purifications on size-exclusion and hydrophobic chromatography columns.

The conjugates may be formulated in Good's biological buffers at a pH of 6 to 7.0, and lyophilized for storage. Preferably, the Good's buffer is selected from the group consisting of 2-(N-morpholino)ethanesulfonic acid (MES), 3-(N-morpholino)propanesulfonic acid (MOPS), 4-(2-hydroxyethyl)piperazine-1-ethanesulfonic acid (HEPES), and 1,4-piperazinediethanesulfonic acid (PIPES), in the pH range of 6-7, preferably in the pH range of 6.5 to 7, and at a buffer concentration of 10-100 mM, preferably 25 mM. The most preferred formulation buffer is 25 mM MES, pH 6.5.

The purified conjugates may be combined with excipients such as trehalose and polysorbate 80, lyophilized, and stored as lyophilates in the temperature range of -20 °C to 8°C.

### Anthracycline Conjugates

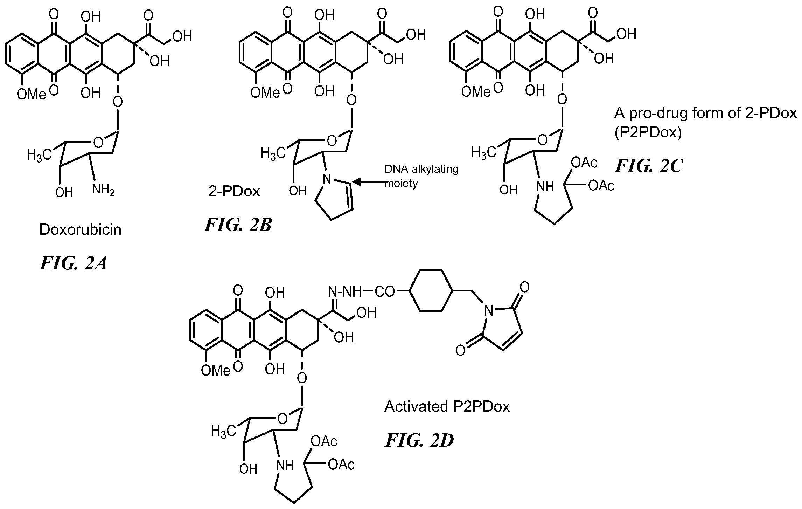

**FIG. 2** shows an exemplary anthracycline, pro-2-pyrrolinodoxorubicin (P2PDox), of use for conjugation to form ADCs. The parent compound, 2-pyrrolinodoxorubicin, was described first in 1996 by Schally's group, who later used it for conjugating to a number of receptor-targeted peptides for preclinical explorations (Nagy et al., 1996a, Proc Natl Acad Sci U S A 93:7269-73; Nagy et al., 1996b, Proc Natl Acad Sci U S A 93:2464-9; Nagy et al., 1997, Proc Natl Acad Sci U S A 94:652-6; Nagy et al., 1998, Proc Natl Acad Sci U S A 95:1794-9). This is a derivative of doxorubicin, with the daunosamine nitrogen incorporated into a 5-membered enamine, making it a highly potent alkylating agent, with cytotoxicity 500-1000 times that of doxorubicin. The drug's ultratoxicity necessitates special handling in isolators, for safety.

A prodrug form of 2-pyrrolinodoxorubicin was investigated by another group, who disclosed a derivative of doxorubicin, namely *N*-(4,4-diacetoxybutyl)doxorubicin (Farquhar et al., 1998, J Med Chem 41:965-72; U.S. Patent Nos. 5,196,522; 6,433,150), which is convertible to 2-pyrrolinodoxorubicin *in vivo.* This derivative was prepared by reductive alkylation of doxorubicin with 4,4-diacetoxybutyraldehyde. However, this prodrug was not attached to an antibody or other targeting molecule using an acid-labile group, such as hydrazone, as the cleavable linker, at the thiols of disulfide-reduced antibodies.

Various of the Examples below use P2PDox as the drug in the ADC for neoadjuvant use. There are several advantages to this: (i) handling only the prodrug, thereby mitigating safety concerns; (ii) the raw material doxorubicin (Dox) is available in quantity in the cGMP grade; and (iii) the chemistry of converting Dox to activated P2PDox (P2PDox) involves only a few synthetic steps. **FIG. 2A-2D** shows the structures of Dox, 2-PDox, P2PDox (P2PDox), and activated P2PDox. For coupling to IgG, we activated P2PDox with SMCC-hydrazide, a procedure that introduces acid-labile hydrazone as well as the maleimide group, the latter for conjugation to thiols of mildly reduced antibody.

The choice of 2-pyrrolinodoxorubicin as one of the drugs for ADC neoadjuvant use, particularly its prodrug form for conjugation to MAbs, enables rapid immunoconjugate development, as the raw material doxorubicin is readily available in the cGMP grade. The ketone on P2PDox provides a handle to incorporate acid-labile hydrazone and antibodybinding maleimide groups in a single step. The derivatization of the amino group of the doxorubicin in the 2-PDox version should overcome multi-drug resistance associated with doxorubicin, based on literature precedents (Farquhar et al., 1998, 41:965-72; Guillemard & Uri, 2004, Oncogene 23:3613-21). The design provides an option to add hydrophilic groups into the linker or *N*-alkyl portion, if desired, for radiolabeling purpose and/or further modulating administrable dose, without affecting the active 2-pyrrolinodoxorubicin structure that is generated *in vivo.*

Most of the ADCs currently being examined by others incorporate tubulin-acting, ultratoxic, maytansinoids and auristatins, which are cell-cycle-phase-specific. Anecdotally, except for trastuzumab-DM1, these ADCs appear to exhibit a relatively narrow therapeutic index clinically in solid cancers. A DNA-alkylating agent, such as 2-PDox, is cell-cycle-*phase-nonspecific.* The proposed ADC, based on a drug component that acts by a different mechanism of cell-killing, an internalizing antibody that shows greater cancer specificity than many others, such as EpCAM MAbs, and the chemistry of linking, offers a departure from other ultratoxic ADCs, and provides an improved therapeutic index. As shown below, preclinical studies conducted to date in aggressive xenograft models of pancreatic, breast, and gastric cancers show the hRS7-P2PDox conjugate to be very active at low and safe doses, leading to complete regressions. Studies in mice bearing human hematological and solid tumors treated with a variety of antibodies targeting such tumors and conjugated with P2PDox also show excellent tumor control (retardation or regression of growth, as compared to control groups), even at infrequent doses, with dose-limiting toxicities due mostly to neutropenia, which is controlled by adjusting the doses to be lower than the maximal tolerated dose (MTD), usually a dose that results in 5% or less mortality. The studies support neoadjuvant use of antibody-P2PDox conjugates.

### General Antibody Techniques

Techniques for preparing monoclonal antibodies against virtually any target antigen are well known in the art. *See,* for example, Kohler and Milstein, Nature 256: 495 (1975), and Coligan et al. (eds.), CURRENT PROTOCOLS IN IMMUNOLOGY, VOL. 1, pages 2.5.1-2.6.7 (John Wiley & Sons 1991). Briefly, monoclonal antibodies can be obtained by injecting mice with a composition comprising an antigen, removing the spleen to obtain B-lymphocytes, fusing the B-lymphocytes with myeloma cells to produce hybridomas, cloning the hybridomas, selecting positive clones which produce antibodies to the antigen, culturing the clones that produce antibodies to the antigen, and isolating the antibodies from the hybridoma cultures.

Various techniques, such as production of chimeric or humanized antibodies, may involve procedures of antibody cloning and construction. The antigen-binding Vκ (variable light chain) and V_{H} (variable heavy chain) sequences for an antibody of interest may be obtained by a variety of molecular cloning procedures, such as RT-PCR, 5'-RACE, and cDNA library screening. The V genes of an antibody from a cell that expresses a murine antibody can be cloned by PCR amplification and sequenced. To confirm their authenticity, the cloned V_{L} and V_{H} genes can be expressed in cell culture as a chimeric Ab as described by Orlandi et al., (Proc. Natl. Acad. Sci., USA, 86: 3833 (1989)). Based on the V gene sequences, a humanized antibody can then be designed and constructed as described by Leung et al. (Mol. Immunol., 32: 1413 (1995)).

cDNA can be prepared from any known hybridoma line or transfected cell line producing a murine antibody by general molecular cloning techniques (Sambrook et al., Molecular Cloning, A laboratory manual, 2nd Ed (1989)). The Vκ sequence for the antibody may be amplified using the primers VK1BACK and VK1FOR (Orlandi *et al.,* 1989) or the extended primer set described by Leung et al. (BioTechniques, 15: 286 (1993)). The V_{H} sequences can be amplified using the primer pair VH1BACK/VH1FOR (Orlandi *et al.,* 1989) or the primers annealing to the constant region of murine IgG described by Leung et al. (Hybridoma, 13:469 (1994)). Humanized V genes can be constructed by a combination of long oligonucleotide template syntheses and PCR amplification as described by Leung et al. (Mol. Immunol., 32: 1413 (1995)).

PCR products for Vκ can be subcloned into a staging vector, such as a pBR327-based staging vector, VKpBR, that contains an Ig promoter, a signal peptide sequence and convenient restriction sites. PCR products for V_{H} can be subcloned into a similar staging vector, such as the pBluescript-based VHpBS. Expression cassettes containing the Vκ and V_{H} sequences together with the promoter and signal peptide sequences can be excised from VKpBR and VHpBS and ligated into appropriate expression vectors, such as pKh and pG1g, respectively (Leung et al., Hybridoma, 13:469 (1994)). The expression vectors can be co-transfected into an appropriate cell and supernatant fluids monitored for production of a chimeric, humanized or human antibody. Alternatively, the Vκ and V_{H} expression cassettes can be excised and subcloned into a single expression vector, such as pdHL2, as described by Gillies et al. (J. Immunol. Methods 125:191 (1989) and also shown in Losman et al., Cancer, 80:2660 (1997)).

In an alternative embodiment, expression vectors may be transfected into host cells that have been pre-adapted for transfection, growth and expression in serum-free medium. Exemplary cell lines that may be used include the Sp/EEE, Sp/ESF and Sp/ESF-X cell lines (see, e.g., U.S. Patent Nos. 7,531,327; 7,537,930 and 7,608,425). These exemplary cell lines are based on the Sp2/0 myeloma cell line, transfected with a mutant Bcl-EEE gene, exposed to methotrexate to amplify transfected gene sequences and pre-adapted to serum-free cell line for protein expression.

### Chimeric and Humanized Antibodies

A chimeric antibody is a recombinant protein in which the variable regions of a human antibody have been replaced by the variable regions of, for example, a mouse antibody, including the complementarity-determining regions (CDRs) of the mouse antibody. Chimeric antibodies exhibit decreased immunogenicity and increased stability when administered to a subject. Methods for constructing chimeric antibodies are well known in the art (*e.g.,* Leung et al., 1994, Hybridoma 13:469).

A chimeric monoclonal antibody may be humanized by transferring the mouse CDRs from the heavy and light variable chains of the mouse immunoglobulin into the corresponding variable domains of a human antibody. The mouse framework regions (FR) in the chimeric monoclonal antibody are also replaced with human FR sequences. To preserve the stability and antigen specificity of the humanized monoclonal, one or more human FR residues may be replaced by the mouse counterpart residues. Humanized monoclonal antibodies may be used for therapeutic treatment of subjects. Techniques for production of humanized monoclonal antibodies are well known in the art. (See, *e.g.,* Jones et al., 1986, Nature, 321:522; Riechmann et al., Nature, 1988, 332:323; Verhoeyen et al., 1988, Science, 239:1534; Carter et al., 1992, Proc. Nat'l Acad. Sci. USA, 89:4285; Sandhu, Crit. Rev. Biotech., 1992, 12:437; Tempest et al., 1991, Biotechnology 9:266; Singer et al., J. Immun., 1993, 150:2844.)

Other examples may concern non-human primate antibodies. General techniques for raising therapeutically useful antibodies in baboons may be found, for example, in Goldenberg et al., WO 91/11465 (1991), and in Losman et al., Int. J. Cancer 46: 310 (1990). In another embodiment, an antibody may be a human monoclonal antibody. Such antibodies may be obtained from transgenic mice that have been engineered to produce specific human antibodies in response to antigenic challenge, as discussed below.

### Human Antibodies

Methods for producing fully human antibodies using either combinatorial approaches or transgenic animals transformed with human immunoglobulin loci are known in the art *(e.g.,* Mancini et al., 2004, New Microbiol. 27:315-28; Conrad and Scheller, 2005, Comb. Chem. High Throughput Screen. 8:117-26; Brekke and Loset, 2003, Curr. Opin. Phamacol. 3:544-50). Such fully human antibodies are expected to exhibit even fewer side effects than chimeric or humanized antibodies and to function *in vivo* as essentially endogenous human antibodies. In certain examples, the disclosed methods and procedures may utilize human antibodies produced by such techniques.

In one alternative, the phage display technique may be used to generate human antibodies *(e.g.,* Dantas-Barbosa et al., 2005, Genet. Mol. Res. 4:126-40). Human antibodies may be generated from normal humans or from humans that exhibit a particular disease state, such as cancer (Dantas-Barbosa et al., 2005). The advantage to constructing human antibodies from a diseased individual is that the circulating antibody repertoire may be biased towards antibodies against disease-associated antigens.

In one non-limiting example of this methodology, Dantas-Barbosa et al. (2005) constructed a phage display library of human Fab antibody fragments from osteosarcoma patients. Generally, total RNA was obtained from circulating blood lymphocytes (*Id*.). Recombinant Fab were cloned from the µ, γ and κ chain antibody repertoires and inserted into a phage display library (*Id*.) RNAs were converted to cDNAs and used to make Fab cDNA libraries using specific primers against the heavy and light chain immunoglobulin sequences (Marks et al., 1991, J. Mol. Biol. 222:581-97). Library construction was performed according to Andris-Widhopf et al. (2000, In: Phage Display Laboratory Manual, Barbas et al. (eds), 1st edition, Cold Spring Harbor Laboratory Press, Cold Spring Harbor, NY pp. 9.1 to 9.22). The final Fab fragments were digested with restriction endonucleases and inserted into the bacteriophage genome to make the phage display library. Such libraries may be screened by standard phage display methods. The skilled artisan will realize that this technique is exemplary only and any known method for making and screening human antibodies or antibody fragments by phage display may be utilized.

In another alternative, transgenic animals that have been genetically engineered to produce human antibodies may be used to generate antibodies against essentially any immunogenic target, using standard immunization protocols as discussed above. Methods for obtaining human antibodies from transgenic mice are described by Green et al., Nature Genet. 7:13 (1994), Lonberg et al., Nature 368:856 (1994), and Taylor et al., Int. Immun. 6:579 (1994). A non-limiting example of such a system is the XenoMouse® (e.g., Green et al., 1999, J. Immunol. Methods 231:11-23) from Abgenix (Fremont, CA). In the XenoMouse® and similar animals, the mouse antibody genes have been inactivated and replaced by functional human antibody genes, while the remainder of the mouse immune system remains intact.

The XenoMouse® was transformed with germline-configured YACs (yeast artificial chromosomes) that contained portions of the human IgH and Ig kappa loci, including the majority of the variable region sequences, along accessory genes and regulatory sequences. The human variable region repertoire may be used to generate antibody producing B cells, which may be processed into hybridomas by known techniques. A XenoMouse® immunized with a target antigen will produce human antibodies by the normal immune response, which may be harvested and/or produced by standard techniques discussed above. A variety of strains of XenoMouse® are available, each of which is capable of producing a different class of antibody. Transgenically produced human antibodies have been shown to have therapeutic potential, while retaining the pharmacokinetic properties of normal human antibodies (Green et al., 1999). The skilled artisan will realize that the claimed compositions and methods are not limited to use of the XenoMouse® system but may utilize any transgenic animal that has been genetically engineered to produce human antibodies.

### Production of Antibody Fragments

Antibody fragments may be obtained, for example, by pepsin or papain digestion of whole antibodies by conventional methods. For example, antibody fragments may be produced by enzymatic cleavage of antibodies with pepsin to provide a 5S fragment denoted F(ab')₂. This fragment may be further cleaved using a thiol reducing agent and, optionally, a blocking group for the sulfhydryl groups resulting from cleavage of disulfide linkages, to produce 3.5S Fab' monovalent fragments. Alternatively, an enzymatic cleavage using pepsin produces two monovalent Fab fragments and an Fc fragment. Exemplary methods for producing antibody fragments are disclosed in U.S. Pat. No. 4,036,945; U.S. Pat. No. 4,331,647; Nisonoff et al., 1960, Arch Biochem Biophys, 89:230; Porter, 1959, Biochem. J., 73:119; Edelman et al., 1967, METHODS IN ENZYMOLOGY, page 422 (Academic Press), and Coligan et al. (eds.), 1991, CURRENT PROTOCOLS IN IMMUNOLOGY, (John Wiley & Sons).

Other methods of cleaving antibodies, such as separation of heavy chains to form monovalent light-heavy chain fragments, further cleavage of fragments or other enzymatic, chemical or genetic techniques also may be used, so long as the fragments bind to the antigen that is recognized by the intact antibody. For example, Fv fragments comprise an association of V_{H} and V_{L} chains. This association can be noncovalent, as described in Inbar et al., 1972, Proc. Nat'l. Acad. Sci. USA, 69:2659. Alternatively, the variable chains may be linked by an intermolecular disulfide bond or cross-linked by chemicals such as glutaraldehyde. See Sandhu, 1992, Crit. Rev. Biotech., 12:437.

Preferably, the Fv fragments comprise V_{H} and V_{L} chains connected by a peptide linker. These single-chain antigen binding proteins (scFv) are prepared by constructing a structural gene comprising DNA sequences encoding the V_{H} and V_{L} domains, connected by an oligonucleotides linker sequence. The structural gene is inserted into an expression vector that is subsequently introduced into a host cell, such as E. coli. The recombinant host cells synthesize a single polypeptide chain with a linker peptide bridging the two V domains. Methods for producing scFvs are well-known in the art. See Whitlow et al., 1991, Methods: A Companion to Methods in Enzymology 2:97; Bird et al., 1988, Science, 242:423; U.S. Pat. No. 4,946,778; Pack et al., 1993, Bio/Technology, 11:1271, and Sandhu, 1992, Crit. Rev. Biotech., 12:437.

Another form of an antibody fragment is a single-domain antibody (dAb), sometimes referred to as a single chain antibody. Techniques for producing single-domain antibodies are well known in the art (see, e.g., Cossins et al., Protein Expression and Purification, 2007, 51:253-59; Shuntao et al., Molec Immunol 06, 43:1912-19; Tanha et al., J. Biol. Chem. 2001, 276:24774-780). Other types of antibody fragments may comprise one or more complementarity-determining regions (CDRs). CDR peptides ("minimal recognition units") can be obtained by constructing genes encoding the CDR of an antibody of interest. Such genes are prepared, for example, by using the polymerase chain reaction to synthesize the variable region from RNA of antibody-producing cells. See Larrick et al., 1991, Methods: A Companion to Methods in Enzymology 2:106; Ritter et al. (eds.), 1995, MONOCLONAL ANTIBODIES: PRODUCTION, ENGINEERING AND CLINICAL APPLICATION, pages 166-179 (Cambridge University Press); Birch et al., (eds.), 1995, MONOCLONAL ANTIBODIES: PRINCIPLES AND APPLICATIONS, pages 137-185 (Wiley-Liss, Inc.)

### Antibody Variations

In certain embodiments of the invention as defined in the claims, the sequences of antibodies, such as the Fc portions of antibodies, may be varied to optimize the physiological characteristics of the conjugates, such as the half-life in serum. Methods of substituting amino acid sequences in proteins are widely known in the art, such as by site-directed mutagenesis (e.g. Sambrook et al., Molecular Cloning, A laboratory manual, 2nd Ed, 1989). In preferred embodiments, the variation may involve the addition or removal of one or more glycosylation sites in the Fc sequence (e.g., U.S. Patent No. 6,254,868). In other preferred embodiments, specific amino acid substitutions in the Fc sequence may be made (e.g., Hornick et al., 2000, J Nucl Med 41:355-62; Hinton et al., 2006, J Immunol 176:346-56; Petkova et al. 2006, Int Immunol 18:1759-69; U.S. Patent No. 7,217,797).

### Antibody Allotypes

Immunogenicity of therapeutic antibodies is associated with increased risk of infusion reactions and decreased duration of therapeutic response (Baert et al., 2003, N Engl J Med 348:602-08). The extent to which therapeutic antibodies induce an immune response in the host may be determined in part by the allotype of the antibody (Stickler et al., 2011, Genes and Immunity 12:213-21). Antibody allotype is related to amino acid sequence variations at specific locations in the constant region sequences of the antibody. The allotypes of IgG antibodies containing a heavy chain γ-type constant region are designated as Gm allotypes (1976, J Immunol 117:1056-59).

For the common IgG1 human antibodies, the most prevalent allotype is G1m1 (Stickler et al., 2011, Genes and Immunity 12:213-21). However, the G1m3 allotype also occurs frequently in Caucasians (Stickler et al., 2011). It has been reported that G1m1 antibodies contain allotypic sequences that tend to induce an immune response when administered to non-G1m1 (nG1m1) recipients, such as G1m3 patients (Stickler et al., 2011). Non-Glml allotype antibodies are not as immunogenic when administered to G1m1 patients (Stickler et al., 2011).

The human G1m1 allotype comprises the amino acids aspartic acid at Kabat position 356 and leucine at Kabat position 358 in the CH3 sequence of the heavy chain IgG1. The nG1m1 allotype comprises the amino acids glutamic acid at Kabat position 356 and methionine at Kabat position 358. Both G1ml and nG1ml allotypes comprise a glutamic acid residue at Kabat position 357 and the allotypes are sometimes referred to as DEL and EEM allotypes. A non-limiting example of the heavy chain constant region sequences for G1m1 and nG1m1 allotype antibodies is shown below for the exemplary antibodies rituximab (SEQ ID NO:1) and veltuzumab (SEQ ID NO:2).
*Rituximab heavy chain variable region sequence (SEQ ID NO:1)*
*Veltuzumab heavy chain variable region (SEQ ID NO:2)*

Jefferis and Lefranc (2009, mAbs 1:1-7) reviewed sequence variations characteristic of IgG allotypes and their effect on immunogenicity. They reported that the G1m3 allotype is characterized by an arginine residue at Kabat position 214, compared to a lysine residue at Kabat 214 in the G1m17 allotype. The nG1m1,2 allotype was characterized by glutamic acid at Kabat position 356, methionine at Kabat position 358 and alanine at Kabat position 431. The G1m1,2 allotype was characterized by aspartic acid at Kabat position 356, leucine at Kabat position 358 and glycine at Kabat position 431. In addition to heavy chain constant region sequence variants, Jefferis and Lefranc (2009) reported allotypic variants in the kappa light chain constant region, with the Km1 allotype characterized by valine at Kabat position 153 and leucine at Kabat position 191, the Km1,2 allotype by alanine at Kabat position 153 and leucine at Kabat position 191, and the Km3 allotypoe characterized by alanine at Kabat position 153 and valine at Kabat position 191.

With regard to therapeutic antibodies, veltuzumab and rituximab are, respectively, humanized and chimeric IgG1 antibodies against CD20, of use for therapy of a wide variety of hematological malignancies. **Table 1** compares the allotype sequences of rituximab vs. veltuzumab. As shown in **Table 1,** rituximab (G1m17,1) is a DEL allotype IgG1, with an additional sequence variation at Kabat position 214 (heavy chain CH1) of lysine in rituximab vs. arginine in veltuzumab. It has been reported that veltuzumab is less immunogenic in subjects than rituximab (*see, e.g.,* Morchhauser et al., 2009, J Clin Oncol 27:3346-53; Goldenberg et al., 2009, Blood 113:1062-70; Robak & Robak, 2011, BioDrugs 25:13-25), an effect that has been attributed to the difference between humanized and chimeric antibodies. However, the difference in allotypes between the EEM and DEL allotypes likely also accounts for the lower immunogenicity of veltuzumab.

**Table 1. Allotypes of Rituximab vs. Veltuzumab**

| | | *Heavy chain position and associated allotypes* | | | | | |
|---|---|---|---|---|---|---|---|
| | *Complete allotype* | *214* | *(allotype)* | *356*/*358* | *(allotype)* | *431* | *(allotype)* |
| *Rituximab* | *G1m17,1* | *K* | 17 | *D*/*L* | *1* | *A* | - |
| *Veltuzumab* | *G1m3* | *R* | *3* | *E*/*M* | - | *A* | - |

In order to reduce the immunogenicity of therapeutic antibodies in individuals of nG1m1 genotype, it is desirable to select the allotype of the antibody to correspond to the G1m3 allotype, characterized by arginine at Kabat 214, and the nG1m1,2 null-allotype, characterized by glutamic acid at Kabat position 356, methionine at Kabat position 358 and alanine at Kabat position 431. Surprisingly, it was found that repeated subcutaneous administration of G1m3 antibodies over a long period of time did not result in a significant immune response. In alternative embodiments, the human IgG4 heavy chain in common with the G1m3 allotype has arginine at Kabat 214, glutamic acid at Kabat 356, methionine at Kabat 359 and alanine at Kabat 431. Since immunogenicity appears to relate at least in part to the residues at those locations, use of the human IgG4 heavy chain constant region sequence for therapeutic antibodies is also a preferred embodiment. Combinations of G1m3 IgG1 antibodies with IgG4 antibodies may also be of use for therapeutic administration.

### Known Antibodies

In various embodiments of the invention as defined in the claims, a variety of antibodies known in the art may be used. Antibodies of use may be commercially obtained from a number of known sources. For example, a variety of antibody secreting hybridoma lines are available from the American Type Culture Collection (ATCC, Manassas, VA). Irrespective thereof, a large number of antibodies against various disease targets, including but not limited to tumor-associated antigens, have been deposited at the ATCC and/or have published variable region sequences and are available for use in the claimed methods and compositions. See, e.g., U.S. Patent Nos. 7,312,318; 7,282,567; 7,151,164; 7,074,403; 7,060,802; 7,056,509; 7,049,060; 7,045,132; 7,041,803; 7,041,802; 7,041,293; 7,038,018; 7,037,498; 7,012,133; 7,001,598; 6,998,468; 6,994,976; 6,994,852; 6,989,241; 6,974,863; 6,965,018; 6,964,854; 6,962,981; 6,962,813; 6,956,107; 6,951,924; 6,949,244; 6,946,129; 6,943,020; 6,939,547; 6,921,645; 6,921,645; 6,921,533; 6,919,433; 6,919,078; 6,916,475; 6,905,681; 6,899,879; 6,893,625; 6,887,468; 6,887,466; 6,884,594; 6,881,405; 6,878,812; 6,875,580; 6,872,568; 6,867,006; 6,864,062; 6,861,511; 6,861,227; 6,861,226; 6,838,282; 6,835,549; 6,835,370; 6,824,780; 6,824,778; 6,812,206; 6,793,924; 6,783,758; 6,770,450; 6,767,711; 6,764,688; 6,764,681; 6,764,679; 6,743,898; 6,733,981; 6,730,307; 6,720,155; 6,716,966; 6,709,653; 6,693,176; 6,692,908; 6,689,607; 6,689,362; 6,689,355; 6,682,737; 6,682,736; 6,682,734; 6,673,344; 6,653,104; 6,652,852; 6,635,482; 6,630,144; 6,610,833; 6,610,294; 6,605,441; 6,605,279; 6,596,852; 6,592,868; 6,576,745; 6,572;856; 6,566,076; 6,562,618; 6,545,130; 6,544,749; 6,534,058; 6,528,625; 6,528,269; 6,521,227; 6,518,404; 6,511,665; 6,491,915; 6,488,930; 6,482,598; 6,482,408; 6,479,247; 6,468,531; 6,468,529; 6,465,173; 6,461,823; 6,458,356; 6,455,044; 6,455,040, 6,451,310; 6,444,206; 6,441,143; 6,432,404; 6,432,402; 6,419,928; 6,413,726; 6,406,694; 6,403,770; 6,403,091; 6,395,276; 6,395,274; 6,387,350; 6,383,759; 6,383,484; 6,376,654; 6,372,215; 6,359,126; 6,355,481; 6,355,444; 6,355,245; 6,355,244; 6,346,246; 6,344,198; 6,340,571; 6,340,459; 6,331,175; 6,306,393; 6,254,868; 6,187,287; 6,183,744; 6,129,914; 6,120,767; 6,096,289; 6,077,499; 5,922,302; 5,874,540; 5,814,440; 5,798,229; 5,789,554; 5,776,456; 5,736,119; 5,716,595; 5,677,136; 5,587,459; 5,443,953; 5,525,338. These are exemplary only and a wide variety of other antibodies and their hybridomas are known in the art. The skilled artisan will realize that antibody sequences or antibody-secreting hybridomas against almost any disease-associated antigen may be obtained by a simple search of the ATCC, NCBI and/or USPTO databases for antibodies against a selected disease-associated target of interest. The antigen binding domains of the cloned antibodies may be amplified, excised, ligated into an expression vector, transfected into an adapted host cell and used for protein production, using standard techniques well known in the art (see, e.g., U.S. Patent Nos. 7,531,327; 7,537,930; 7,608,425 and 7,785,880).

Particular antibodies that may be of use within the instant disclosure methods and compositions include, but are not limited to, LL1 (anti-CD74), LL2 or RFB4 (anti-CD22), veltuzumab (hA20, anti-CD20), rituxumab (anti-CD20), obinutuzumab (GA101, anti-CD20), lambrolizumab (anti-PD-1 receptor), nivolumab (anti-PD-1 receptor), ipilimumab (anti-CTLA-4), , PAM4 or KC4 (both anti-mucin), MN-14 (anti-carcinoembryonic antigen (CEA, also known as CD66e or CEACAM5), MN-15 or MN-3 (anti-CEACAM6), Mu-9 (anti-colon-specific antigen-p), Immu 31 (an anti-alpha-fetoprotein), R1 (anti-IGF-1R), A19 (anti-CD19), TAG-72 (e.g., CC49), Tn, J591 or HuJ591 (anti-PSMA (prostate-specific membrane antigen)), AB-PG1-XG1-026 (anti-PSMA dimer), D2/B (anti-PSMA), G250 (an anti-carbonic anhydrase IX MAb), L243 (anti-HLA-DR) alemtuzumab (anti-CD52), bevacizumab (anti-VEGF), cetuximab (anti-EGFR), gemtuzumab (anti-CD33), ibritumomab tiuxetan (anti-CD20); panitumumab (anti-EGFR); tositumomab (anti-CD20); PAM4 (aka clivatuzumab, anti-mucin) and trastuzumab (anti-ErbB2). Such antibodies are known in the art (e.g., U.S. Patent Nos. 5,686,072; 5,874,540; 6,107,090; 6,183,744; 6,306,393; 6,653,104; 6,730.300; 6,899,864; 6,926,893; 6,962,702; 7,074,403; 7,230,084; 7,238,785; 7,238,786; 7,256,004; 7,282,567; 7,300,655; 7,312,318; 7,585,491; 7,612,180; 7,642,239; and U.S. Patent Application Publ. No. 20050271671; 20060193865; 20060210475; 20070087001. Specific known antibodies of use include hPAM4 (U.S. Patent No. 7,282,567), hA20 (U.S. Patent No. 7,151,164), hA19 (U.S. Patent No. 7,109,304), hIMMU-31 (U.S. Patent No. 7,300,655), hLL1 (U.S. Patent No. 7,312,318,), hLL2 (U.S. Patent No. 5,789,554), hMu-9 (U.S. Patent No. 7,387,772), hL243 (U.S. Patent No. 7,612,180), hMN-14 (U.S. Patent No. 6,676,924), hMN-15 (U.S. Patent No. 8,287,865), hR1 (U.S. Patent Application 12/772,645), hRS7 (anti-epithelial glycoprotein-1 (EGP-1, also known as TROP-2) (U.S. Patent No. 7,238,785), hMN-3 (U.S. Patent No. 7,541,440), AB-PG1-XG1-026 (U.S. Patent Application 11/983,372, deposited as ATCC PTA-4405 and PTA-4406) and D2/B (WO 2009/130575).

Other useful antigens that may be targeted using the described conjugates include carbonic anhydrase IX, alpha-fetoprotein (AFP), α-actinin-4, A3, antigen specific for A33 antibody, ART-4, B7, Ba 733, BAGE, BrE3-antigen, CA125, CAMEL, CAP-1, CASP-8/m, CCL19, CCL21, CD1, CD1a, CD2, CD3, CD4, CD5, CD8, CD11A, CD14, CD15, CD16, CD18, CD19, CD20, CD21, CD22, CD23, CD25, CD29, CD30, CD32b, CD33, CD37, CD38, CD40, CD40L, CD44, CD45, CD46, CD52, CD54, CD55, CD59, CD64, CD66a-e, CD67, CD70, CD70L, CD74, CD79a, CD80, CD83, CD95, CD126, CD132, CD133, CD138, CD147, CD154, CDC27, CDK-4/m, CDKN2A, CTLA-4, CXCR4, CXCR7, CXCL12, HIF-1α, colon-specific antigen-p (CSAp), CEACAM5, CEACAM6, c-Met, DAM, EGFR, EGFRvIII, EGP-1 (TROP-2), EGP-2, ELF2-M, Ep-CAM, fibroblast growth factor (FGF), Flt-1, Flt-3, folate receptor, G250 antigen, GAGE, gp100, GRO-β, HLA-DR, HM1.24, human chorionic gonadotropin (HCG) and its subunits, HER2/neu, HMGB-1, hypoxia inducible factor (HIF-1), HSP70-2M, HST-2, la, IGF-1R, IFN-γ, IFN-α, IFN-β, IFN-λ, IL-4R, IL-6R, IL-13R, IL-15R, IL-17R, IL-18R, IL-2, IL-6, IL-8, IL-12, IL-15, IL-17, IL-18, IL-23, IL-25, insulin-like growth factor-1 (IGF-1), KC4-antigen, KS-1-antigen, KS1-4, Le-Y, LDR/FUT, macrophage migration inhibitory factor (MIF), MAGE, MAGE-3, MART-1, MART-2, NY-ESO-1, TRAG-3, mCRP, MCP-1, MIP-1A, MIP-1B, MIF, MUC1, MUC2, MUC3, MUC4, MUC5ac, MUC13, MUC16, MUM-1/2, MUM-3, NCA66, NCA95, NCA90, PAM4 antigen, pancreatic cancer mucin, PD-1, PD-L1, PD-1 receptor, placental growth factor, p53, PLAGL2, prostatic acid phosphatase, PSA, PRAME, PSMA, P1GF, ILGF, ILGF-1R, IL-6, IL-25, RS5, RANTES, T101, SAGE, S100, survivin, survivin-2B, TAC, TAG-72, tenascin, TRAIL receptors, TNF-α, Tn antigen, Thomson-Friedenreich antigens, tumor necrosis antigens, VEGFR, ED-B fibronectin, WT-1, 17-1A-antigen, complement factors C3, C3a, C3b, C5a, C5, an angiogenesis marker, bcl-2, bcl-6, Kras, an oncogene marker and an oncogene product (see, e.g., Sensi et al., Clin Cancer Res 2006, 12:5023-32; Parmiani et al., J Immunol 2007, 178:1975-79; Novellino et al. Cancer Immunol Immunother 2005, 54:187-207).

A comprehensive analysis of suitable antigen (Cluster Designation, or CD) targets on hematopoietic malignant cells, as shown by flow cytometry and which can be a guide to selecting suitable antibodies for drug-conjugated immunotherapy, is Craig and Foon, Blood prepublished online January 15, 2008; DOL 10.1182/blood-2007-11-120535.

The CD66 antigens consist of five different glycoproteins with similar structures, CD66a-e, encoded by the carcinoembryonic antigen (CEA) gene family members, BCG, CGM6, NCA, CGM1 and CEA, respectively. These CD66 antigens (e.g., CEACAM6) are expressed mainly in granulocytes, normal epithelial cells of the digestive tract and tumor cells of various tissues. Also included as suitable targets for cancers are cancer testis antigens, such as NY-ESO-1 (Theurillat et al., Int. J. Cancer 2007; 120(11):2411-7), as well as CD79a in myeloid leukemia (Kozlov et al., Cancer Genet. Cytogenet. 2005; 163(1):62-7) and also B-cell diseases, and CD79b for non-Hodgkin's lymphoma (Poison et al., Blood 110(2):616-623). A number of the aforementioned antigens are disclosed in U.S. Provisional Application Serial No. 60/426,379, entitled "Use of Multi-specific, Non-covalent Complexes for Targeted Delivery of Therapeutics," filed November 15, 2002. Cancer stem cells, which are ascribed to be more therapy-resistant precursor malignant cell populations (Hill and Perris, J. Natl. Cancer Inst. 2007; 99:1435-40), have antigens that can be targeted in certain cancer types, such as CD133 in prostate cancer (Maitland et al., Ernst Schering Found. Sympos. Proc. 2006; 5:155-79), non-small-cell lung cancer (Donnenberg et al., J. Control Release 2007; 122(3):385-91), and glioblastoma (Beier et al., Cancer Res. 2007; 67(9):4010-5), and CD44 in colorectal cancer (Dalerba er al., Proc. Natl. Acad. Sci. USA 2007; 104(24)10158-63), pancreatic cancer (Li et al., Cancer Res. 2007; 67(3): 1030-7), and in head and neck squamous cell carcinoma (Prince et al., Proc. Natl. Acad. Sci. USA 2007; 104(3)973-8). Another useful target for breast cancer therapy is the LIV-1 antigen described by Taylor et al. (Biochem. J. 2003; 375:51-9).

For multiple myeloma therapy, suitable targeting antibodies have been described against, for example, CD38 and CD138 (Stevenson, Mol Med 2006; 12(11-12):345-346; Tassone et al., Blood 2004; 104(12):3688-96), CD74 (Stein et al., *ibid.*)*,* CS1 (Tai et al., Blood 2008; 112(4):1329-37, and CD40 (Tai et al., 2005; Cancer Res. 65(13):5898-5906).

Macrophage migration inhibitory factor (MIF) is an important regulator of innate and adaptive immunity and apoptosis. It has been reported that CD74 is the endogenous receptor for MIF (Leng et al., 2003, JExp Med 197:1467-76). The therapeutic effect of antagonistic anti-CD74 antibodies on MIF-mediated intracellular pathways may be of use for treatment of a broad range of disease states, such as cancers of the bladder, prostate, breast, lung, colon and chronic lymphocytic leukemia (e.g., Meyer-Siegler et al., 2004, BMC Cancer 12:34; Shachar & Haran, 2011, Leuk Lymphoma 52:1446-54). Milatuzumab (hLL1) is an exemplary anti-CD74 antibody of therapeutic use for treatment of MIF-mediated diseases.

Anti-TNF-a antibodies are known in the art and may be of use to treat cancer. Known antibodies against TNF-α include the human antibody CDP571 (Ofei et al., 2011, Diabetes 45:881-85); murine antibodies MTNFAI, M2TNFAI, M3TNFAI, M3TNFABI, M302B and M303 (Thermo Scientific, Rockford, IL); infliximab (Centocor, Malvern, PA); certolizumab pegol (UCB, Brussels, Belgium); and adalimumab (Abbott, Abbott Park, IL). These and many other known anti-TNF-a antibodies may be used in the disclosed methods and compositions.

Checkpoint inhibitor antibodies have been used primarily in cancer therapy. Immune checkpoints refer to inhibitory pathways in the immune system that are responsible for maintaining self-tolerance and modulating the degree of immune system response to minimize peripheral tissue damage. However, tumor cells can also activate immune system checkpoints to decrease the effectiveness of immune response against tumor tissues. Exemplary checkpoint inhibitor antibodies against cytotoxic T-lymphocyte antigen 4 (CTLA4, also known as CD152), programmed cell death protein 1 (PD1, also known as CD279) and programmed cell death 1 ligand 1 (PD-L1, also known as CD274), may be used in combination with one or more other agents to enhance the effectiveness of immune response against cancer cells or tissues. Exemplary anti-PD1 antibodies include lambrolizumab (MK-3475, MERCK), nivolumab (BMS-936558, BRISTOL-MYERS SQUIBB), AMP-224 (MERCK), and pidilizumab (CT-011, CURETECH LTD.). Anti-PD1 antibodies are commercially available, for example from ABCAM® (AB137132), BIOLEGEND® (EH12.2H7, RMP1-14) and AFFYMETRIX EBIOSCIENCE (J105, J116, MIH4). Exemplary anti-PD-L1 antibodies include MDX-1105 (MEDAREX), MEDI4736 (MEDIMMUNE) MPDL3280A (GENENTECH) and BMS-936559 (BRISTOL-MYERS SQUIBB). Anti-PD-L1 antibodies are also commercially available, for example from AFFYMETRIX EBIOSCIENCE (MIH1). Exemplary anti-CTLA4 antibodies include ipilimumab (Bristol-Myers Squibb) and tremelimumab (PFIZER). Anti-PD1 antibodies are commercially available, for example from ABCAM® (AB134090), SINO BIOLOGICAL INC. (11159-H03H, 11159-H08H), and THERMO SCIENTIFIC PIERCE (PA5-29572, PA5-23967, PA5-26465, MA1-12205, MA1-35914). Ipilimumab has recently received FDA approval for treatment of metastatic melanoma (Wada et al., 2013, J Transl Med 11:89).

Antibodies may be preferably usedthat internalize rapidly and are then re-expressed, processed and presented on cell surfaces, enabling continual uptake and accretion of circulating conjugate by the cell. An example of a most-preferred antibody/antigen pair is LL1, an anti-CD74 MAb (invariant chain, class II-specific chaperone, Ii) (see, e.g., U.S. Patent Nos. 6,653,104; 7,312,318). The CD74 antigen is highly expressed on B-cell lymphomas (including multiple myeloma) and leukemias, certain T-cell lymphomas, melanomas, colonic, lung, and renal cancers, glioblastomas, and certain other cancers (Ong et al., Immunology 98:296-302 (1999)). A review of the use of CD74 antibodies in cancer is contained in Stein et al., Clin Cancer Res. 2007 Sep 15;13(18 Pt 2):5556s-5563s.

The diseases that are preferably treated with anti-CD74 antibodies include, but are not limited to, non-Hodgkin's lymphoma, Hodgkin's disease, melanoma, lung, renal, colonic cancers, glioblastome multiforme, histiocytomas, myeloid leukemias, and multiple myeloma. Continual expression of the CD74 antigen for short periods of time on the surface of target cells, followed by internalization of the antigen, and re-expression of the antigen, enables the targeting LL1 antibody to be internalized along with any chemotherapeutic moiety it carries. This allows a high, and therapeutic, concentration of LL1-chemotherapeutic drug conjugate to be accumulated inside such cells. Internalized LL1-chemotherapeutic drug conjugates are cycled through lysosomes and endosomes, and the chemotherapeutic moiety is released in an active form within the target cells.

The antibodies discussed above and other known antibodies against tumor-associated antigens may be used as immunoconjugates, in the practice of the disclosed methods and compositions.

### Bispecific and Multispecific Antibodies

Bispecific antibodies are useful in a number of biomedical applications. For instance, a bispecific antibody with binding sites for a tumor cell surface antigen and for a T-cell surface receptor can direct the lysis of specific tumor cells by T cells. Bispecific antibodies recognizing gliomas and the CD3 epitope on T cells have been successfully used in treating brain tumors in human patients (Nitta, et al. Lancet. 1990; 355:368-371). In certain embodiments, the techniques and compositions for therapeutic agent conjugation disclosed herein may be used with bispecific or multispecific antibodies as the antibody moieties.

Numerous methods to produce bispecific or multispecific antibodies are known, as disclosed, for example, in U.S. Patent No. 7,405,320. Bispecific antibodies can be produced by the quadroma method, which involves the fusion of two different hybridomas, each producing a monoclonal antibody recognizing a different antigenic site (Milstein and Cuello, Nature, 1983; 305:537-540).

Another method for producing bispecific antibodies uses heterobifunctional crosslinkers to chemically tether two different monoclonal antibodies (Staerz, et al. Nature. 1985; 314:628-631; Perez, et al. Nature. 1985; 316:354-356). Bispecific antibodies can also be produced by reduction of each of two parental monoclonal antibodies to the respective half molecules, which are then mixed and allowed to reoxidize to obtain the hybrid structure (Staerz and Bevan. Proc Natl Acad Sci U S A. 1986; 83:1453-1457). Another alternative involves chemically cross-linking two or three separately purified Fab' fragments using appropriate linkers. (See, e.g.,
European Patent Application 0453082).

Other methods include improving the efficiency of generating hybrid hybridomas by gene transfer of distinct selectable markers via retrovirus-derived shuttle vectors into respective parental hybridomas, which are fused subsequently (DeMonte, et al. Proc Natl Acad Sci U S A. 1990, 87:2941-2945); or transfection of a hybridoma cell line with expression plasmids containing the heavy and light chain genes of a different antibody.

Cognate V_{H} and V_{L} domains can be joined with a peptide linker of appropriate composition and length (usually consisting of more than 12 amino acid residues) to form a single-chain Fv (scFv) with binding activity. Methods of manufacturing scFvs are disclosed in U.S. Pat. No. 4,946,778 and U.S. Pat. No. 5,132,405. Reduction of the peptide linker length to less than 12 amino acid residues prevents pairing of V_{H} and V_{L} domains on the same chain and forces pairing of V_{H} and V_{L} domains with complementary domains on other chains, resulting in the formation of functional multimers. Polypeptide chains of V_{H} and V_{L} domains that are joined with linkers between 3 and 12 amino acid residues form predominantly dimers (termed diabodies). With linkers between 0 and 2 amino acid residues, trimers (termed triabody) and tetramers (termed tetrabody) are favored, but the exact patterns of oligomerization appear to depend on the composition as well as the orientation of V-domains (V_{H}-linker-V_{L} or V_{L}-linker-V_{H}), in addition to the linker length.

These techniques for producing multispecific or bispecific antibodies exhibit various difficulties in terms of low yield, necessity for purification, low stability or the laborintensiveness of the technique. More recently, bispecific constructs known as "DOCK-AND-LOCK™" (DNL™) have been used to produce combinations of virtually any desired antibodies, antibody fragments and other effector molecules (see, e.g., U.S. Patent Nos. 7,550,143; 7,521,056; 7,534,866; 7,527,787 and USSN 11/925,408). The technique utilizes complementary protein binding domains, referred to as anchoring domains (AD) and dimerization and docking domains (DDD), which bind to each other and allow the assembly of complex structures, ranging from dimers, trimers, tetramers, quintamers and hexamers. These form stable complexes in high yield without requirement for extensive purification. The technique allows the assembly of monospecific, bispecific or multispecific antibodies. Any of the techniques known in the art for making bispecific or multispecific antibodies may be utilized.

Combinations of use, such as are preferred for cancer therapies, include CD20 + CD22 antibodies, CD74 + CD20 antibodies, CD74 + CD22 antibodies, CEACAM5 (CEA) + CEACAM6 (NCA) antibodies, insulin-like growth factor (ILGF) + CEACAM5 antibodies, EGP-1 (e.g., RS-7) + ILGF antibodies, CEACAM5 + EGFR antibodies. Such antibodies need not only be used in combination, but can be combined as fusion proteins of various forms, such as IgG, Fab, scFv, and the like, as described in U.S. Patent Nos. 6,083,477; 6,183,744 and 6,962,702 and U.S. Patent Application Publication Nos. 20030124058; 20030219433; 20040001825; 20040202666; 20040219156; 20040219203; 20040235065; 20050002945; 20050014207; 20050025709; 20050079184; 20050169926; 20050175582; 20050249738; 20060014245 and 20060034759.

### Pre-Targeting

Bispecific or multispecific antibodies may also be utilized in pre-targeting techniques. Pre-targeting is a multistep process originally developed to resolve the slow blood clearance of directly targeting antibodies, which contributes to undesirable toxicity to normal tissues such as bone marrow. With pre-targeting, a radionuclide or other therapeutic agent is attached to a small delivery molecule (targetable construct) that is cleared within minutes from the blood. A pre-targeting bispecific or multispecific antibody, which has binding sites for the targetable construct as well as a target antigen, is administered first, free antibody is allowed to clear from circulation and then the targetable construct is administered.

Pre-targeting methods are disclosed, for example, in Goodwin et al., U.S. Pat. No. 4,863,713; Goodwin et al., J. Nucl. Med. 29:226, 1988; Hnatowich et al., J. Nucl. Med. 28:1294, 1987; Oehr et al., J. Nucl. Med. 29:728, 1988; Klibanov et al., J. Nucl. Med. 29:1951, 1988; Sinitsyn et al., J. Nucl. Med. 30:66, 1989; Kalofonos et al., J. Nucl. Med. 31:1791, 1990; Schechter et al., Int. J. Cancer 48:167, 1991; Paganelli et al., Cancer Res. 51:5960, 1991; Paganelli et al., Nucl. Med. Commun. 12:211, 1991; U.S. Pat. No. 5,256,395; Stickney et al., Cancer Res. 51:6650, 1991; Yuan et al., Cancer Res. 51:3119, 1991; U.S. Pat. Nos. 6,077,499; 7,011,812; 7,300,644; 7,074,405; 6,962,702; 7,387,772; 7,052,872; 7,138,103; 6,090,381; 6,472,511; 6,962,702; and 6,962,702.

A pre-targeting method of treating or diagnosing a disease or disorder in a subject may be provided by: (1) administering to the subject a bispecific antibody or antibody fragment; (2) optionally administering to the subject a clearing composition, and allowing the composition to clear the antibody from circulation; and (3) administering to the subject the targetable construct, containing one or more chelated or chemically bound therapeutic or diagnostic agents, such as SN-38 or P2PDox. A pre-targeting technique may be use as a step in neoadjuvant therapy.

### Targetable Constructs

In certain embodiments of the invention as defined in the claims, targetable construct peptides labeled with one or more therapeutic or diagnostic agents for use in pre-targeting may be selected to bind to a bispecific antibody with one or more binding sites for a targetable construct peptide and one or more binding sites for a target antigen associated with a disease or condition. Bispecific antibodies may be used in a pretargeting technique wherein the antibody may be administered first to a subject. Sufficient time may be allowed for the bispecific antibody to bind to a target antigen and for unbound antibody to clear from circulation. Then a targetable construct, such as a labeled peptide, may be administered to the subject and allowed to bind to the bispecific antibody and localize at the diseased cell or tissue.

Such targetable constructs can be of diverse structure and are selected not only for the availability of an antibody or fragment that binds with high affinity to the targetable construct, but also for rapid *in vivo* clearance when used within the pre-targeting method and bispecific antibodies (bsAb) or multispecific antibodies. Hydrophobic agents are best at eliciting strong immune responses, whereas hydrophilic agents are preferred for rapid *in vivo* clearance. Thus, a balance between hydrophobic and hydrophilic character is established. This may be accomplished, in part, by using hydrophilic chelating agents to offset the inherent hydrophobicity of many organic moieties. Also, sub-units of the targetable construct may be chosen which have opposite solution properties, for example, peptides, which contain amino acids, some of which are hydrophobic and some of which are hydrophilic.

Peptides having as few as two amino acid residues, preferably two to ten residues, may be used and may also be coupled to other moieties, such as chelating agents. The linker should be a low molecular weight conjugate, preferably having a molecular weight of less than 50,000 daltons, and advantageously less than about 20,000 daltons, 10,000 daltons or 5,000 daltons. More usually, the targetable construct peptide will have four or more residues and one or more haptens for binding, e.g., to a bispecific antibody. Exemplary haptens may include In-DTPA (indium-diethylene triamine pentaacetic acid) or HSG (histamine succinyl glycine). The targetable construct may also comprise one or more chelating moieties, such as DOTA (1,4,7,10-tetraazacyclododecane1,4,7,10-tetraacetic acid), NOTA (1,4,7-triaza-cyclononane-1,4,7-triacetic acid), TETA (*p*-bromoacetamido-benzyl-tetraethylaminetetraacetic acid), NETA ([2-(4,7-biscarboxymethyl[1,4,7]triazacyclononan-1-yl-ethyl]-2-carbonylmethyl-amino]acetic acid) or other known chelating moieties. Chelating moieties may be used, for example, to bind to a therapeutic and or diagnostic radionuclide, paramagnetic ion or contrast agent.

The targetable construct may also comprise unnatural amino acids, e.g., D-amino acids, in the backbone structure to increase the stability of the peptide *in vivo.* In alternative embodiments, other backbone structures such as those constructed from non-natural amino acids or peptoids may be used.

The peptides used as targetable constructs are conveniently synthesized on an automated peptide synthesizer using a solid-phase support and standard techniques of repetitive orthogonal deprotection and coupling. Free amino groups in the peptide, that are to be used later for conjugation of chelating moieties or other agents, are advantageously blocked with standard protecting groups such as a Boc group, while N-terminal residues may be acetylated to increase serum stability. Such protecting groups are well known to the skilled artisan. See Greene and Wuts Protective Groups in Organic Synthesis, 1999 (John Wiley and Sons, N.Y.). When the peptides are prepared for later use within the bispecific antibody system, they are advantageously cleaved from the resins to generate the corresponding C-terminal amides, in order to inhibit *in vivo* carboxypeptidase activity.

Where pretargeting with bispecific antibodies is used, the antibody will contain a first binding site for an antigen produced by or associated with a target tissue and a second binding site for a hapten on the targetable construct. Exemplary haptens include, but are not limited to, HSG and In-DTPA. Antibodies raised to the HSG hapten are known (e.g. 679 antibody) and can be easily incorporated into the appropriate bispecific antibody (see, e.g., U.S. Patent Nos. 6,962,702; 7,138,103 and 7,300,644). However, other haptens and antibodies that bind to them are known in the art and may be used, such as In-DTPA and the 734 antibody (e.g., U.S. Patent No.7,534,431).

### DOCK-AND-LOCK™ (DNL™)

In preferred embodiments, a bivalent or multivalent antibody is formed as a DOCK-AND-LOCK™ (DNL™) complex (see, e.g., U.S. Patent Nos. 7,521,056; 7,527,787; 7,534,866; 7,550,143 and 7,666,400.) Generally, the technique takes advantage of the specific and high-affinity binding interactions that occur between a dimerization and docking domain (DDD) sequence of the regulatory (R) subunits of cAMP-dependent protein kinase (PKA) and an anchor domain (AD) sequence derived from any of a variety of AKAP proteins (Baillie et al., FEBS Letters. 2005; 579: 3264. Wong and Scott, Nat. Rev. Mol. Cell Biol. 2004; 5: 959). The DDD and AD peptides may be attached to any protein, peptide or other molecule. Because the DDD sequences spontaneously dimerize and bind to the AD sequence, the technique allows the formation of complexes between any selected molecules that may be attached to DDD or AD sequences.

Although the standard DNL™ complex comprises a trimer with two DDD-linked molecules attached to one AD-linked molecule, variations in complex structure allow the formation of dimers, trimers, tetramers, pentamers, hexamers and other multimers. In some embodiments, the DNL™ complex may comprise two or more antibodies, antibody fragments or fusion proteins which bind to the same antigenic determinant or to two or more different antigens. The DNL™ complex may also comprise one or more other effectors, such as proteins, peptides, immunomodulators, cytokines, interleukins, interferons, binding proteins, peptide ligands, carrier proteins, toxins, ribonucleases such as onconase, inhibitory oligonucleotides such as siRNA, antigens or xenoantigens, polymers such as PEG, enzymes, therapeutic agents, hormones, cytotoxic agents, anti-angiogenic agents, pro-apoptotic agents or any other molecule or aggregate.

PKA, which plays a central role in one of the best studied signal transduction pathways triggered by the binding of the second messenger cAMP to the R subunits, was first isolated from rabbit skeletal muscle in 1968 (Walsh et al., J. Biol. Chem. 1968;243:3763). The structure of the holoenzyme consists of two catalytic subunits held in an inactive form by the R subunits (Taylor, J. Biol. Chem. 1989;264:8443). Isozymes of PKA are found with two types of R subunits (RI and RII), and each type has α and β isoforms (Scott, Pharmacol. Ther. 1991;50:123). Thus, the four isoforms of PKA regulatory subunits are RIα, RIβ, RIIα and RIIβ. The R subunits have been isolated only as stable dimers and the dimerization domain has been shown to consist of the first 44 amino-terminal residues of RIIα (Newlon et al., Nat. Struct. Biol. 1999; 6:222). As discussed below, similar portions of the amino acid sequences of other regulatory subunits are involved in dimerization and docking, each located near the N-terminal end of the regulatory subunit. Binding of cAMP to the R subunits leads to the release of active catalytic subunits for a broad spectrum of serine/threonine kinase activities, which are oriented toward selected substrates through the compartmentalization of PKA via its docking with AKAPs (Scott et al., J. Biol. Chem. 1990;265;21561)

Since the first AKAP, microtubule-associated protein-2, was characterized in 1984 (Lohmann et al., Proc. Natl. Acad. Sci USA. 1984; 81:6723), more than 50 AKAPs that localize to various sub-cellular sites, including plasma membrane, actin cytoskeleton, nucleus, mitochondria, and endoplasmic reticulum, have been identified with diverse structures in species ranging from yeast to humans (Wong and Scott, Nat. Rev. Mol. Cell Biol. 2004;5:959). The AD of AKAPs for PKA is an amphipathic helix of 14-18 residues (Carr et al., J. Biol. Chem. 1991;266:14188). The amino acid sequences of the AD are quite varied among individual AKAPs, with the binding affinities reported for RII dimers ranging from 2 to 90 nM (Alto et al., Proc. Natl. Acad. Sci. USA. 2003; 100:4445). AKAPs will only bind to dimeric R subunits. For human RIIα, the AD binds to a hydrophobic surface formed by the 23 amino-terminal residues (Colledge and Scott, Trends Cell Biol. 1999; 6:216). Thus, the dimerization domain and AKAP binding domain of human RIIα are both located within the same N-terminal 44 amino acid sequence (Newlon et al., Nat. Struct. Biol. 1999;6:222; Newlon et al., EMBO J. 2001;20:1651), which is termed the DDD herein.

We have developed a platform technology to utilize the DDD of human PKA regulatory subunits and the AD of AKAP as an excellent pair of linker modules for docking any two entities, referred to hereafter as **A** and **B,** into a noncovalent complex, which could be further locked into a DNL™ complex through the introduction of cysteine residues into both the DDD and AD at strategic positions to facilitate the formation of disulfide bonds. The general methodology of the approach is as follows. Entity **A** is constructed by linking a DDD sequence to a precursor of **A,** resulting in a first component hereafter referred to as **a.** Because the DDD sequence would effect the spontaneous formation of a dimer, **A** would thus be composed of **a₂.** Entity **B** is constructed by linking an AD sequence to a precursor of **B,** resulting in a second component hereafter referred to as **b.** The dimeric motif of DDD contained in **a₂** will create a docking site for binding to the AD sequence contained in b, thus facilitating a ready association of **a₂** and **b** to form a binary, trimeric complex composed of **a₂b.** This binding event is made irreversible with a subsequent reaction to covalently secure the two entities via disulfide bridges, which occurs very efficiently based on the principle of effective local concentration because the initial binding interactions should bring the reactive thiol groups placed onto both the DDD and AD into proximity (Chmura et al., Proc. Natl. Acad. Sci. USA. 2001;98:8480) to ligate site-specifically. Using various combinations of linkers, adaptor modules and precursors, a wide variety of DNL™ constructs of different stoichiometry may be produced and used (see, e.g., U.S. Nos. 7,550,143; 7,521,056; 7,534,866; 7,527,787 and 7,666,400.)

By attaching the DDD and AD away from the functional groups of the two precursors, such site-specific ligations are also expected to preserve the original activities of the two precursors. This approach is modular in nature and potentially can be applied to link, site-specifically and covalently, a wide range of substances, including peptides, proteins, antibodies, antibody fragments, and other effector moieties with a wide range of activities. Utilizing the fusion protein method of constructing AD and DDD conjugated effectors described in the Examples below, virtually any protein or peptide may be incorporated into a DNL™ construct. However, the technique is not limiting and other methods of conjugation may be utilized.

A variety of methods are known for making fusion proteins, including nucleic acid synthesis, hybridization and/or amplification to produce a synthetic double-stranded nucleic acid encoding a fusion protein of interest. Such double-stranded nucleic acids may be inserted into expression vectors for fusion protein production by standard molecular biology techniques (see, e.g. Sambrook et al., Molecular Cloning, A laboratory manual, 2nd Ed, 1989). In such preferred embodiments, the AD and/or DDD moiety may be attached to either the N-terminal or C-terminal end of an effector protein or peptide. However, the skilled artisan will realize that the site of attachment of an AD or DDD moiety to an effector moiety may vary, depending on the chemical nature of the effector moiety and the part(s) of the effector moiety involved in its physiological activity. Site-specific attachment of a variety of effector moieties may be performed using techniques known in the art, such as the use of bivalent cross-linking reagents and/or other chemical conjugation techniques.

### Alternative DNL™ Structures

In certain alternative embodiments, DNL™ constructs may be formed using alternatively constructed antibodies or antibody fragments, in which an AD moiety may be attached at the C-terminal end of the kappa light chain (Cₖ), instead of the C-terminal end of the Fc on the heavy chain. The alternatively formed DNL™ constructs may be prepared as disclosed in Provisional U.S. Patent Application Serial Nos. 61/654,310, filed June 1, 2012, 61/662,086, filed June 20, 2012, 61/673,553, filed July 19, 2012, and 61/682,531, filed August 13, 2012. The light chain conjugated DNL™ constructs exhibit enhanced Fc-effector function activity *in vitro* and improved pharmacokinetics, stability and anti-lymphoma activity *in vivo* (Rossi et al., 2013, Bioconjug Chem 24:63-71).

Cₖ-conjugated DNL™ constructs may be prepared as disclosed in Provisional U.S. Patent Application Serial Nos. 61/654,310, 61/662,086, 61/673,553, and 61/682,531. Briefly, Cₖ-AD2-IgG, was generated by recombinant engineering, whereby the AD2 peptide was fused to the C-terminal end of the kappa light chain. Because the natural C-terminus of C_{K} is a cysteine residue, which forms a disulfide bridge to C_{H}1, a 16-amino acid residue "hinge" linker was used to space the AD2 from the C_{K}-V_{H}1 disulfide bridge. The mammalian expression vectors for Cₖ-AD2-IgG-veltuzumab and Cₖ-AD2-IgG-epratuzumab were constructed using the pdHL2 vector, which was used previously for expression of the homologous C_{H}3-AD2-IgG modules. A 2208-bp nucleotide sequence was synthesized comprising the pdHL2 vector sequence ranging from the *Bam HI* restriction site within the V_{K}/C_{K} intron to the *Xho I* restriction site 3' of the Cₖ intron, with the insertion of the coding sequence for the hinge linker and AD2, in frame at the 3' end of the coding sequence for C_{K}. This synthetic sequence was inserted into the IgG-pdHL2 expression vectors for veltuzumab and epratuzumab *via Bam HI* and *Xho I* restriction sites. Generation of production clones with SpESFX-10 were performed as described for the C_{H}3-AD2-IgG modules. Cₖ-AD2-IgG-veltuzumab and Cₖ-AD2-IgG-epratuzumab were produced by stably-transfected production clones in batch roller bottle culture, and purified from the supernatant fluid in a single step using MabSelect (GE Healthcare) Protein A affinity chromatography.

### Phage Display

Certain embodiments of the invention as defined in the claims may comprises the use of binding peptides and/or peptide mimetics of various target molecules, cells or tissues. Binding peptides may be identified by any method known in the art, including but not limiting to the phage display technique. Various methods of phage display and techniques for producing diverse populations of peptides are well known in the art. For example, U.S. Pat. Nos. 5,223,409; 5,622,699 and 6,068,829 disclose methods for preparing a phage library. The phage display technique involves genetically manipulating bacteriophage so that small peptides can be expressed on their surface (Smith and Scott, 1985, Science 228:1315-1317; Smith and Scott, 1993, Meth. Enzymol. 21:228-257). In addition to peptides, larger protein domains such as single-chain antibodies may also be displayed on the surface of phage particles (Arap et al., 1998, Science 279:377-380).

Targeting amino acid sequences selective for a given organ, tissue, cell type or target molecule may be isolated by panning (Pasqualini and Ruoslahti, 1996, Nature 380:364-366; Pasqualini, 1999, The Quart. J. Nucl. Med. 43:159-162). In brief, a library of phage containing putative targeting peptides is administered to an intact organism or to isolated organs, tissues, cell types or target molecules and samples containing bound phage are collected. Phage that bind to a target may be eluted from a target organ, tissue, cell type or target molecule and then amplified by growing them in host bacteria.

In certain examples, the phage may be propagated in host bacteria between rounds of panning. Rather than being lysed by the phage, the bacteria may instead secrete multiple copies of phage that display a particular insert. If desired, the amplified phage may be exposed to the target organs, tissues, cell types or target molecule again and collected for additional rounds of panning. Multiple rounds of panning may be performed until a population of selective or specific binders is obtained. The amino acid sequence of the peptides may be determined by sequencing the DNA corresponding to the targeting peptide insert in the phage genome. The identified targeting peptide may then be produced as a synthetic peptide by standard protein chemistry techniques (Arap *et al.,* 1998, Smith *et al.,* 1985).

In some examples, a subtraction protocol may be used to further reduce background phage binding. The purpose of subtraction is to remove phage from the library that bind to targets other than the target of interest. In alternative examples, the phage library may be prescreened against a control cell, tissue or organ. For example, tumor-binding peptides may be identified after prescreening a library against a control normal cell line. After subtraction the library may be screened against the molecule, cell, tissue or organ of interest. Other methods of subtraction protocols are known and may be used in the practice of the claimed methods, for example as disclosed in U.S Patent Nos. 5,840,841, 5,705,610, 5,670,312 and 5,492,807.

### Nanobodies

Nanobodies are single-domain antibodies of about 12-15 kDa in size (about 110 amino acids in length). Nanobodies can selectively bind to target antigens, like full-size antibodies, and have similar affinities for antigens. However, because of their much smaller size, they may be capable of better penetration into solid tumors. The smaller size also contributes to the stability of the nanobody, which is more resistant to pH and temperature extremes than full size antibodies (Van Der Linden et al., 1999, Biochim Biophys Act 1431:37-46). Single-domain antibodies were originally developed following the discovery that camelids (camels, alpacas, llamas) possess fully functional antibodies without light chains (e.g., Hamsen et al., 2007, Appl Microbiol Biotechnol 77:13-22). The heavy-chain antibodies consist of a single variable domain (V_{HH}) and two constant domains (C_{H}2 and C_{H}3). Like antibodies, nanobodies may be developed and used as multivalent and/or bispecific constructs. Humanized forms of nanobodies are in commercial development that are targeted to a variety of target antigens, such as IL-6R, vWF, TNF, RSV, RANKL, IL-17A & F and IgE (e.g., ABLYNX®, Ghent, Belgium), with potential clinical use in cancer (e.g., Saerens et al., 2008, Curr Opin Pharmacol 8:600-8; Muyldermans, 2013, Ann Rev Biochem 82:775-97).

The plasma half-life of nanobodies is shorter than that of full-size antibodies, with elimination primarily by the renal route. Because they lack an Fc region, they do not exhibit complement dependent cytotoxicity.

Nanobodies may be produced by immunization of camels, llamas, alpacas or sharks with target antigen, following by isolation of mRNA, cloning into libraries and screening for antigen binding. Nanobody sequences may be humanized by standard techniques (e.g., Jones et al., 1986, Nature 321: 522, Riechmann et al., 1988, Nature 332: 323, Verhoeyen et al., 1988, Science 239: 1534, Carter et al., 1992, Proc. Nat'l Acad. Sci. USA 89: 4285, Sandhu, 1992, Crit. Rev. Biotech. 12: 437, Singer et al., 1993, J. Immun. 150: 2844). Humanization is relatively straight-forward because of the high homology between camelid and human FR sequences.

In various embodiments of the invention as defined in the claims, the subject immunoconjugates may comprise nanobodies for targeted delivery of conjugated drug to cells, tissues or organs. Nanobodies of use are disclosed, for example, in U.S. Patent Nos. 7,807,162; 7,939,277; 8,188,223; 8,217,140; 8,372,398; 8,557,965; 8,623,361 and 8,629,244.)

### Conjugation Protocols

The preferred conjugation protocol is based on a thiol-maleimide, a thiolvinylsulfone, a thiol-bromoacetamide, or a thiol-iodoacetamide reaction that are facile at neutral or acidic pH. This obviates the need for higher pH conditions for conjugations as, for instance, would be necessitated when using active esters. Further details of exemplary conjugation protocols are described below in the Examples section.

### Therapeutic Treatment

According to the invention as defined in the claims a therapeutically effective amount of a therapeutic conjugate is administered to a subject. Diseases that may be treated with the therapeutic conjugates described herein include, but are not limited to B-cell malignancies (e.g., non-Hodgkin's lymphoma, mantle cell lymphoma, multiple myeloma, Hodgkin's lymphoma, diffuse large B cell lymphoma, Burkitt lymphoma, follicular lymphoma, acute lymphocytic leukemia, chronic lymphocytic leukemia, hairy cell leukemia) using, for example an anti-CD22 antibody such as the hLL2 MAb (epratuzumab, *see* U.S. Patent No. 6,183,744), against another CD22 epitope (hRFB4) or antibodies against other B cell antigens, such as CD19, CD20, CD21, CD22, CD23, CD37, CD40, CD40L, CD52, CD74, CD80 or HLA-DR. Other diseases include, but are not limited to, adenocarcinomas of endodermally-derived digestive system epithelia, cancers such as breast cancer and non-small cell lung cancer, and other carcinomas, sarcomas, glial tumors, myeloid leukemias, etc. In particular, antibodies against an antigen, e.g., an oncofetal antigen, produced by or associated with a malignant solid tumor or hematopoietic neoplasm, e.g., a gastrointestinal, stomach, colon, esophageal, liver, lung, breast, pancreatic, liver, prostate, ovarian, testicular, brain, bone or lymphatic tumor, a sarcoma or a melanoma, are advantageously used. Such therapeutics can be given once or repeatedly, depending on the disease state and tolerability of the conjugate, and can also be used optionally in combination with other therapeutic modalities, such as surgery, external radiation, radioimmunotherapy, immunotherapy, chemotherapy, antisense therapy, interference RNA therapy, gene therapy, and the like. Each combination will be adapted to the tumor type, stage, patient condition and prior therapy, and other factors considered by the managing physician.

As used herein, the term "subject" refers to any animal (i.e., vertebrates and invertebrates) including, but not limited to mammals, including humans. It is not intended that the term be limited to a particular age or sex. Thus, adult and newborn subjects, as well as fetuses, whether male or female, are encompassed by the term. Doses given herein are for humans, but can be adjusted to the size of other mammals, as well as children, in accordance with weight or square meter size.

In a preferred embodiment of the invention as defined in the claims, therapeutic conjugates comprising an anti-EGP-1 (anti-TROP-2) antibody such as the hRS7 MAb can be used to treat carcinomas such as carcinomas of the esophagus, pancreas, lung, stomach, colon and rectum, urinary bladder, breast, ovary, uterus, kidney and prostate, as disclosed in U.S. Patent No. 7,238,785; 7,517,964 and 8,084,583. An hRS7 antibody is a humanized antibody that comprises light chain complementarity-determining region (CDR) sequences CDR1 (KASQDVSIAVA, SEQ ID NO:3); CDR2 (SASYRYT, SEQ ID NO:4); and CDR3 (QQHYITPLT, SEQ ID NO:5) and heavy chain CDR sequences CDR1 (NYGMN, SEQ ID NO:6); CDR2 (WINTYTGEPTYTDDFKG, SEQ ID NO:7) and CDR3 (GGFGSSYWYFDV, SEQ ID NO:8).

According to the instant disclosure, therapeutic conjugates may comprising an anti-CEACAM5 antibody (e.g., hMN-14, labretuzumab) and/or an anti-CEACAM6 antibody (e.g., hMN-3 or hMN-15) may be used to treat any of a variety of cancers that express CEACAM5 and/or CEACAM6, as disclosed in U.S. Patent Nos. 8,287,865; 7,951,369; 5,874,540; 6,676,924 and 8,267,865. Solid tumors that may be treated using anti-CEACAM5, anti-CEACAM6, or a combination of the two include but are not limited to breast, lung, pancreatic, esophageal, medullary thyroid, ovarian, colon, rectum, urinary bladder, mouth and stomach cancers. A majority of carcinomas, including gastrointestinal, respiratory, genitourinary and breast cancers express CEACAM5 and may be treated with the subject immunoconjugates. An hMN-14 antibody is a humanized antibody that comprises light chain variable region CDR sequences CDR1 (KASQDVGTSVA; SEQ ID NO:9), CDR2 (WTSTRHT; SEQ ID NO:10), and CDR3 (QQYSLYRS; SEQ ID NO:11), and the heavy chain variable region CDR sequences CDR1 (TYWMS; SEQ ID NO:12), CDR2 (EIHPDSSTINYAPSLKD; SEQ ID NO:13) and CDR3 (LYFGFPWFAY; SEQ ID NO:14).

An hMN-3 antibody is a humanized antibody that comprises light chain variable region CDR sequences CDR1 (RSSQSIVHSNGNTYLE, SEQ ID NO:15), CDR2 (KVSNRFS, SEQ ID NO:16) and CDR3 (FQGSHVPPT, SEQ ID NO:17) and the heavy chain CDR sequences CDR1 (NYGMN, SEQ ID NO:18), CDR2 (WINTYTGEPTYADDFKG, SEQ ID NO:19) and CDR3 (KGWMDFNSSLDY, SEQ ID NO:20).

An hMN-15 antibody is a humanized antibody that comprises light chain variable region CDR sequences SASSRVSYIH (SEQ ID NO:21); GTSTLAS (SEQ ID NO:22); and QQWSYNPPT (SEQ ID NO:23); and heavy chain variable region CDR sequences DYYMS (SEQ ID NO:24); FIANKANGHTTDYSPSVKG (SEQ ID NO:25); and DMGIRWNFDV (SEQ ID NO:26).

In another example of the instant disclosure, therapeutic conjugates comprising an anti-CD74 antibody (e.g., hLL1, milatuzumab, disclosed in U.S. Patent Nos. 7,074,403; 7,312,318; 7,772,373; 7,919,087 and 7,931,903) may be used to treat any of a variety of cancers that express CD74, including but not limited to renal, lung, intestinal, stomach, breast, prostate or ovarian cancer, as well as several hematological cancers, such as multiple myeloma, chronic lymphocytic leukemia, acute lymphoblastic leukemia, non-Hodgkin lymphoma, and Hodgkin lymphoma. An hLL1 antibody is a humanized antibody comprising the light chain CDR sequences CDR1 (RSSQSLVHRNGNTYLH; SEQ ID NO:27), CDR2 (TVSNRFS; SEQ ID NO:28), and CDR3 (SQSSHVPPT; SEQ ID NO:29) and the heavy chain variable region CDR sequences CDR1 (NYGVN; SEQ ID NO:30), CDR2 (WINPNTGEPTFDDDFKG; SEQ ID NO:31), and CDR3 (SRGKNEAWFAY; SEQ ID NO:32).

In another example of the instant disclosure, therapeutic conjugates comprising an anti-CD22 antibody (e.g., hLL2, epratuzumab, disclosed in U.S. Patent Nos. 5,789,554; 6,183,744; 6,187,287; 6,306,393; 7,074,403 and 7,641,901, or the chimeric or humanized RFB4 antibody) may be used to treat any of a variety of cancers that express CD22, including but not limited to indolent forms of B-cell lymphomas, aggressive forms of B-cell lymphomas, chronic lymphatic leukemias, acute lymphatic leukemias, non-Hodgkin's lymphoma, Hodgkin's lymphoma, Burkitt lymphoma, follicular lymphoma or diffuse B-cell lymphoma. An hLL2 antibody is a humanized antibody comprising light chain CDR sequences CDR1 (KSSQSVLYSANHKYLA, SEQ ID NO:33), CDR2 (WASTRES, SEQ ID NO:34), and CDR3 (HQYLSSWTF, SEQ ID NO:35) and the heavy chain CDR sequences CDR1 (SYWLH, SEQ ID NO:36), CDR2 (YINPRNDYTEYNQNFKD, SEQ ID NO:37), and CDR3 (RDITTFY, SEQ ID NO:38)

In an example of the instant disclosure, therapeutic conjugates comprising anti-CSAp antibodies, such as the hMu-9 MAb, can be used to treat colorectal, as well as pancreatic and ovarian cancers as disclosed in U.S. Patent Nos. 6,962,702; 7,387,772; 7,414,121; 7,553,953; 7,641,891 and 7,670,804. An hMu-9 antibody is a humanized antibody comprising light chain CDR sequences CDR1 (RSSQSIVHSNGNTYLE, SEQ ID NO:39), CDR2 (KVSNRFS, SEQ ID NO:40), and CDR3 (FQGSRVPYT, SEQ ID NO:41), and heavy chain variable CDR sequences CDR1 (EYVIT, SEQ ID NO:42), CDR2 (EIYPGSGSTSYNEKFK, SEQ ID NO:43), and CDR3 (EDL, SEQ ID NO:44).

Therapeutic conjugates comprising the hPAM4 MAb can be used to treat pancreatic cancer or other solid tumors, as disclosed in U.S. Patent Nos. 7,238,786 and 7,282,567. An hPAM4 antibody is a humanized antibody comprising light chain variable region CDR sequencs CDR1 (SASSSVSSSYLY, SEQ ID NO:45); CDR2 (STSNLAS, SEQ ID NO:46); and CDR3 (HQWNRYPYT, SEQ ID NO:47); and heavy chain CDR sequences CDR1 (SYVLH, SEQ ID NO:48); CDR2 (YINPYNDGTQYNEKFKG, SEQ ID NO:49) and CDR3 (GFGGSYGFAY, SEQ ID NO:50).

In another example of the instant disclosure, therapeutic conjugates comprising an anti-AFP MAb, such as IMMU31, can be used to treat hepatocellular carcinoma, germ cell tumors, and other AFP-producing tumors using humanized, chimeric and human antibody forms, as disclosed in U.S. Patent No. 7,300,655. An IMMU31 antibody is a humanized antibody comprising the heavy chain CDR sequences CDR1 (SYVIH, SEQ ID NO:51), CDR2 (YIHPYNGGTKYNEKFKG, SEQ ID NO:52) and CDR3 (SGGGDPFAY, SEQ ID NO:53) and the light chain CDR1 (KASQDINKYIG, SEQ ID NO:54), CDR2 (YTSALLP, SEQ ID NO:55) and CDR3 (LQYDDLWT, SEQ ID NO:56).

In another example of the instant disclosure, therapeutic conjugates comprising an anti-HLA-DR MAb, such as hL243, can be used to treat lymphoma, leukemia, cancers of the skin, esophagus, stomach, colon, rectum, pancreas, lung, breast, ovary, bladder, endometrium, cervix, testes, kidney, liver, melanoma or other HLA-DR-producing tumors, as disclosed in U.S. Patent No. 7,612,180. An hL243 antibody is a humanized antibody comprising the heavy chain CDR sequences CDR1 (NYGMN, SEQ ID NO:57), CDR2 (WINTYTREPTYADDFKG, SEQ ID NO:58), and CDR3 (DITAVVPTGFDY, SEQ ID NO:59) and light chain CDR sequences CDR1 (RASENIYSNLA, SEQ ID NO:60), CDR2 (AASNLAD, SEQ ID NO:61), and CDR3 (QHFWTTPWA, SEQ ID NO:62).

In another example of the instant disclosure, therapeutic conjugates comprising an anti-CD20 MAb, such as veltuzumab (hA20), 1F5, obinutuzumab (GA101), or rituximab, can be used to treat lymphoma, leukemia, Burkitt lymphoma, non-Hodgkin's lymphoma, follicular lymphoma, small lymphocytic lymphoma, diffuse B-cell lymphoma, marginal zone lymphoma, chronic lymphocytic leukemia, acute lymphocytic leukemia, as disclosed in U.S. Patent Nos. 7,435,803 or 8,287,864. An hA20 (veltuzumab) antibody is a humanized antibody comprising the light chain CDR sequences CDRL1 (RASSSVSYIH, SEQ ID NO:63), CDRL2 (ATSNLAS, SEQ ID NO:64) and CDRL3 (QQWTSNPPT, SEQ ID NO:65) and heavy chain CDR sequences CDRH1 (SYNMH, SEQ ID NO:66), CDRH2 (AIYPGNGDTSYNQKFKG, SEQ ID NO:67) and CDRH3 (STYYGGDWYFDV, SEQ ID NO:68).

In another example of the instant disclosure, therapeutic conjugates comprising an anti-CD 19 MAb, such as hA19, can be used to treat B-cell related lymphomas and leukemias, such as non-Hodgkin's lymphoma, chronic lymphocytic leukemia or acute lymphoblastic leukemia, as disclosed in U.S. Patent Nos. 7,109,304, 7,462,352, 7,902,338, 8,147,831 and 8,337,840. An hA19 antibody is a humanized antibody comprising the light chain CDR sequences CDR1 KASQSVDYDGDSYLN (SEQ ID NO: 69); CDR2 DASNLVS (SEQ ID NO:70); and CDR3 QQSTEDPWT (SEQ ID NO: 71) and the heavy chain CDR sequences CDR1 SYWMN (SEQ ID NO: 72); CDR2 QIWPGDGDTNYNGKFKG (SEQ ID NO:73) and CDR3 RETTTVGRYYYAMDY (SEQ ID NO:74).

Therapeutic conjugates comprising anti-tenascin antibodies can be used to treat hematopoietic and solid tumors, and conjugates comprising antibodies to tenascin can be used to treat solid tumors, preferably brain cancers like glioblastomas.

Preferably, the antibodies that are used in the treatment of human disease are human or humanized (CDR-grafted) versions of antibodies; although murine and chimeric versions of antibodies can be used. Same species IgG molecules as delivery agents are mostly preferred to minimize immune responses. This is particularly important when considering repeat treatments. For humans, a human or humanized IgG antibody is less likely to generate an anti-IgG immune response from patients. Antibodies such as hLL1 and hLL2 rapidly internalize after binding to internalizing antigen on target cells, which means that the chemotherapeutic drug being carried is rapidly internalized into cells as well. However, antibodies that have slower rates of internalization can also be used to effect selective therapy.

The person of ordinary skill will realize that the subject immunoconjugates, comprising a camptothecin or anthracycline conjugated to an antibody or antibody fragment, may be used alone or in combination with one or more other therapeutic agents, such as a second antibody, second antibody fragment, second immunoconjugate, radionuclide, toxin, drug, chemotherapeutic agent, radiation therapy, chemokine, cytokine, immunomodulator, enzyme, hormone, oligonucleotide, RNAi or siRNA. Such additional therapeutic agents may be administered separately, in combination with, or attached to the subject antibody-drug immunoconjugates.

In certain embodiments of the invention as defined in the claims, a therapeutic agent used in combination with the immunoconjugates of this invention may comprise one or more isotopes. Radioactive isotopes useful for treating diseased tissue include, but are not limited to- ¹¹¹In, ¹⁷⁷Lu, ²¹²Bi, ²¹³Bi, ²¹¹At, ⁶²Cu, ⁶⁷Cu, ⁹⁰Y, ¹²⁵I, ¹³¹I, ³²P, ³³P, ⁴⁷Sc, ¹¹¹Ag, ⁶⁷Ga, ¹⁴²Pr, ¹⁵³Sm, ¹⁶¹Tb, ¹⁶⁶Dy, ¹⁶⁶Ho, ¹⁸⁶Re, ¹⁸⁸Re, ¹⁸⁹Re, ²¹²Pb, ²²³Ra, ²²⁵Ac, ⁵⁹Fe, ⁷⁵Se, ⁷⁷As, ⁸⁹Sr, ⁹⁹Mo, ¹⁰⁵Rh , ¹⁰⁹Pd, ¹⁴³Pr, ¹⁴⁹Pm, ¹⁶⁹Er, ¹⁹⁴Ir, ¹⁹⁸Au, ¹⁹⁹Au ²²⁷Th and ²¹¹pb. The therapeutic radionuclide preferably has a decay-energy in the range of 20 to 6,000 keV, preferably in the ranges 60 to 200 keV for an Auger emitter, 100-2,500 keV for a beta emitter, and 4,000-6,000 keV for an alpha emitter. Maximum decay energies of useful beta-particle-emitting nuclides are preferably 20-5,000 keV, more preferably 100-4,000 keV, and most preferably 500-2,500 keV. Also preferred are radionuclides that substantially decay with Auger-emitting particles. For example, Co-58, Ga-67, Br-80m, Tc-99m, Rh-103m, Pt-109, In-111, Sb-119, 1-125, Ho-161, Os-189m and Ir-192. Decay energies of useful beta-particle-emitting nuclides are preferably <1,000 keV, more preferably <100 keV, and most preferably <70 keV. Also preferred are radionuclides that substantially decay with generation of alpha-particles. Such radionuclides include, but are not limited to: Dy-152, At-211, Bi-212, Ra-223, Rn-219, Po-215, Bi-211, Ac-225, Fr-221, At-217, Bi-213, Th-227 and Fm-255. Decay energies of useful alpha-particle-emitting radionuclides are preferably 2,000-10,000 keV, more preferably 3,000-8,000 keV, and most preferably 4,000-7,000 keV. Additional potential radioisotopes of use include ¹¹C, ¹³N, ¹⁵O, ⁷⁵Br, ¹⁹⁸Au, ²²⁴Ac, ¹²⁶I, ¹³³I, ⁷⁷Br, ^{113m}In, ⁹⁵Ru, ⁹⁷Ru, ¹⁰³Ru, ¹⁰⁵Ru, ¹⁰⁷Hg, ²⁰³Hg, ^{121m}Te, ^{122m}Te, ^{125m}Te, ¹⁶⁵Tm, ¹⁶⁷Tm, ¹⁶⁸Tm, ¹⁹⁷Pt, ¹⁰⁹Pd, ¹⁰⁵Rh , ¹⁴²Pr, ¹⁴³Pr, ¹⁶¹Tb, ¹⁶⁶Ho, ¹⁹⁹Au, ⁵⁷Co, ⁵⁸Co, ⁵¹Cr, ⁵⁹Fe, ⁷⁵Se, ²⁰¹Tl, ²²⁵Ac, ⁷⁶Br, ¹⁶⁹Yb, and the like.

Radionuclides and other metals may be delivered, for example, using chelating groups attached to an antibody or conjugate. Macrocyclic chelates such as NOTA, DOTA, and TETA are of use with a variety of metals and radiometals, most particularly with radionuclides of gallium, yttrium and copper, respectively. Such metal-chelate complexes can be made very stable by tailoring the ring size to the metal of interest. Other ring-type chelates, such as macrocyclic polyethers for complexing ²²³Ra, may be used.

Therapeutic agents of use in combination with the immunoconjugates described herein also include, for example, chemotherapeutic drugs such as vinca alkaloids, anthracyclines, epidophyllotoxins, taxanes, antimetabolites, tyrosine kinase inhibitors, alkylating agents, antibiotics, Cox-2 inhibitors, antimitotics, antiangiogenic and proapoptotic agents, particularly doxorubicin, methotrexate, taxol, other camptothecins, and others from these and other classes of anticancer agents, and the like. Other cancer chemotherapeutic drugs include nitrogen mustards, alkyl sulfonates, nitrosoureas, triazenes, folic acid analogs, pyrimidine analogs, purine analogs, platinum coordination complexes, hormones, and the like. Suitable chemotherapeutic agents are described in REMINGTON'S PHARMACEUTICAL SCIENCES, 19th Ed. (Mack Publishing Co. 1995), and in GOODMAN AND GILMAN'S THE PHARMACOLOGICAL BASIS OF THERAPEUTICS, 7th Ed. (MacMillan Publishing Co. 1985), as well as revised editions of these publications. Other suitable chemotherapeutic agents, such as experimental drugs, are known to those of skill in the art.

Exemplary drugs of use include, but are not limited to, 5-fluorouracil, afatinib, aplidin, azaribine, anastrozole, anthracyclines, axitinib, AVL-101, AVL-291, bendamustine, bleomycin, bortezomib, bosutinib, bryostatin-1, busulfan, calicheamycin, camptothecin, carboplatin, 10-hydroxycamptothecin, carmustine, celebrex, chlorambucil, cisplatin (CDDP), Cox-2 inhibitors, irinotecan (CPT-11), SN-38, carboplatin, cladribine, camptothecans, crizotinib, cyclophosphamide, cytarabine, dacarbazine, dasatinib, dinaciclib, docetaxel, dactinomycin, daunorubicin, doxorubicin, 2-pyrrolinodoxorubicine (2P-DOX), cyano-morpholino doxorubicin, doxorubicin glucuronide, epirubicin glucuronide, erlotinib, estramustine, epidophyllotoxin, erlotinib, entinostat, estrogen receptor binding agents, etoposide (VP16), etoposide glucuronide, etoposide phosphate, exemestane, fingolimod, floxuridine (FUdR), 3',5'-O-dioleoyl-FudR (FUdR-dO), fludarabine, flutamide, farnesyl-protein transferase inhibitors, flavopiridol, fostamatinib, ganetespib, GDC-0834, GS-1101, gefitinib, gemcitabine, hydroxyurea, ibrutinib, idarubicin, idelalisib, ifosfamide, imatinib, L-asparaginase, lapatinib, lenolidamide, leucovorin, LFM-A13, lomustine, mechlorethamine, melphalan, mercaptopurine, 6-mercaptopurine, methotrexate, mitoxantrone, mithramycin, mitomycin, mitotane, navelbine, neratinib, nilotinib, nitrosurea, olaparib, plicomycin, procarbazine, paclitaxel, PCI-32765, pentostatin, PSI-341, raloxifene, semustine, sorafenib, streptozocin, SU11248, sunitinib, tamoxifen, temazolomide (an aqueous form of DTIC), transplatinum, thalidomide, thioguanine, thiotepa, teniposide, topotecan, uracil mustard, vatalanib, vinorelbine, vinblastine, vincristine, vinca alkaloids and ZD1839. Such agents may be part of the conjugates described herein or may alternatively be administered in combination with the described conjugates, either prior to, simultaneously with or after the conjugate. Alternatively, one or more therapeutic naked antibodies as are known in the art may be used in combination with the described conjugates. Exemplary therapeutic naked antibodies are described above.

Therapeutic agents that may be used in concert with the immunoconjugates also may comprise toxins conjugated to targeting moieties. Toxins that may be used in this regard include ricin, abrin, ribonuclease (RNase), DNase I, Staphylococcal enterotoxin-A, pokeweed antiviral protein, gelonin, diphtheria toxin, *Pseudomonas* exotoxin, and *Pseudomonas* endotoxin. (See, e.g., Pastan. et al., Cell (1986), 47:641, and Sharkey and Goldenberg, CA Cancer J Clin. 2006 Jul-Aug;56(4):226-43.) Additional toxins suitable for use herein are known to those of skill in the art and are disclosed in U.S. 6,077,499.

Yet another class of therapeutic agent may comprise one or more immunomodulators. Immunomodulators of use may be selected from a cytokine, a stem cell growth factor, a lymphotoxin, an hematopoietic factor, a colony stimulating factor (CSF), an interferon (IFN), erythropoietin, thrombopoietin and a combination thereof. Specifically useful are lymphotoxins such as tumor necrosis factor (TNF), hematopoietic factors, such as interleukin (IL), colony stimulating factor, such as granulocyte-colony stimulating factor (G-CSF) or granulocyte macrophage-colony stimulating factor (GM-CSF), interferon, such as interferons-α, -β, -γ or -λ, and stem cell growth factor, such as that designated "S1 factor". Included among the cytokines are growth hormones such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; prostaglandin, fibroblast growth factor; prolactin; placental lactogen, OB protein; tumor necrosis factor-a and - β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF- α and TGF- β; insulin-like growth factor-I and -II; erythropoietin (EPO); osteoinductive factors; interferons such as interferon-α, -β, -γ and -λ; colony stimulating factors (CSFs) such as macrophage-CSF (M-CSF); interleukins (ILs) such as IL-1, IL-1α, IL-2, IL-3, IL-4, IL-5, IL-6, IL-7, IL-8, IL-9, IL-10, IL-11, IL-12; IL-13, IL-14, IL-15, IL-16, IL-17, IL-18, IL-21, IL-25, LIF, kit-ligand or FLT-3, angiostatin, thrombospondin, endostatin, tumor necrosis factor and lymphotoxin (LT). As used herein, the term cytokine includes proteins from natural sources or from recombinant cell culture and biologically active equivalents of the native sequence cytokines.

Chemokines of use include RANTES, MCAF, MIP1-alpha, MIP1-Beta and IP-10.

### Formulation and Administration

Suitable routes of administration of the conjugates include, without limitation, oral, parenteral, subcutaneous, rectal, transmucosal, intestinal administration, intramuscular, intramedullary, intrathecal, direct intraventricular, intravenous, intravitreal, intraperitoneal, intranasal, or intraocular injections. The preferred routes of administration are parenteral. Alternatively, one may administer the compound in a local rather than systemic manner, for example, via injection of the compound directly into a solid tumor.

Immunoconjugates can be formulated according to known methods to prepare pharmaceutically useful compositions, whereby the immunoconjugate is combined in a mixture with a pharmaceutically suitable excipient. Sterile phosphate-buffered saline is one example of a pharmaceutically suitable excipient. Other suitable excipients are well-known to those in the art. See, for example, Ansel et al., PHARMACEUTICAL DOSAGE FORMS AND DRUG DELIVERY SYSTEMS, 5th Edition (Lea & Febiger 1990), and Gennaro (ed.), REMINGTON'S PHARMACEUTICAL SCIENCES, 18th Edition (Mack Publishing Company 1990), and revised editions thereof.

In a preferred embodiment, the immunoconjugate is formulated in Good's biological buffer (pH 6-7), using a buffer selected from the group consisting of N-(2-acetamido)-2-aminoethanesulfonic acid (ACES); N-(2-acetamido)iminodiacetic acid (ADA); N,N-bis(2-hydroxyethyl)-2-aminoethanesulfonic acid (BES); 4-(2-hydroxyethyl)piperazine-1-ethanesulfonic acid (HEPES); 2-(N-morpholino)ethanesulfonic acid (MES); 3-(N-morpholino)propanesulfonic acid (MOPS); 3-(N-morpholinyl)-2-hydroxypropanesulfonic acid (MOPSO); and piperazine-N,N'-bis(2-ethanesulfonic acid) [Pipes]. More preferred buffers are MES or MOPS, preferably in the concentration range of 20 to 100 mM, more preferably about 25 mM. Most preferred is 25 mM MES, pH 6.5. The formulation may further comprise 25 mM trehalose and 0.01% v/v polysorbate 80 as excipients, with the final buffer concentration modified to 22.25 mM as a result of added excipients. The preferred method of storage is as a lyophilized formulation of the conjugates, stored in the temperature range of -20 °C to 2 °C, with the most preferred storage at 2 °C to 8 °C.

The immunoconjugate can be formulated for intravenous administration via, for example, bolus injection, slow infusion or continuous infusion. Preferably, the antibody of the present invention is infused over a period of less than about 4 hours, and more preferably, over a period of less than about 3 hours. For example, the first 25-50 mg could be infused within 30 minutes, preferably even 15 min, and the remainder infused over the next 2-3 hrs. Formulations for injection can be presented in unit dosage form, e.g., in ampoules or in multidose containers, with an added preservative. The compositions can take such forms as suspensions, solutions or emulsions in oily or aqueous vehicles, and can contain formulatory agents such as suspending, stabilizing and/or dispersing agents. Alternatively, the active ingredient can be in powder form for constitution with a suitable vehicle, e.g., sterile pyrogen-free water, before use.

Additional pharmaceutical methods may be employed to control the duration of action of the therapeutic conjugate. Control release preparations can be prepared through the use of polymers to complex or adsorb the immunoconjugate. For example, biocompatible polymers include matrices of poly(ethylene-co-vinyl acetate) and matrices of a polyanhydride copolymer of a stearic acid dimer and sebacic acid. Sherwood et al., Bio/Technology 10: 1446 (1992). The rate of release of an immunoconjugate from such a matrix depends upon the molecular weight of the immunoconjugate, the amount of immunoconjugate within the matrix, and the size of dispersed particles. Saltzman et al., Biophys. J. 55: 163 (1989); Sherwood *et al*., *supra.* Other solid dosage forms are described in Ansel et al., PHARMACEUTICAL DOSAGE FORMS AND DRUG DELIVERY SYSTEMS, 5th Edition (Lea & Febiger 1990), and Gennaro (ed.), REMINGTON'S PHARMACEUTICAL SCIENCES, 18th Edition (Mack Publishing Company 1990), and revised editions thereof.

Generally, the dosage of an administered immunoconjugate for humans will vary depending upon such factors as the patient's age, weight, height, sex, general medical condition and previous medical history. It may be desirable to provide the recipient with a dosage of immunoconjugate that is in the range of from about 1 mg/kg to 24 mg/kg as a single intravenous infusion, although a lower or higher dosage also may be administered as circumstances dictate. A dosage of 1-20 mg/kg for a 70 kg patient, for example, is 70-1,400 mg, or 41-824 mg/m² for a 1.7-m patient. The dosage may be repeated as needed, for example, once per week for 4-10 weeks, once per week for 8 weeks, or once per week for 4 weeks. It may also be given less frequently, such as every other week for several months, or monthly or quarterly for many months, as needed in a maintenance therapy. Preferred dosages may include, but are not limited to, 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, 20 mg/kg, 22 mg/kg and 24 mg/kg. Any amount in the range of 1 to 24 mg/kg may be used. The dosage is preferably administered multiple times, once or twice a week. A minimum dosage schedule of 4 weeks, more preferably 8 weeks, more preferably 16 weeks or longer may be used. The schedule of administration may comprise administration once or twice a week, on a cycle selected from the group consisting of: (i) weekly; (ii) every other week; (iii) one week of therapy followed by two, three or four weeks off; (iv) two weeks of therapy followed by one, two, three or four weeks off; (v) three weeks of therapy followed by one, two, three, four or five week off; (vi) four weeks of therapy followed by one, two, three, four or five week off; (vii) five weeks of therapy followed by one, two, three, four or five week off; and (viii) monthly. The cycle may be repeated 4, 6, 8, 10, 12, 16 or 20 times or more.

Alternatively, an immunoconjugate may be administered as one dosage every 2 or 3 weeks, repeated for a total of at least 3 dosages. Or, twice per week for 4-6 weeks. If the dosage is lowered to approximately 200-300 mg/m² (340 mg per dosage for a 1.7-m patient, or 4.9 mg/kg for a 70 kg patient), it may be administered once or even twice weekly for 4 to 10 weeks. Alternatively, the dosage schedule may be decreased, namely every 2 or 3 weeks for 2-3 months. It has been determined, however, that even higher doses, such as 12 mg/kg once weekly or once every 2-3 weeks can be administered by slow i.v. infusion, for repeated dosing cycles. The dosing schedule can optionally be repeated at other intervals and dosage may be given through various parenteral routes, with appropriate adjustment of the dose and schedule.

In preferred embodiments of the invention as defined in the claims, the immunoconjugates are for use in the therapy of cancer. Examples of cancers include, but are not limited to, carcinoma, lymphoma, glioblastoma, melanoma, sarcoma, and leukemia, myeloma, or lymphoid malignancies. More particular examples of such cancers are noted below and include: squamous cell cancer (e.g., epithelial squamous cell cancer), Ewing sarcoma, Wilms tumor, astrocytomas, lung cancer including small-cell lung cancer, non-small cell lung cancer, adenocarcinoma of the lung and squamous carcinoma of the lung, cancer of the peritoneum, gastric or stomach cancer including gastrointestinal cancer, pancreatic cancer, glioblastoma multiforme, cervical cancer, ovarian cancer, liver cancer, bladder cancer, hepatoma, hepatocellular carcinoma, neuroendocrine tumors, medullary thyroid cancer, differentiated thyroid carcinoma, breast cancer, ovarian cancer, colon cancer, rectal cancer, endometrial cancer or uterine carcinoma, salivary gland carcinoma, kidney or renal cancer, prostate cancer, vulvar cancer, anal carcinoma, penile carcinoma, as well as head-and-neck cancer. The term "cancer" includes primary malignant cells or tumors (e.g., those whose cells have not migrated to sites in the subject's body other than the site of the original malignancy or tumor) and secondary malignant cells or tumors (e.g., those arising from metastasis, the migration of malignant cells or tumor cells to secondary sites that are different from the site of the original tumor).

Other examples of cancers or malignancies include, but are not limited to: Acute Childhood Lymphoblastic Leukemia, Acute Lymphoblastic Leukemia, Acute Lymphocytic Leukemia, Acute Myeloid Leukemia, Adrenocortical Carcinoma, Adult (Primary) Hepatocellular Cancer, Adult (Primary) Liver Cancer, Adult Acute Lymphocytic Leukemia, Adult Acute Myeloid Leukemia, Adult Hodgkin's Lymphoma, Adult Lymphocytic Leukemia, Adult Non-Hodgkin's Lymphoma, Adult Primary Liver Cancer, Adult Soft Tissue Sarcoma, AIDS-Related Lymphoma, AIDS-Related Malignancies, Anal Cancer, Astrocytoma, Bile Duct Cancer, Bladder Cancer, Bone Cancer, Brain Stem Glioma, Brain Tumors, Breast Cancer, Cancer of the Renal Pelvis and Ureter, Central Nervous System (Primary) Lymphoma, Central Nervous System Lymphoma, Cerebellar Astrocytoma, Cerebral Astrocytoma, Cervical Cancer, Childhood (Primary) Hepatocellular Cancer, Childhood (Primary) Liver Cancer, Childhood Acute Lymphoblastic Leukemia, Childhood Acute Myeloid Leukemia, Childhood Brain Stem Glioma, Childhood Cerebellar Astrocytoma, Childhood Cerebral Astrocytoma, Childhood Extracranial Germ Cell Tumors, Childhood Hodgkin's Disease, Childhood Hodgkin's Lymphoma, Childhood Hypothalamic and Visual Pathway Glioma, Childhood Lymphoblastic Leukemia, Childhood Medulloblastoma, Childhood Non-Hodgkin's Lymphoma, Childhood Pineal and Supratentorial Primitive Neuroectodermal Tumors, Childhood Primary Liver Cancer, Childhood Rhabdomyosarcoma, Childhood Soft Tissue Sarcoma, Childhood Visual Pathway and Hypothalamic Glioma, Chronic Lymphocytic Leukemia, Chronic Myelogenous Leukemia, Colon Cancer, Cutaneous T-Cell Lymphoma, Endocrine Pancreas Islet Cell Carcinoma, Endometrial Cancer, Ependymoma, Epithelial Cancer, Esophageal Cancer, Ewing's Sarcoma and Related Tumors, Exocrine Pancreatic Cancer, Extracranial Germ Cell Tumor, Extragonadal Germ Cell Tumor, Extrahepatic Bile Duct Cancer, Eye Cancer, Female Breast Cancer, Gaucher's Disease, Gallbladder Cancer, Gastric Cancer, Gastrointestinal Carcinoid Tumor, Gastrointestinal Tumors, Germ Cell Tumors, Gestational Trophoblastic Tumor, Hairy Cell Leukemia, Head and Neck Cancer, Hepatocellular Cancer, Hodgkin's Lymphoma, Hypergammaglobulinemia, Hypopharyngeal Cancer, Intestinal Cancers, Intraocular Melanoma, Islet Cell Carcinoma, Islet Cell Pancreatic Cancer, Kaposi's Sarcoma, Kidney Cancer, Laryngeal Cancer, Lip and Oral Cavity Cancer, Liver Cancer, Lung Cancer, Lymphoproliferative Disorders, Macroglobulinemia, Male Breast Cancer, Malignant Mesothelioma, Malignant Thymoma, Medulloblastoma, Melanoma, Mesothelioma, Metastatic Occult Primary Squamous Neck Cancer, Metastatic Primary Squamous Neck Cancer, Metastatic Squamous Neck Cancer, Multiple Myeloma, Multiple Myeloma/Plasma Cell Neoplasm, Myelodysplastic Syndrome, Myelogenous Leukemia, Myeloid Leukemia, Myeloproliferative Disorders, Nasal Cavity and Paranasal Sinus Cancer, Nasopharyngeal Cancer, Neuroblastoma, Non-Hodgkin's Lymphoma, Nonmelanoma Skin Cancer, Non-Small Cell Lung Cancer, Occult Primary Metastatic Squamous Neck Cancer, Oropharyngeal Cancer, Osteo-/Malignant Fibrous Sarcoma, Osteosarcoma/Malignant Fibrous Histiocytoma, Osteosarcoma/Malignant Fibrous Histiocytoma of Bone, Ovarian Epithelial Cancer, Ovarian Germ Cell Tumor, Ovarian Low Malignant Potential Tumor, Pancreatic Cancer, Paraproteinemias, Polycythemia vera, Parathyroid Cancer, Penile Cancer, Pheochromocytoma, Pituitary Tumor, Primary Central Nervous System Lymphoma, Primary Liver Cancer, Prostate Cancer, Rectal Cancer, Renal Cell Cancer, Renal Pelvis and Ureter Cancer, Retinoblastoma, Rhabdomyosarcoma, Salivary Gland Cancer, Sarcoidosis Sarcomas, Sezary Syndrome, Skin Cancer, Small Cell Lung Cancer, Small Intestine Cancer, Soft Tissue Sarcoma, Squamous Neck Cancer, Stomach Cancer, Supratentorial Primitive Neuroectodermal and Pineal Tumors, T-Cell Lymphoma, Testicular Cancer, Thymoma, Thyroid Cancer, Transitional Cell Cancer of the Renal Pelvis and Ureter, Transitional Renal Pelvis and Ureter Cancer, Trophoblastic Tumors, Ureter and Renal Pelvis Cell Cancer, Urethral Cancer, Uterine Cancer, Uterine Sarcoma, Vaginal Cancer, Visual Pathway and Hypothalamic Glioma, Vulvar Cancer, Waldenström's macroglobulinemia, Wilms' tumor, and any other hyperproliferative disease, besides neoplasia, located in an organ system listed above.

The technical teaching of the disclosure may be used to treat malignant or premalignant conditions and to prevent progression to a neoplastic or malignant state, including but not limited to those disorders described above. Such uses are indicated in conditions known or suspected of preceding progression to neoplasia or cancer, in particular, where non-neoplastic cell growth consisting of hyperplasia, metaplasia, or most particularly, dysplasia has occurred (for review of such abnormal growth conditions, see Robbins and Angell, Basic Pathology, 2d Ed., W. B. Saunders Co., Philadelphia, pp. 68-79 (1976)).

Dysplasia is frequently a forerunner of cancer, and is found mainly in the epithelia. It is the most disorderly form of non-neoplastic cell growth, involving a loss in individual cell uniformity and in the architectural orientation of cells. Dysplasia characteristically occurs where there exists chronic irritation or inflammation. Dysplastic disorders which can be treated include, but are not limited to, anhidrotic ectodermal dysplasia, anterofacial dysplasia, asphyxiating thoracic dysplasia, atriodigital dysplasia, bronchopulmonary dysplasia, cerebral dysplasia, cervical dysplasia, chondroectodermal dysplasia, cleidocranial dysplasia, congenital ectodermal dysplasia, craniodiaphysial dysplasia, craniocarpotarsal dysplasia, craniometaphysial dysplasia, dentin dysplasia, diaphysial dysplasia, ectodermal dysplasia, enamel dysplasia, encephalo-ophthalmic dysplasia, dysplasia epiphysialis hemimelia, dysplasia epiphysialis multiplex, dysplasia epiphysialis punctata, epithelial dysplasia, faciodigitogenital dysplasia, familial fibrous dysplasia of jaws, familial white folded dysplasia, fibromuscular dysplasia, fibrous dysplasia of bone, florid osseous dysplasia, hereditary renal-retinal dysplasia, hidrotic ectodermal dysplasia, hypohidrotic ectodermal dysplasia, lymphopenic thymic dysplasia, mammary dysplasia, mandibulofacial dysplasia, metaphysial dysplasia, Mondini dysplasia, monostotic fibrous dysplasia, mucoepithelial dysplasia, multiple epiphysial dysplasia, oculoauriculovertebral dysplasia, oculodentodigital dysplasia, oculovertebral dysplasia, odontogenic dysplasia, opthalmomandibulomelic dysplasia, periapical cemental dysplasia, polyostotic fibrous dysplasia, pseudoachondroplastic spondyloepiphysial dysplasia, retinal dysplasia, septo-optic dysplasia, spondyloepiphysial dysplasia, and ventriculoradial dysplasia.

Additional pre-neoplastic disorders which can be treated include, but are not limited to, benign dysproliferative disorders (e.g., benign tumors, fibrocystic conditions, tissue hypertrophy, intestinal polyps or adenomas, and esophageal dysplasia), leukoplakia, keratoses, Bowen's disease, Farmer's Skin, solar cheilitis, and solar keratosis.

In preferred embodiments of the invention as defined in the claims, growth, progression, and/or metastasis of cancers, in particular those listed above, is inhibited.

Additional hyperproliferative diseases, disorders, and/or conditions include, but are not limited to, progression, and/or metastases of malignancies and related disorders such as leukemia (including acute leukemias; e.g., acute lymphocytic leukemia, acute myelocytic leukemia [including myeloblastic, promyelocytic, myelomonocytic, monocytic, and erythroleukemia]) and chronic leukemias (e.g., chronic myelocytic [granulocytic] leukemia and chronic lymphocytic leukemia), polycythemia vera, lymphomas (e.g., Hodgkin's disease and non-Hodgkin's disease), multiple myeloma, Waldenström's macroglobulinemia, heavy chain disease, and solid tumors including, but not limited to, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, emangioblastoma, acoustic neuroma, oligodendroglioma, meningioma, melanoma, neuroblastoma, and retinoblastoma.

### Triple Negative Breast Cancer (TNBC)

In specific embodiments of the invention as defined in the claims, the ADC immunoconjugate and standard anti-cancer therapy is for use in neoadjuvant treatment of triple negative breast cancer. TNBC, as the term suggests, refers to breast cancers that are devoid of the expression of estrogen and progesterone receptors (ER, PR), and also HER2 (ERBB2). This type comprises about 15-20% of all invasive breast cancers and is highly malignant. TNBC patients show higher rates of distant recurrence and death compared to other breast cancers (Millikan et al., Breast Cancer Res Treat 2008; 109:123-139), with the median survival of those with metastatic disease being only 13 months (Kassam et al., Clin Breast Cancer 2009; 9:29-33). In terms of prevalence, TNBC is more frequent in younger patients, in *BRCA1* mutation carriers, and in specific ethnic groups, such as African-Americans and Hispanic women (Bauer et al., Cancer 2007, 109:1721-1728; Sorlie et al., Proc Natl Acad Sci USA 2003, 100: 8418-8423; 26-28; Foulkes et al., N Engl J Med 2010, 363: 1938-1948). This suggests that a germline genetic background plays a role in the transcription and differentiation of TNBC. Histologically, most TNBC tumors are invasive ductal carcinomas, and are higher histologic grade, larger tumor size, and most often are lymph node positive at diagnosis (Dent et al., Clin Cancer Res 2007, 13:4429-4434). Prognosis is related to metastatic behavior, and the different subtypes show different patterns of metastasis. Breast cancer metastasizes commonly to the bone, but basal-like disease is dominated by metastasis to brain, lung, and distant lymph nodes (Kennecke et al., J Clin Oncol 2010, 28: 3271-3277; Sihto et al., Breast Cancer Res 2011, 13:R87). The non-basal forms show a similar pattern of metastasis, but with more frequent metastasis to liver.

Lehmann et al. (Lehmann et al., J Clin Invest 2011, 121(7):2750-2767) proposed six specific molecular subtypes for TNBC: two basal-like, BL1 and BL2, which are the most prevalent and received this designation because of being similar to the basal-like intrinsic subtype, and expression of genes expressed by such cells, such as cytokeratins 5, 6 or 17; a third immunomodulatory subtype; mesenchymal (M) and mesenchymal stem-like (MSL) subtypes; and a sixth being luminal androgen receptor (LAR).

The basal-like tumors show high proliferation and frequent mutations of *TP53,* and are associated with *BRCA1* mutation status. Such tumors are thought to have deregulated DNA repair by deficiency in homologous recombination with BRCA inactivated in *BRCA* mutation carriers (Rehman et al., Nat Rev Clin Oncol 2010, 7:718-724). BL1 sublines have been found to be preferentially responsive to cisplatin, which causes DNA damage through the formation of guanine cross-linkages. Several clinical trials have evaluated and continue to evaluate the role of platinum agents in the basal-like TNBCs (Ademuyiwa et al., J Onc 2013;2013:219869). The LAR type may be responsive to anti-androgen receptor treatment, which is under clinical evaluation.

Treatment of TNBC, similar to typical breast cancer, involves surgery, radiotherapy, and chemotherapy, since no targeted therapies are currently available. The most active chemotherapeutics are anthracyclines and taxanes. A meta-analysis of seven neoadjuvant clinical trials showed a pathologic complete response (absence of invasive breast cancer in the breast and lymph nodes) was achieved in 36% of patients undergoing therapy with anthracyclines and taxanes (von Minckwitz et al. J Clin Oncol 2012, 30(15): 1796-1804). A summary of various trials of neoadjuvant therapy in TNBC has indicated that such common cancer drugs as doxorubin, cyclophosphamide, doxorubicin, taxane, epirubicin, docetaxel, paclitaxel, bevacizumab, gemcitabine, methotrexate, 5-fluorouracil, capcitabine, vincristine, cisplatin, and carboplatin, given in various combinations, resulted in pCR rates of 28-52% in trials involving more than 100 patients (Engebraaten et al. Am J Pathol 2013 Oct;183(4): 1064-1074). Although platinum-containing regimens are active in breast cancer, TNBC results with such regimens have not been equally superior (Engebraaten et al. Am J Pathol 2013 Oct;183(4):1064-1074). The addition of carboplatin to a regimen of epirubicin, cyclophosphamide, and docataxel did not show an advantage of adding carboplatin in the neoadjuvant setting (Alba et al. Breast Cancer Res Treat 2012 Nov;136(2):487-493).

In general, patients with TNBC have a higher response to chemotherapy than those with ER-positive, HER2-negative tumors, but paradoxically they have a poorer outcome (Carey et al., Clin Cancer Res. 2007 Apr 15;13(8):2329-2334). The rate of pCR in TNBC can be 20% or higher (Engebraaten et al. Am J Pathol 2013 Oct;183(4): 1064-1074), and those responding have a good long-term outcome, but the majority of TNBC patients do not achieve pCR, and thus progress more aggressively than patients with ER⁺ disease (Carey et al., Clin Cancer Res. 2007 Apr 15;13(8):2329-2334; Liedtke et al., J Clin Oncol. 2008 Mar 10;26(8):1275-1281). For TNBC patients who do not achieve a pCR, the recurrence rate is between 40 and 50% over a period of five years (von Minckwitz et al. J Clin Oncol 2012, 30(15): 1796-1804; Liedtke et al., J Clin Oncol. 2008 Mar 10;26(8):1275-1281), confirming the poor outcome despite a likely higher chemosensitivity of TNBC patients. This emphasizes the critical unmet need for novel therapeutics to achieve a higher rate of pCR in TNBC.

Biologic therapeutic agents also are being evaluated in patients with TNBC, such as PARP inhibitors and vascular endothelial growth factor (VEGF) inhibitors (Schneider et al., J Clin Oncol 2005;23(8): 1782-1790; von Minckwitz et al., N Eng J Med 2012; 366(4): 299-309). Indeed, because TNBC has clinical similarities to BRCA-associated breast cancer, PARP inhibitors are attractive candidates for the therapy of TNBC, but as yet this has not been substantiated. However, IMMU-132, which has a similar mechanism of action as a topoisomerase I inhibitor (Cardillo et al., Clin Cancer Res 2011; 17:3157-3169), has shown clinical regressions as a monotherapy in patients with metastatic TNBC, as well as in TNBC cell lines (Cardillo et al., Clin Cancer Res 2011; 17:3157-3169 and Examples below).

### IMMU-132 in TNBC Neoadjuvant Therapy

The invention as defined in the claims concerns the neoadjuvant use in TNBC of IMMU-132, an ADC comprising multiple copies of the SN-38 camptothecin type drug attached to the anti-TROP-2 hRS7 antibody. SN-38 is the active metabolite of CPT-11 (irinotecan), which is not a standard drug in breast cancer, but may offer a unique therapeutic benefit in TNBC. It has been postulated that TNBC is similar in its phenotype to cancers expressing the *BRCA1* mutation, and therefore may be responsive to agents that are effective in such *BRCA1* patients, such as platinum and alkylating agents, as well as topoisomerase I inhibitors (such as camptothecins like topotecan and irinotecan), due to deficiencies in the DNA repair mechanisms associated with *BRCA.* These act by inducing breaks in doublestranded DNA, affecting DNA repair and causing cell death (Hastak et al., Cancer Res 2010;70:7970-7980). A similar mechanism of action, inhibiting poly (adenosine diphosphate ribose) polymerase (PARP), which also plays a critical role in DNA repair, like BRCA, but PARP inhibitors cause damage to one strand of DNA, which cannot be repaired by homologous recombination due to *BRCA* mutation and PARP inhibition.

We have found that the immunoconjugate IMMU-132, comprising SN-38, a topoisomerase I inhibitor, conjugated to a humanized monoclonal antibody (mAb) that selectively targets a protein (TROP-2) enhanced in expression in TNBC (and other epithelial cancers), has a similar effect of breaking DNA strands via PARP inhibition (Cardillo et al., Clin Cancer Res 2011; 17:3157-3169). Therefore, this immunoconjugate may replace platinum and other DNA-damaging drugs (e.g., cisplatin, carboplatin), which have high toxicities, in a combination therapy of TNBC. Current results with IMMU-132 in patients with metastatic solid cancers, including metastatic TNBC, indicate that it is better tolerated than the parental irinotecan drug because of its apparent high therapeutic index, with more manageable toxicities than when irinotecan is given. Although irinotecan and topoisomerase I inhibitors are not known to be active in breast cancers, including TNBC, current trials in TNBC with IMMU-132 suggest that it is effective in metastatic TNBC (Starodub et al., J Clin Oncol 32:5s, 2014 (suppl; abstr 3032)), possibly because a higher ratio of this DNA-damaging drug is targeted to the tumor sites by selective binding of the carrier antibody to the TROP-2 protein on TNBC cells, than when irinotecan is given (based on preclinical findings discussed in the Examples below). Indeed, our preclinical studies have confirmed activity of IMMU-132 in TNBC xenograft models. Other attributes of IMMU-132 are that it involves an internalizing antibody (RS7), thus selectively incorporating the toxic drug, SN-38, into the targeted cancer cells. Further, TROP-2, the target of IMMU-132, is a calcium signal-transducing protein expressed by many epithelial cancers in higher amounts than normal cells, and has been shown in some cancers to be a prognostic indicator for increased malignancy. The current experience with IMMU-132 in both a variety of advanced solid cancers, as well as TNBC, confirms the preclinical human cancer xenograft study results, described below and published in part (Cardillo et al., Clin Cancer Res 2011; 17:3157-3169).

TROP-2, the target antigen of the IMMU-132 immunoconjugate, is a 36-kDa cell-surface glycoprotein expressed on a variety of human carcinomas including lung, breast, colorectal, pancreas, prostate and ovarian (see, e.g., Lipinski et al. Proc Natl Acad Sci USA 1981;78:5147-5150; Stein et al. Cancer Res 1990;50:1330-1336; Alberti et al. Hybridoma 1992; 11:539-545; Wang et al. Mol Cancer Ther 2008;7:280-285; Cubas et al. Biochim Biophys Acta 2009; 1796:309-314). It has been shown to function as a calcium signal transducer (Ripani et al. Int J Cancer 1998; 76:671-676) and linked to cell migration and anchorage-independent growth (Hastak et al., Cancer Res 2010;70:7970-7980). Importantly, high expression of TROP-2 is associated with more aggressive disease and a poor prognosis, making it an ideal target for cancer therapy (Shimada et al. Cancer Sci 2005; 96:668-675; Wang et al. Mol Cancer Ther 2008;7:280-285; Cubas et al. Biochim Biophys Acta 2009; 1796:309-314).

SN-38 cannot be used as such due to its insolubility in aqueous media. The bioconversion of irinotecan to the active drug, SN-38, is very inefficient, and is also patient-variable. In order to make SN-38 directly available to target sites, an antibody-drug conjugate (ADC) of SN-38 was prepared with an anti-TROP-2 (hRS7) antibody. A number of linkers were examined, and one, 'CL2A', was chosen as the optimally performing linker (Cardillo et al., Clin Cancer Res 2011; 17:3157-3169; Moon et al. J Med Chem 2008. 51, 6916-6926; Govindan et al. Clin Cancer Res 2009;15:6052-6061; Govindan et al. Mol Cancer Ther 2013 Jun;12(6):968-978). The conjugate of the SN-38 derivative, CL2A-SN-38, indicating the conjugation site and the drug cleavage site is shown in **FIG. 1**. The conjugation of CL2A-SN-38 to mildly reduced hRS7 had been described (Moon et al. J Med Chem 2008. 51, 6916-6926; Govindan et al. Clin Cancer Res 2009;15:6052-6061). Antigen-binding for TROP-2⁺ cell lines was preserved in the ADC, which also exhibited single-digit nanomolar potency similar to that for free drug, in a number of TROP-2⁺ cell lines *in vitro.* The average drug-antibody substitution ratio (DAR) was determined to be 7.6 by mass spectral measurements, and the same was corroborated by absorbance measurements. The level of unconjugated free drug was <5%, within the specification limit for clinical grade preparations.

Large-scale preparations of the ADC have been carried out on 200-gram scale of the antibody, under cGMP conditions and QA oversight of both the CL2A-SN-38 component and the ADC. Eight such preparations (8 x 200 grams) of the conjugate had been prepared to-date, with product stored at 2-8 °C as lyophilized preparations in 100-mg aliquots. The stability of the preparation to storage has been documented for 3 years now.

In human serum, at 37 °C *in vitro,* hRS7-CL2A-SN-38 has been shown to release 50% of free drug in -24 h. The CL2A linker leads to much higher bioavailability of free drug compared to the conjugate with a different, highly stable, linker. This is because the liberation of free drug is not contingent upon internalization of the antibody conjugate and subsequent cellular processing, which also makes it attractive in situations where the antigen density on the tumor surface is low.

### Pathologic Complete Response (pCR) as a Surrogate Endpoint in TNBC

pCR has become an accepted endpoint for neoadjuvant therapy trials in patients with TNBC (Bardia & Baselga, Clin Cancer Res 2013 Dec 1;19(23):6360-6370; Ademuyiwa et al., J Onc 2013;2013:219869; Food and Drug Administration. Draft Guidance for Industry: Pathologic Complete Response in Neoadjuvant Treatment of High-Risk Early-Stage Breast Cancer: Use as an Endpoint to Support Accelerated Approval. May 2012). pCR is defined in the FDA Guidance document "as the absence of any residual invasive cancer on hematoxylin and eosin evaluation of the resected breast specimen and *all sampled* ipsilateral lymph nodes following completion of neoadjuvant systemic therapy (i.e., ypT0 ypN0 in the current AJCC staging system)" (Food and Drug Administration. Draft Guidance for Industry: Pathologic Complete Response in Neoadjuvant Treatment of High-Risk Early-Stage Breast Cancer: Use as an Endpoint to Support Accelerated Approval. May 2012) (italics emphasis added). Although this allows drugs to be tested quickly and in relatively smaller numbers of patients than in the setting of metastatic disease, with assessment of efficacy at surgery possibly leading to accelerated (provisional) drug approval based on surgical results within about 6 months or earlier after onset of the neoadjuvant therapy, follow-up to determine event-free survival (EFS) and overall survival (OS) rates are still needed to substantiate clinical benefit leading to final drug approval. This pathway for accelerated approval means that the drug is made available to general use much earlier, increasing its inclusion in a variety of clinical studies and settings, which can offer very rapid benefit to women facing surgery for TNBC. If the toxicity profile of IMMU-132 is truly better than the platinum and PARP drugs that address a similar mechanism of action, then addition of this novel therapeutic may invoke a paradigm change in the neoadjuvant (and perhaps also adjuvant and metastatic settings) of TNBC therapy. Also, since the target of IMMU-132, TROP-2, is also expressed in high prevalence in non-triple-negative (TN) tumors, this agent may also show unique activity in these other breast cancer types. Promising results in the therapy of metastatic and early TNBC would encourage studying this agent also in patients with non-TN cancers.

Among neoadjuvant trials for TNBC reviewed in one meta-analysis, only 3 trials reported long-term disease-free survival and OS, and these were excellent for patients who received platinum-based neoadjuvant therapy (Petrelli et al. Breast Cancer Res Treat 2014; 144:223-232). Patients with TNBC who achieved a pCR in breast and axilla (ypT0N0) had a better event-free survival than patients with residual disease. In yet another analysis of 12 international trials involving 11,955 patients, pCR in TNBC proved to be a surrogate endpoint for improved EFS and OS (Cortazar et al. Lancet 2014 Jul 12;384(9938): 164-172). In terms of EFS, such as at 3 years, pCR patients showed a 90% rate compared to about 60% for non-pCR TNBC patients (Cortazar et al. Lancet 2014 Jul 12;384(9938): 164-172).

In conclusion, neoadjuvant chemotherapy has become a standard of care for a responsive subgroup of patients with TNBC, despite the fact that no specific therapy regimen has been established as preferred; only use of taxanes and anthracyclines seem to be standard modalities. Adding a new targeted therapeutic that shows good activity in metastatic TNBC patients who have failed prior systemic treatments may be best and most rapidly evaluated in a neoadjuvant setting, where it is added to conventional combination therapy and compared to the same therapy without this candidate therapeutic. The testing of novel agents in comparator trials may help establish a standard therapy, and also may permit the expansion of the responsive types to other subgroups and to increase the pCR response rate beyond the levels being achieved currently at high levels of toxicity to the patients. It is generally agreed that anthracyclines combined with taxanes and cyclophosphamide provide a high rate of pCR in TNBC (von Minckwitz & Fontanella, Breast 2013 Aug;22 Suppl 2:S149-S151), this comes with a high toxicity. The addition of platinum agents, such as cisplatin and carboplatin, are also under investigation. The studies below assess the addition of IMMU-132, as a topoisomerase I inhibitor, to the TAC regimen, because carboplatin comes with increased toxicity when combined with TAC. This approach should result in more breast conservation, more tolerable treatments, and an increased cure rate.

### Kits

The instant disclosure is also related to kits containing components suitable for treating a patient. Exemplary kits may contain at least one conjugated antibody or other targeting moiety as described herein. If the composition containing components for administration is not formulated for delivery via the alimentary canal, such as by oral delivery, a device capable of delivering the kit components through some other route may be included. One type of device, for applications such as parenteral delivery, is a syringe that is used to inject the composition into the body of a subject. Inhalation devices may also be used.

The kit components may be packaged together or separated into two or more containers. In some examples, the containers may be vials that contain sterile, lyophilized formulations of a composition that are suitable for reconstitution. A kit may also contain one or more buffers suitable for reconstitution and/or dilution of other reagents. Other containers that may be used include, but are not limited to, a pouch, tray, box, tube, or the like. Kit components may be packaged and maintained sterilely within the containers. Another component that can be included is instructions to a person using a kit for its use. Various embodiments of the present disclosure are illustrated by the following examples, which are for reference.

### EXAMPLES

### Example 1. Preparation of CL2A-SN-38 Immunoconjugates

In a preferred reaction scheme for synthesis of CL2A-SN-38, EEDQ (0.382g) was added to a mixture of commercially available Fmoc-Lys(MMT)-OH (0.943g) and *p-*aminobenzyl alcohol (0.190g) in methylene choloride (10 mL) at room temperature and stirred for 4 h. Extractive work up followed by flash chromatograph yielded 1.051 g of material as white foam. Electrospray mass spectrum showed peaks at m/e 745.8 (M+H) and m/e 780.3 (M+Cl⁻), consistent with structure. The Lys(MMT)-PABOH intermediate (0.93 g) was dissolved in diethylamine (10 mL) and stirred for 2 h. After solvent removal, the residue was washed in hexane to obtain 0.6 g of the intermediate as colorless precipitate (91.6% pure by HPLC). HPLC ret. time: 2.06 min. Electrospray mass spectrum showed peaks at m/e 523.8 (M+H), m/e 546.2 (M+Na) and m/e 522.5 (M-H).

This crude intermediate (0.565g) was coupled with commercially available O-(2-azidoethyl)-O'-(N-diglycolyl-2-aminoethyl)heptaethyleneglycol ('PEG-N3', 0.627g) using EEDQ in methylene chloride (10 mL). Solvent removal and flash chromatography yielded 0.99 g of azido-PEG-Lys(MMT)-PABOH intermediate (in light yellow oil; 87% yield). Electrospray mass spectrum showed peaks at m/e 1061.3 (M+H), m/e 1082.7 (M+Na) and m/e 1058.8(M-H), consistent with structure. The intermediate 3 (0.92 g) was reacted with 10-*O*-TBDMS-SN-38-20-*O*-chloroformate in methylene chloride (10 mL) for 10 min under argon. The mixture was purified by flash chromatography to obtain 0.944g of azido-PEG-Lys(MMT)-PAB-*O*-SN-38-TBDMS (yield = 68%) as light yellow oil. To this intermediate (0.94 g) in methylene chloride (10 mL) was added a mixture of TBAF (1M in THF, 0.885 mL) and acetic acid (0.085 mL) in methylene chloride (3 mL), then stirred for 10 min. The mixture was diluted with methylene chloride (100 mL) and washed with 0.25 M sodium citrate and brine. The solvent removal yielded 0.835g of azido-PEG-Lys(MMT)-PAB-*O*-SN-38 as a yellow oily product (99% purity). Electrospray mass spectrum showed peaks at m/e 1478 (M+H), m/e 1500.6 (M+Na), m/e 1476.5 (M-H), m/e 1590.5 (M+TFA), consistent with structure.

This azido-derivatized SN-38 intermediate (0.803g) was reacted with 4-(N-maleimidomethyl)-N-(2-propynyl)cyclohexane-1-carboxamide ("MCC-yne"; 0.233 g) in methylene chloride (10 mL) in the presence of CuBr (0.0083 g,), DIEA (0.01 mL) and triphenylphosphine (0.015 g), for 18 h. Extractive work up, including washing with and 0.1M EDTA (10 mL), and flash chromatography yielded 0.891 g of MCC-PEG-Lys(MMT)-PAB-*O*-SN-38 intermediate (yield = 93%) as yellow foam. Electrospray mass spectrum showed peaks at m/e 1753.3 (M+H), m/e 1751.6 (M-H), 1864.5 (M+TFA), consistent with structure. Finally, deprotection with a mixture of dichloroacetic acid (0.3 mL) and anisole (0.03 mL) in methylene chloride (3 mL), followed by precipitation with ether yielded 0.18 g (97% yield) of CL2A-SN-38 final product as light yellow powder. Electrospray mass spectrum showed peaks at m/e 1480.7 (M+H), 1478.5 (M-H), consistent with structure.

### Conjugation to antibodies

The anti-CEACAM5 humanized MAb, hMN-14, the anti-CD22 humanized MAb, hLL2, the anti-CD20 humanized MAb, hA20, the anti-EGP-1 humanized MAb, hRS7, and anti-mucin humanized MAb, hPAM4, were conjugated to CL2A-SN-38 to prepare immunoconjugates. Each antibody was mildly reduced with Tris (2-carboxyethyl) phosphine (TCEP) in phosphate buffer at pH in the range of 7-7.4, the pH was adjusted to 6.5, and reacted with ∼ 10-fold molar excess of CL2A-SN-38 using DMSO at 5-10 % v/v as co-solvent, and incubating for 20 min at ambient temperature. Any excess thiol was capped with N-ethylmaleimide used as an aqueous solution at a 10-fold molar excess with respect to antibody.

The conjugate was purified by tangential flow filtration (TFF), using 20-30 diafiltration volumes of the final formulation buffer, 25 mM MES, pH 6.5. This method avoided cumbersome sequential purification on size-exclusion and hydrophobic columns, thereby enabling hundreds of grams of conjugates to be purified in a facile manner. The product was assayed for SN-38 by absorbance at 366 nm and correlating with standard values. The protein concentration was deduced from absorbance at 280 nm, corrected for spillover of SN-38 absorbance at this wavelength. From these, the SN-38/MAb substitution ratios (DAR) were determined. The purified conjugates were stored as lyophilized formulations in glass vials, capped under vacuum and stored in a -20 °C freezer. Drug-antibody rations (DAR) were typically in the 5-to-7 range (i.e., 5 to 7 drug moieties per antibody moiety).

The ADCs described above were purified and buffer-exchanged with 2-(N-morpholino)ethanesulfonic acid (MES), pH 6.5, and further formulated with trehalose (25 mM final concentration) and polysorbate 80 (0.01% v/v final concentration), with the final buffer concentration becoming 22.25 mM as a result of excipient addition. The formulated conjugates were lyophilized and stored in sealed vials, with storage at 2 °C - 8 °C. The lyophilized immunoconjugates were stable under the storage conditions and maintained their physiological activities.

### Example 2. Pre-Clinical Studies In Various Solid Tumors Treated With IMMU-132

*In Vitro* Characterization - A TROP-2-positive human prostate carcinoma cell line, PC-3, was used as a target to assess possible changes in antigen binding by IMMU-132 in comparison to unconjugated hRS7 IgG. As measured on three separate occasions, there was no significant difference between the binding of IMMU-132 and unconjugated hRS7 IgG (*K*_{D}-value, 0.658 ± 0.140 nM vs. 0.564 ± 0.055 nM, respectively).

Human neonatal receptor (FcRn) binding was determined by surface plasmon resonance (BIACore) analysis using a low density FcRn biosensor chip. Three binding runs using three separate sets of five dilutions for each test agent demonstrated that conjugation of SN-38 to hRS7 IgG did not significantly affect its binding affinity for FcRn (*K*_{D}-values 92.4 ± 5.7 nM and 191.9 ± 47.6 nM, respectively; *P* = 0.07).

TROP-2 is expressed in a wide range of human solid tumor cell lines, including TNBC cell lines (e.g., MDA-MB-231 and MDA-MB-268). Expression levels vary between tumor types and within types (**Table 2**). For example, there is approximately 10-fold more TROP-2 expressed by MDA-MB-468 compared to MDA-MB-231, but there is no difference when compared to the HER2⁺ SK-BR-3 tumor line. All these tumor types are sensitive to the cytotoxic effects IMMU-132. Concentrations needed to cause 50% growth inhibition (IC₅₀-values) are in the low nanomolar range. In these assays, free SN-38 tends to be more cytotoxic than the ADC, but this is most likely due to the ready availability of the drug in the free form *versus* the time it takes for the IMMMU-132 to bind to, and be taken up by the cell. There does not appear to be a correlation between TROP-2 expression levels and sensitivity to SN-38. BxPC-3 has the highest expression level of TROP-2, but there is a 4-fold difference in ICso-values between the free SN-38 and IMMU-132, whereas COLO 205 expressed approximately 8-fold less TROP-2 in comparison but exhibits only a 2-fold difference in IC₅₀-values.

| **Table 2.** TROP-2 Expression Levels and Calculated IC₅₀-values (nM) for free-SN-38 and IMMU-132 in Various Solid Cancer Tumor Lines. | | | | | |
|---|---|---|---|---|---|
| **Cell Line** | **Tumor Type** | **¹TROP-2 Expression** | **Free SN-38** | **²IMMU-132** | **³ADC/SN-38 activity** |
| Capan-1 | Pancreatic | 157,376 ± 36,976 | 6 | 9 | 1.5 |
| BxPC-3 | Pancreatic | 493,773 ± 97,779 | 1 | 4 | 4 |
| Calu-3 | NSCLC | 128,201 ± 50,708 | 7 | 20 | 2.9 |
| SK-MES-1 | Squamous Cell Lung | 29,488 ± 5,824 | 9 | 23 | 2.6 |
| NCI-N87 | Gastric | 246,857 ± 64,651 | 4 | 4 | 1 |
| COLO 205 | Colon | 58,179 ± 6,909 | 1 | 2 | 2 |
| SK-OV-4 | Ovarian | n.d. | 18 | 18 | 1 |
| PC-3 | Prostate | n.d. | 2 | 4 | 2 |
| MDA-MB-468 | TNBC | 301,603 ± 29,470 | 2 | 4 | 2 |
| MDA-MB-231 | TNBC | 32,380 ± 5,460 | 6 | 19 | 3.2 |
| SK-BR-3 | *HER2*⁺ Breast | 328,281 ± 47,996 | 2 | 3 | 1.5 |
| ¹Number of surface TROP-2 molecules per cell; ²IC₅₀-value is shown as SN-38 equivalents of IMMU-132; ³Fold-difference in IC₅₀-values between free SN-38 and ADC. n.d. Not Done (TROP-2 expression confirmed by FACS analysis, but number of copies per cell have not been determined). | | | | | |

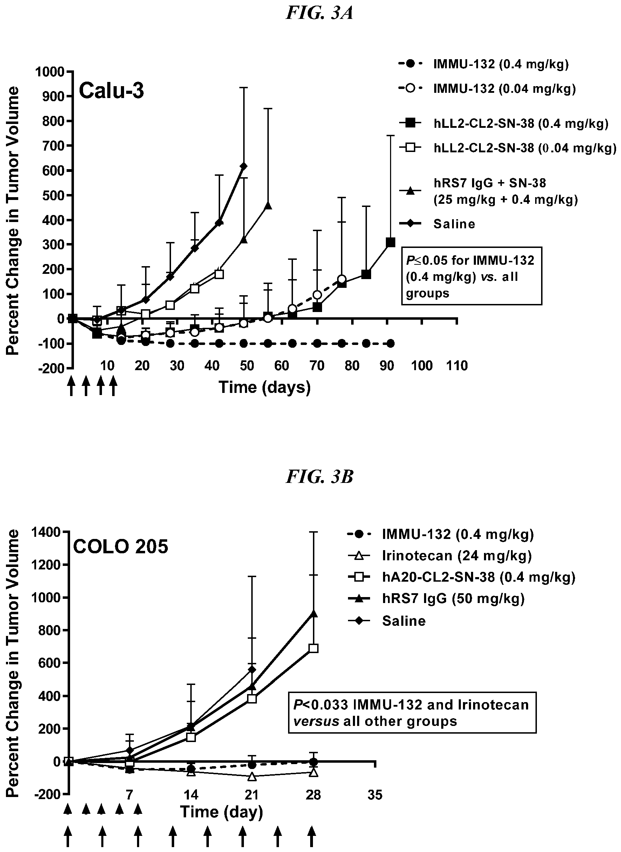

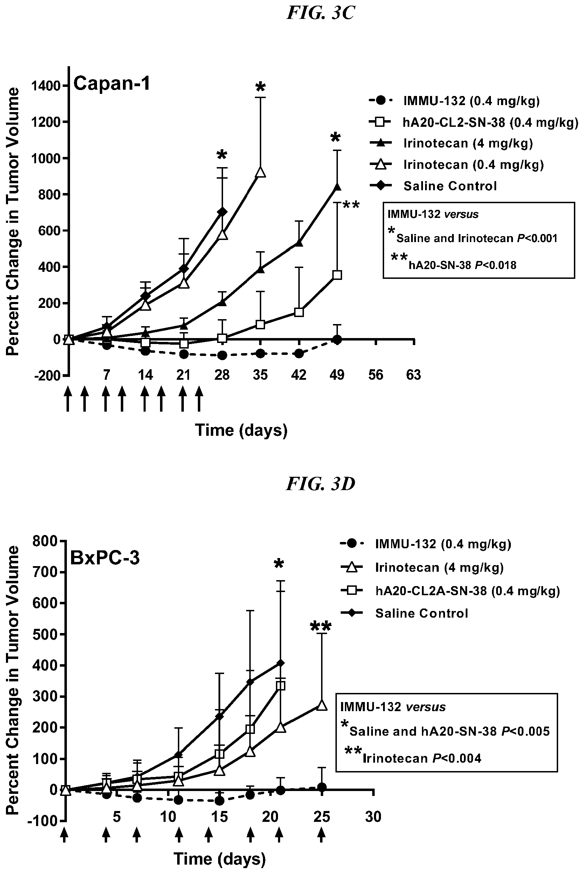

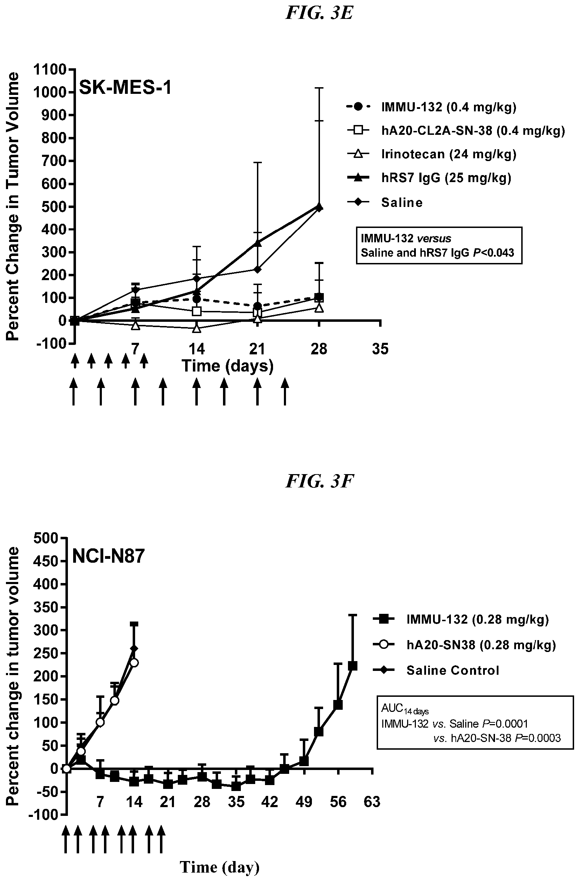

*In Vivo Efficacy in Various Solid Tumors* - Initial efficacy studies of IMMU-132 were performed in multiple solid tumor xenograft models (Cardillo et al., Clin Cancer Res 2011; 17:3157-3169). These included pancreatic, lung, colon, and gastric cancers (**FIG. 3**; doses are given in SN-38 equivalents). In all 6 solid tumors examined, IMMU-132 significantly inhibited tumor growth when compared to saline control (*P*<0.043, area under the curve AUC). In 5 of 6, the specific IMMU-132 therapy provided a significant anti-tumor response when compared to a non-tumor targeting control ADC (*P*<0.05, AUC). Additionally, IMMU-132 provided a significantly greater anti-tumor effect when compared to mice administered the equivalent of 10-fold more SN-38 in the form of irinotecan (*FIG. 3A**, C, &* **D**). Only when the MTD of irinotecan was administered to the animals was parity with the ADC achieved in these experiments (**FIG. 3B** **&** **E**). However, the total SN-38 equivalents contained in the irinotecan regimen given to mice totaled ∼ 2,400 µg, which was 37.5-fold greater than the SN-38 equivalents administered with IMMU-132 treatment regimen (64 µg). Importantly, mice convert irinotecan to SN-38 more efficiently, by as much as 5- to 10-fold higher than humans (Morton et al. Cancer Res 2000; 60(15):4206-4210; Furman et al. J Clin Oncol 1999; 17(6):1815-1824; Zamboni et al. Clin Cancer Res 1998; 4(2):455-462). Thus, even with this great irinotecan advantage in the mice, IMMU-132 provided an equivalent anti-tumor effect.

### Example 3. In Vivo Studies in TNBC Treated With IMMU-132

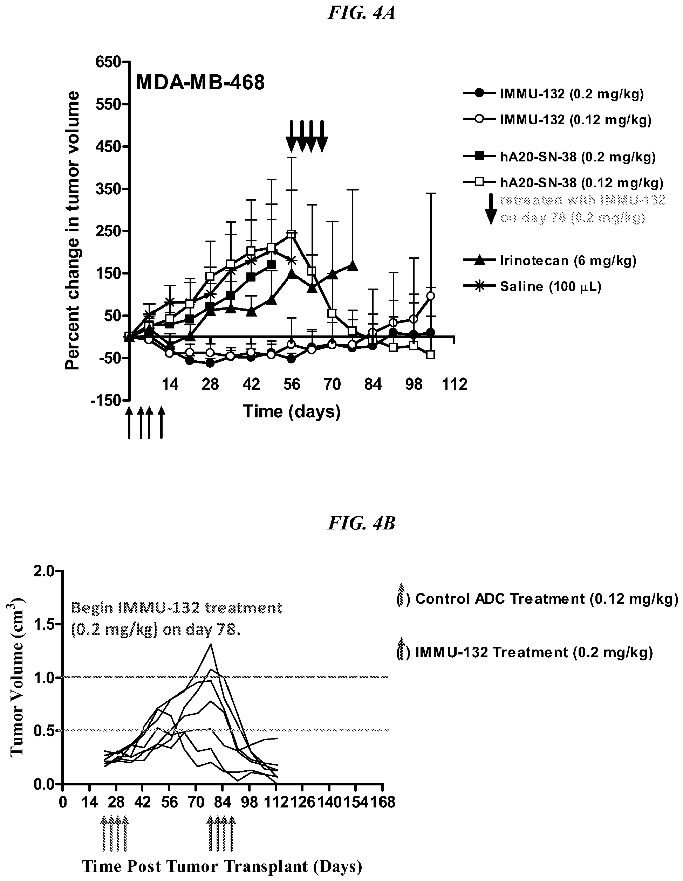

IMMU-132 was assessed in mice bearing MDA-MB-468 TNBC tumors (**FIG. 4A**; doses are given in SN-38 equivalents). IMMU-132 (0.2 mg/kg) caused significant tumor regressions when compared to saline, irinotecan (6 mg/kg), or control anti-CD20 ADC, hA20-CL2A-SN-38 (P<0.0012, AUC). Even when the dose was lowered to 0.12 mg/kg, IMMU-132 significantly reduced tumor volume in the mice (*P*<0.0017, AUC). Importantly, the total amount of SN-38 equivalents given at this low amount of IMMU-132 was only 9.6 µg, whereas the 6 mg/kg administered to the mice represented a 62.5-fold advantage (600 µg cumulative dose). However, at the time the first mouse in the irinotecan group was lost due to disease progression, tumor volumes (TV) in the mice treated with IMMU-132 (0.12 mg/kg) were significantly smaller than in those mice treated with the irinotecan (TV=0.17 ± 0.12 cm³ *vs*. 0.53 ± 0.29 cm³, respectively; *P*=0.0094, two-tailed *t*-test). As was found in the other solid tumor models, specific targeting of a small amount of SN-38 to the tumor with IMMU-132 was much more effective than a much larger dose of untargeted drug.

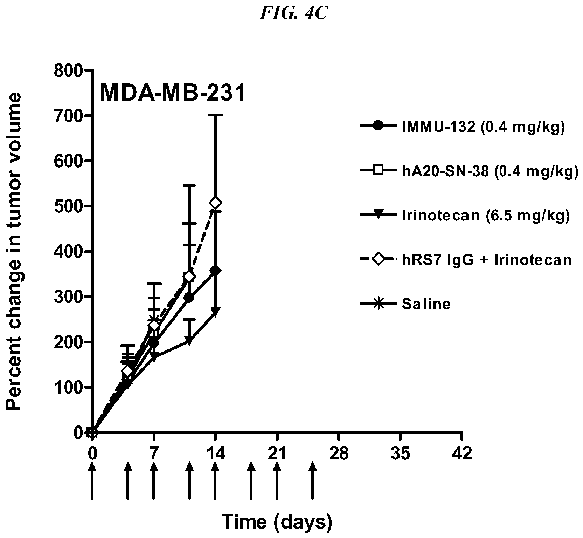

On therapy day 56 (Day 78 post-transplant), tumors in mice in the low dose hA20-CL2A-SN-38 (anti-CD20) control group (0.12 mg/kg) progressed to a point that they were no different than saline control mice (TV=0.74 ± 0.41 cm³ *vs*. 0.63 ± 0.37 cm³, respectively). At that time-point, it was decided to determine if these tumors would respond to the IMMU-132 treatment, despite their progression to a considerably larger size (**FIG. 4B**). All of the mice demonstrated a positive response with the tumors significantly smaller five weeks later than when therapy with IMMU-132 began on day 78 (TV=0.14 ± 0.14 cm³ *vs*. 0.74 ± 0.41 cm³, respectively; *P*=0.0031, two-tailed *t*-test), even tumors that were greater than 1.0 cm³ at the time IMMU-132 treatment began, regressed more than 88% (1.32 cm³ regressed to 0.06 cm³ and 1.08 cm³ regressed to 0.13 cm³). In contrast to the effects observed in MDA-MB-468, mice bearing MDA-MB-231 TNBC tumors did not respond to IMMU-132 treatment (**FIG. 4C**), which may be related to the very rapid proliferation of this tumor and/or its very low expression of TROP-2.

### Example 4. Human Clinical Trials With IMMU-132

IMMU-132 has completed a dose-finding Phase I trial in 25 patients with diverse metastatic solid cancers, who relapsed after their last therapy and had a median of 3 prior therapies. Twenty-three of the 25 were assessable for RECIST1.1 by computed tomography (CT), and the findings were as follows. (1) A dose schedule of treatment on days 1 and 8 of a 21-day cycle, giving 8-10 mg/kg per dose, was tolerable and effective. (2) Repeated cycles could be administered, even up to 10 months, with occasional dose delays and/or dose reductions, but minimal need of G-CSF hematopoietic support. (3) Despite repeated courses of therapy, no anti-drug or anti-antibody antibodies were detected by a sensitive ELISA test. (4) The major toxicities were neutropenia, diarrhea, and alopecia, consistent with those of irinotecan, the parental compound of SN-38 when given alone, but more manageable with IMMU-132. (5) IMMU-132 showed antitumor activity in human subjects *in vivo* by achieving stable disease in 13 patients, partial response in 3 (TNBC, small-cell lung cancer, and colorectal cancer), and progressive disease in 7, as best response. The longest time-to-progression was almost 57 weeks, in a patient with metastatic hormone-refractive prostate cancer in the Phase I study.

These results are being confirmed in a Phase II trial, which has already enrolled over 100 patients with diverse metastatic solid cancers at either the 8 or 10 mg/kg doses given on days 1 and 8 of a 21-day cycle, repeated as frequently as the patient's condition and tolerance permits. The side effects were no different than experience in the Phase I experience, but efficacy has been quite encouraging, especially in metastatic TNBC patients.

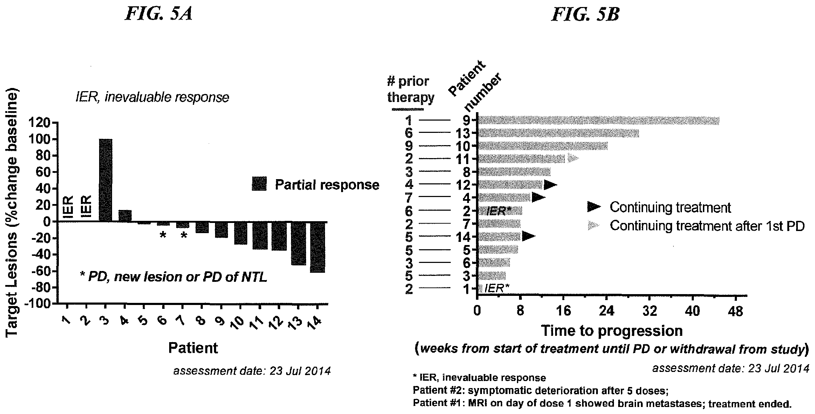

At present, among 14 CT-evaluable TNBC patients, 4 have experienced a partial response, 6 have stabilization of disease (one showing a 27% shrinkage by RECIST1.1), and 4 having progressive disease as best response (**FIG. 5A-B**). Enrollment of TNBC in this trial continues, with the expectation that 20 patients will be assessed for safety and response. These TNBC patients had 1-9 prior therapies, relapsing to the last one prior to being enrolled. Noteworthy, patients 12-14, showing PRs, had 2-6 prior therapies prior to being enrolled.

The first patient responding in the Phase I portion showed an impressive response between skin involvement before and after therapy (not shown). Other patients with partial responses in the Phase II portion include small-cell lung cancer, non-small-cell lung cancer, esophageal cancer, and urinary bladder cancer.

These results confirm that IMMU-132 has a better therapeutic index than irinotecan, since it is active at less toxic doses, indicating that the higher doses of SN-38 selectively targeted by the anti-TROP-2 antibody provides sufficient selective DNA-damage by this topoisomerase I inhibitor to achieve responses in more cancer types than are known to respond to this class of agents. Surprising is the high response rate observed in patients with metastatic TNBC after they had failed repeated prior therapies, since it is well-known that responses diminish with each successive therapy. Evaluation by immunohistochemistry has shown that almost all of the archived tumor specimens in this trial expressed the target antigen, TROP-2. In a separate study of tumor microarrays of TNBC and non-TNBC, we have confirmed that over 90% express TROP-2, of which over 65% have intense 2+ and 3+ staining. Hence, we conclude that TNBC, and probably also non-TNBC, are sensitive neoplasms to this SN-38-conjugated antibody targeting TROP-2 in these cancers, thus representing the first tumor-targeted therapy for TNBC.

### Dose-limiting toxicities and potential concerns when combined with other agents

The tolerable neutropenia (no evidence of neuropathies or other side effects that are prominent for adriamycins, taxanes, and platinum agents) suggests that IMMU-132 can be combined with one or more of these agents, especially in treatment-naive patients in a neoadjuvant setting. Given the extended use of paclitaxel followed or preceded by doxorubicin + cyclophosphamide, we reasoned that IMMU-132 should be given in a combination with paclitaxel for twelve weeks, where the typical cycle of IMMU-132 is 8 mg/kg given on days 1 and 8 every 21 days. After completing the paclitaxel ± IMMU-132 12-week treatment regimen, doxorubicin and cyclophosphamide are administered for 4 courses, every other week, as per standard protocols being used (examples below). The control arm just has therapy with paclitaxel, doxorubicin + cyclophosphamide, as per the investigational arm where IMMU-132 is an add-on. In order to limit neutropenia and avoid febrile neutropenia, dose reduction of IMMU-132 in its regimen is allowed according to the grade and duration of neutropenia experienced by the combination. As in current experience, paclitaxel may also need reduction, and the option of hematopoietic support with G-CSF (e.g., Neupogen) is left to the discretion of the managing physician for the first therapy course. However, we require prophylactic G-CSF therapy after beginning of the second arm of doxorubicin + cyclophosphamide (based on recommendation of co-investigators).

Based on our experience with IMMU-132 in over 125 metastatic cancer patients treated to-date with multiple courses of therapy, and where the patients had many prior cytotoxic therapies, the following IMMU-132 dose and schedule are used. Although patients with several prior therapies have tolerated multiple doses of IMMU-132 at 8, 10, and even 12 mg/kg, we are using 8 mg/kg for this combination since this dose level rarely results in > grade 2 neutropenia, even after multiple doses, and is therapeutically active at this level. While we expect neutropenic effects to occur with paclitaxel, the 8.0 mg/kg dose level of IMMU-132 mitigates more severe outcomes in this combination.

### Example 5. Pharmacokinetics and Immunogenicity of Patients with Diverse Advanced Cancers Receiving IMMU-132.

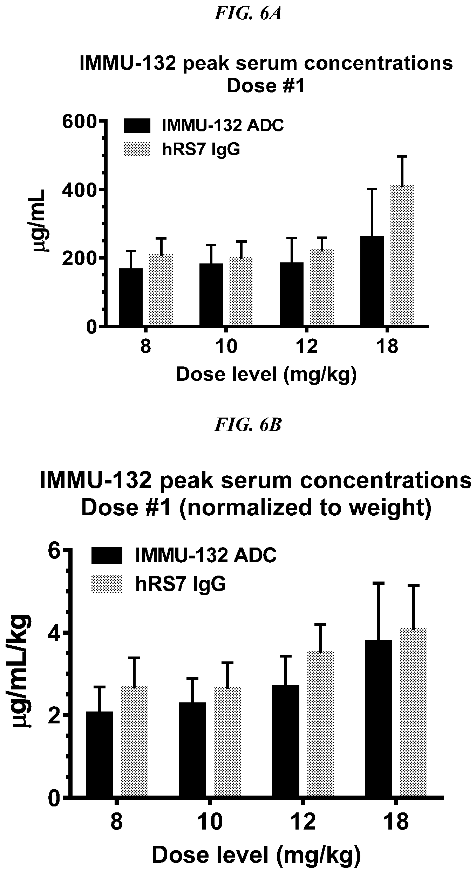

In the Phase I trial discussed above, serum samples were collected within 30 min of the end of the infusion (most infusions lasted 2 to 3.5 h), representing "peak" levels", and then right before the next dose, representing "trough" levels, for each treatment. Two ELISA assays were used to measure peak and trough serum samples. One assay uses plates coated with an anti-SN-38 antibody (developed by Immunomedics) to capture the product by binding SN-38 attached to the intact conjugate (ADC). The bound product is then identified as the antibody of interest using a specific anti-idiotype antibody (i.e., anti-hRS7 IgG). Thus, this assay measures the intact conjugate. The other assay is configured to detect hRS7 IgG in the serum. **FIG. 6A** shows the peak levels of the IgG and ADC in the 4 dose levels. When the peak levels are normalized to the patient's weight (i.e., µg/mL/kg), a trend for increasing concentrations as the dose increased is seen (**FIG. 6B**).

Analysis of the concentration of the 2 products in the serum of a representative patient over multiple doses showed peak levels remained at similar levels, adjusting lower when the dose was reduced (not shown). No ADC was present in any of the trough samples, but low concentrations of the IgG could be detected within 7-14 days of the previous dose (not shown). This finding is consistent with *in vitro* stability data indicating 50% of the SN-38 is released from the IgG in ∼1 day (Cardillo et al., Clin Cancer Res 2011; 17:3157-3169).

Select peak and trough serum samples from patients enrolled in the Phase I portion of the trial were subjected to SN-38 determinations by extracting serum, with analysis by reversed-phase HPLC. The analysis was performed in 2 parts, one that detected unbound SN-38 in the extracted serum (Free), while the other process included an acid-hydrolysis step prior to extraction that released SN-38 bound to the IgG (Total), which would then be measured as part of the total SN-38 (i.e., bound + Free SN-38). In 5 patients with determinations of unbound SN-38, its level was ≤3% of the total amount of SN-38 found in the serum (**Table 3**). Thus, ≥97% of the SN-38 in the serum was bound to the IgG within 30 minutes of the end of the infusion.

**Table 3. Serum concentrations (ng/mL) of SN-38, unbound (free) and total (after acid hydrolysis). Samples were taken 0.5 h after the first dose in select patients.**

| | **10 mg/kg** | |
|---|---|---|
| **Patient** # | **Total** | **Free** |
| 10 | 7355 | 179 |

| | **12 mg/kg** | |
|---|---|---|
| 15 | 3230 | 97 |
| 17 | 3081 | 65 |
| 18 | 6675 | 118 |
| 22 | 6138 | 183 |

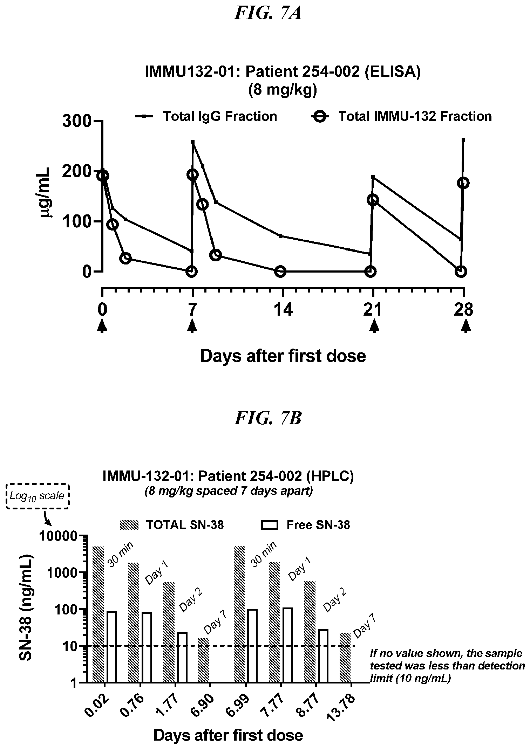

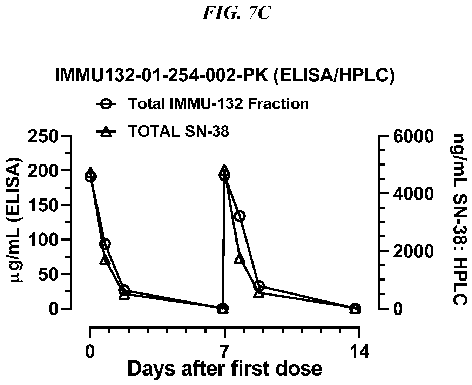

In select patients enrolled in the expansion portion of the trial, additional PK samples were collected at 1, 2, and 3 days after the first and second dose to examine the clearance rate of the IgG, conjugate, and SN-38 more carefully. **FIG. 7** shows a representative patient who received 8 mg/kg, with additional samples collected 1 and 2 days after each of the first 2 treatments. **FIG. 7A** plots the clearance of the IgG and IMMU-132 for the first 2 doses, and the peak/trough samples that were collected for the next cycle (days 21 and 28). These data confirm the intact conjugate clears more quickly than the IgG, but again, this occurs because the SN-38 is being released. By day 7, all of the SN-38 would have been released and therefore no IMMU-132 was detected, while there was still a small amount of the IgG present. **FIG. 7B** provides the corresponding concentrations of SN-38, TOTAL and FREE. Again, the amount of FREE SN-38 was only 1.8 to 6.1% of the total SN-38 in the samples. By day 7, only a trace amount of SN-38 remains in the serum. **FIG. 7C** plots the clearance of IMMU-132 based on the ELISA method of detection or on the SN-38 concentration in the serum. Both sets of data overlap, providing evidence that the ELISA is a reasonable surrogate determination for SN-38 clearance, which reflects the fact that most of the SN-38 in the serum is bound to the IgG.

IMMU-132 lacks immunogenicity, even after repeated injections over many months. ELISA assays to detect antibody responses to the hRS7 IgG or SN-38 are performed prior to the start of the study and throughout the treatments every 4 to 6 weeks. Only one patient tested to date had a positive baseline antibody to hRS7 IgG (i.e., >50 ng/mL). However, there have been no anti-hRS7 IgG nor anti-SN-38 antibody responses detected over the course of treatment in any patient to date, and this is in patients who have received as many as 30 injections.

### Example 6. Clinical Dosing Schemes

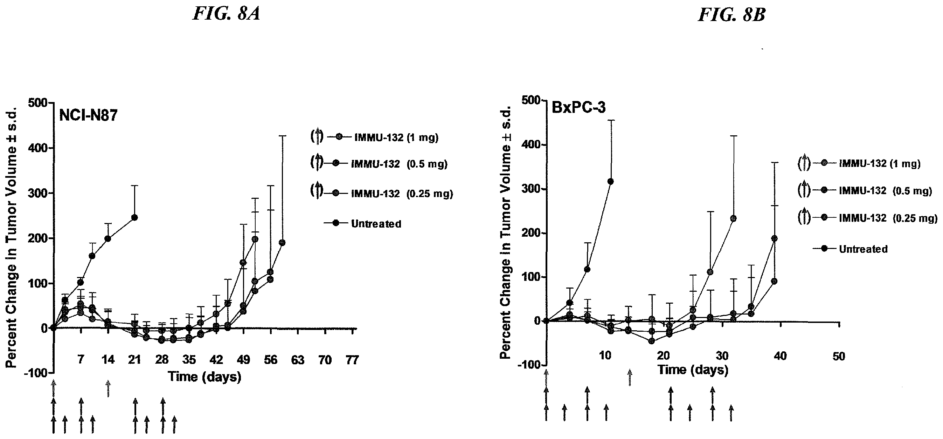

Clinically, IMMU-132 is being administered at 8 mg/kg (i.e., ∼0.16 µg/kg SN-38 equivalents). A human dose of 8 mg/kg translates to a mouse dose of 98.4 mg/kg or approximately 2 mg to a 20-g mouse. This dose was fractionated in one of three different dosing schemes, with one group of animals receiving two IMMU-132 doses of 1 mg (days 1 and 15), representing an every-other-week dosing regimen, one group was given four doses of 500 µg (days 1, 8, 22, and 29), representing a once weekly for 2 weeks in a 21-day treatment cycle, and a final group given eight doses of 250 µg (days 1, 4, 8, 11, 22, 25, 29, and 32), representing a twice weekly for 2 weeks in a 21-day treatment cycle. These dosing schemes were tested in human gastric carcinoma and pancreatic adenocarcinoma xenograft models (**FIG. 8**).

In animals bearing NCI-N87 human gastric carcinoma xenografts (**FIG. 8A**), all three (2x1 mg, 4 x 0.5 mg, and 8 x 0.25 mg) dosages provided a significant anti-tumor effect when compared to untreated control animals (P<0.0001; AUC). While there were no significant differences in survival, both the 4 x 0.5 mg and 8 x 0.25 mg groups resulted in 88% and 100% positive response rates *versus* only 22% for the 2 x 1 mg group. Additionally, on day 49 (the day the first mouse in the 2 x 1mg group reached >1.0 cm³ tumor volume), tumors were significantly smaller in the 4 x 0.5 mg group (mean TV=0.637 ± 0.274 cm³ *vs*. 0.341 ± 0.255 cm³, respectively; P=0.0259). In all, 3 of 8 mice were still alive and still demonstrating a positive response in the 4 x 0.5 mg group when the study ended on day 98 *versus* 0 of 9 in the 2 x 1 mg group.

Likewise, in the BxPC-3 human pancreatic adenocarcinoma xenograft model (**FIG. 8B**), all three (2x1 mg, 4 x 0.5 mg, and 8 x 0.25 mg) dosages significantly inhibited tumor growth when compared to untreated control animals (*P*<0.0009; AUC). Both the 4 x 0.5 mg and 8 x 0.25 mg groups resulted in significantly smaller tumors on therapy day 32 when compared to the 2 x 1 mg treatment group (day when first mice euthanized for disease progression; *P*<0.0093). Overall, mice treated with 4 x 0.5 mg IMMU-132 demonstrated a significant anti-tumor effect when compared to the 2 x 1mg treatment group (*P*=0.0357). In terms of survival there was no difference between the 2 x 1 mg group and the 4 x 0.5 mg group (median survival = 35 and 46 days, respectively). However, mice treated with 8 x 0.25 mg did demonstrate a superior survival benefit when compared to the other two treatment groups (MST=53 days; *P*<0.0349; log-rank). All three had greater than 70% positive response rate and there was no significant difference in time to tumor progression. Overall, these data suggest that fractionating the dose provides better growth control than giving a large bolus dose every other week. This is supportive of the weekly dosing being implemented clinically.

### Example 7. Tolerability

Both mice and cynomolgus monkeys were utilized to assess toxicokinetics of IMMU-132 (Cardillo et al., Clin Cancer Res 2011; 17:3157-3169) In Swiss-Webster mice, two doses of IMMU-132 at SN-38 equivalents of 4, 8, or 12 mg/kg (250, 500, or 750 mg/kg protein dose) were administered three days apart. No overt signs of toxicity were observed in the animals as indicated by no loss in body weight (results on file). No hematopoietic toxicities occurred and serum chemistries only revealed elevated aspartate transaminase (AST) and alanine transaminase (ALT) levels seven days after the second injection. However, these enzymes returned to normal and there was no evidence of hepatic lesions upon histopathologic examination.

Since IMMU-132 does not cross-react with the TROP-2 expressed by mice, a second study was performed in a cynomolgus monkey model, whose TROP-2 is recognized by IMMU-132. In this experiment, one group of six monkeys (3 male and 3 female) received 0.96 mg/kg SN-38 equivalents (60 mg/kg protein dose) IMMU-132 while a second group of six received 1.92 mg/kg (120 mg/kg protein dose). Doses were administered three days apart. All monkeys tolerated the two 0.96 mg/kg IMMU-132 doses with only transient decreases in blood counts, all of which remained in the normal range. Weight loss was less the 8%, which returned to baseline within 15 days post-injection. Histopathology showed only minimal to moderate microscopic changes to hematopoietic and gastrointestinal organs as well as female reproductive organs, eight days after the second injection. All these were returning to normal by day 32, the end of the recovery period. In contrast, the two 1.92 mg/kg doses (120 mg/kg protein dose) proved to be toxic to the monkeys. One animal died as a result of gastrointestinal complications and severe bone marrow suppression. The remaining animals in this group likewise demonstrated similar organ toxicities as those observed in the monkeys that received the 0.96 mg/kg doses but to a much more severe level. These end-organ toxicities were consistent with irinotecan, but most importantly there was no evidence of TROP-2-targeted toxicity in the many different normal tissues expressing TROP-2. This study suggested the maximum tolerated dose for two injections of IMMU-132 was between 0.96 and 1.92 mg/kg, which the human equivalent dose would be between 20 and 40 mg/kg (protein doses).

### Example 8. Mechanism of Action

Generally, cancer cells can undergo apoptosis *via* one of two main pathways referred to as either the extrinsic or intrinsic apoptotic pathways (Fulda & Debatin, Oncogene 2006; 25(34):4798-4811). The extrinsic pathway is characterized by engagement of cell surface death receptors (e.g., TNF family receptors) leading to activation of caspase-8, which leads to activation of down-stream caspases such as caspase-3, and ultimately to cleavage of poly-ADP-ribose polymerase (PARP), DNA fragmentation and cell death. Conversely, the intrinsic pathway can be triggered either by direct DNA damage (e.g. ionizing radiation) or by other cellular stresses (e.g., cell cycle arrest) that lead to cytochrome c release from the mitochondria into the cytoplasm. Cytochrome c then forms a complex with apoptotic protease activating factor-1 (APAF-1), which acts as a platform to activate caspase-9, that then begins the caspase activation cascade including caspase-3 and -7, PARP cleavage, and cell death.

SN-38 is a known topoisomerase-I inhibitor that induces significant damage to a cell's DNA. It mediates the up-regulation of early pro-apoptotic proteins, p53 and p21^{WAF1/Cip1}, resulting in caspase activation and PARP cleavage (Cusack et al. Cancer Res 2001; 61:3535-3540; Liu et al. Cancer Lett 2009; 274:47-53; Lagadec et al. Br J Cancer 2008; 98:335-344; Whitacre et al. Clin Cancer Res 1999; 5:665-672). Expression of p21^{WAF1/Cip1}is associated with G₁ arrest of the cell cycle and is thus a hallmark of the intrinsic pathway (Sherr & Roberts, Genes Dev 1995; 9:1149-1163). We previously demonstrated that IMMU-132 likewise could mediate the up-regulation of early pro-apoptosis signaling events (p53 and p21^{WAF1/Cip1}), resulting in PARP cleavage in NSCLC (Calu-3) and pancreatic (BxPC-3) cell lines (Cardillo et al., Clin Cancer Res 2011; 17:3157-3169).

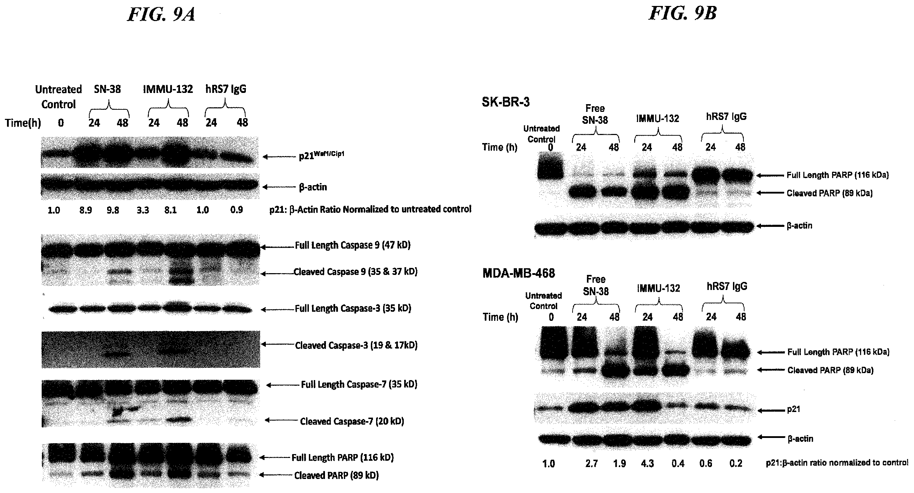

In order to better define the apoptotic pathway utilized by IMMU-132, the NCI-N87 human gastric carcinoma cell line was exposed to 1 µM of free SN-38 or the equivalent amount of IMMU-132 (**FIG. 9A**). Both free SN-38 and IMMU-132 mediate the up-regulation of p21^{WAF1/Cip1}, though it is not until 48 h that the amount of up-regulation is the same between cells exposed to free SN-38 *versus* IMMU-132. This may be due to the delay in uptake and release of SN-38 from the ADC compared to the ready availability when just the free drug is added to the cultured cells. Given the rapid clearance of irinotecan relative to IMMU-132 *in vivo,* one would expect the advantage of SN-38 accumulation in the tumors to be with IMMU-132, as is demonstrated below in the pharmaco-toxicological studies.

In terms of caspase activation, both the free SN-38 and IMMU-132 demonstrate cleavage of caspase-9 and -7 within 48 h of exposure. This result is consistent with a previous report in a HT-29 colonic cell line in which 10 nM caused cleavage of caspase 9 after a 72 h exposure (Lagadec et al. Br J Cancer 2008; 98:335-344). However, in that same report, they did not see caspase-3 cleavage, whereas we do show cleavage. It could be that this is an early event that is not detected at 72 h or the amount of SN-38 they used (10 nM) was not high enough to show this compared to the 1 µM we used in our assays. Finally, both free SN-38 and IMMU-132 mediated PARP cleavage. This first becomes evident at 24 h with increased cleavage at 48 h. Taken together, these data confirm that the SN-38 contained in IMMU-132 has the same activity as free SN-38 and that the intrinsic apoptotic pathway is being induced by this ADC.

The microtubule-inhibitor, paclitaxel, mediates the induction of p21^{WAF1/Cip1} in human breast carcinomas (Blagosklonny et al. Cancer Res 1995; 55: 4623-4626). This induction can be independent of p53 status (Li et al. Cancer Res 1996; 56: 5055-5062). In addition, paclitaxel has been shown to activate caspase-2 in human breast cancer cells which in turn begins the caspase-activation cascade and PARP cleavage (Jelinek et al. Cancer Cell Int 2013; 13: 42). As shown (**FIG. 9B**), IMMU-132 likewise was shown to mediate PARP cleavage in two different human breast cell lines, including the TNBC MDA-MB-468. Near complete cleavage was evident within 48 h of exposure to 1 µM free SN-38 or the equivalent amount of IMMU-132. Additionally, p21^{WAF1/Cip1} was up-regulated in MDA-MB-468, which contains mutant *p53.* Ongoing experiments will expand on determining which caspases are activated in breast cancer cells by IMMU-132 and what, if any, overlap may exist with paclitaxel.

A best-case scenario when combining two chemotherapeutics would be the achievement of synergy. To do this the two agents should either work by two independent pathways leading to the same end or to work in concert to amplify a single pathway. An example of this in TNBC was shown when methylseleninic acid was combined with paclitaxel (Qi et al. PLoS ONE 2012; 7: e31539). Both agents were capable of activating caspase-3 and induce PARP cleavage alone, but when combined, they increased the degree of this activation with the result of synergistically inhibiting cell growth. Like methylseleninic acid, IMMU-132 makes uses many of these same apoptotic pathways and therefore might also work in synergy with paclitaxel to amplify these signals.

Combinations of paclitaxel with 5-FU, gemcitabine, and carboplatin have been shown to be antagonistic if not administered in the correct sequence (Qi et al. PLoS ONE 2012; 7: e31539; Johnson et al. Clin Cancer Res 1997; 3: 1739-1745; Johnson et al. Clin Cancer Res 1999; 5: 2559-2565; Sui et al. Cancer Biol Ther 2006; 5: 1015-1021; Xiong et al. Cancer Biol Ther 2007; 6: 1067-1073). In the case of gemcitabine and carboplatin, the administration of paclitaxel prior to these other agents was necessary to prevent antagonism (Sui et al. Cancer Biol Ther 2006; 5: 1015-1021; Xiong et al. Cancer Biol Ther 2007; 6: 1067-1073). However, for 5-FU, the sequencing did not change this antagonistic outcome (Johnson et al. Clin Cancer Res 1999; 5: 2559-2565). A key finding was that 5-FU blocked paclitaxel induction of p21^{WAF1/Cip1}. Interestingly, when SN-38 was combined with 5-FU, antagonism was observed if 5-FU was added before SN-38 or if they were added simultaneously. Only when cells were exposed to SN-38 first, could 5-FU be added to achieve synergy (Torigoe et al. Anticancer Res 2009; 29: 2083-2090). Given that IMMU-132, like paclitaxel, mediates the up-regulation of p21^{WAF1/Cip1}, uses similar apoptotic pathways, and both had similar antagonistic problems with 5-FU, it is possible the two agents would work in synergy. Ongoing experiments are examining the possible benefits of combining IMMU-132 therapy with paclitaxel. These include *in vitro* apoptosis signaling events and *in vivo* models of TNBC, in which mice are being treated with the combination of IMMU-132 and paclitaxel.

### Example 9. Pharmacotoxicology Studies of IMMU-132 vs. Irinotecan in a Pancreatic Cancer Xenograft Model

The clearance and uptake of SN-38 in tissues were examined in nude mice bearing Capan-1 pancreatic cancer xenografts (∼0.06-0.27 g) injected IV with 40 mg/kg of irinotecan (773 µg; total SN-38 equivalents = 448 µg) or IMMU-132 (1.0 mg with a DAR= 7.6, SN-38 equivalents = 20 µg). The irinotecan dose is the MTD in mice and is equivalent in humans to 3.25 mg/kg or ∼126 mg/m². The IMMU-132 dose is well below the MTD in mice, and represents a human equivalent dose of ∼4 mg/kg IMMU-132 (80 µg/kg SN-38 equivalents). Groups of animals (n = 3) were necropsied at 5 intervals; for irinotecan: 5 min, 1, 2, 6, and 24 h and for IMMU-132: 1, 6, 24, 48, 72 h. Serum taken from animals were diluted 1:2 in water and then extracted with equal parts of in methanol:ethylene glycol: 1M ZnSO4. In this media, SN-38, SN-38G (glucuronidate), and irinotecan is taken into the organic phase, while proteins are precipitated. Thus, any SN-38 bound to the IgG would be precipitated and go undetected.

In order to detect the SN-38 bound to the IgG, the serum sample first had to be acid hydrolyzed with 6M HCl and then neutralized before adding the extraction media. This process releases all IgG-bound SN-38, which would be isolated in the organic phase of the extraction media, along with any unbound (i.e., free) SN-38 that was in the sample. The acid-hydrolyzed extracted samples are said to measure the TOTAL amount of SN-38 in the sample, while samples that are only extracted measure the unbound or free SN-38 in the sample. The amount of IgG-bound SN-38 can be derived by subtracting the free from the TOTAL. Serum and tissue homogenates from IMMU-132-treated animals are therefore process in 2 ways, non-hydrolyzed/extracted to isolate unbound SN-38 and acid-hydrolyzed/extracted samples giving the TOTAL SN-38. The tumor, liver, and small intestinal contents were first homogenized in water (10 parts water to 1 part tissue). All samples prior to extraction are spiked with an internal standard, 10-hydroxyl-camptothecin (10-OH CT), which aids in quantitation and monitoring recovery. A small portion (e.g., 5 to 50 µL) of the organic phase of the extract is applied to an analytical C-18 HPLC column that was pre-calibrated with standards from each of the products of interest. The AUC for each product peak (i.e., SN-38, SN-38G, irinotecan, and 10-OH CT) is determined. A ratio of the product peak to the internal standard peak is calculated and plotted against a standard curve ranging from 10 to 10,000 ng/mL for each agent. Log-transformed data are fit using linear regression. The sensitivity of the assay for the serum samples was 20 ng/mL, since samples were diluted 2:1 for analysis, while the sensitivity for tissue samples was 110 ng/mL, since the homogenate was diluted 11:1 (1 part tissue to 10 parts water).

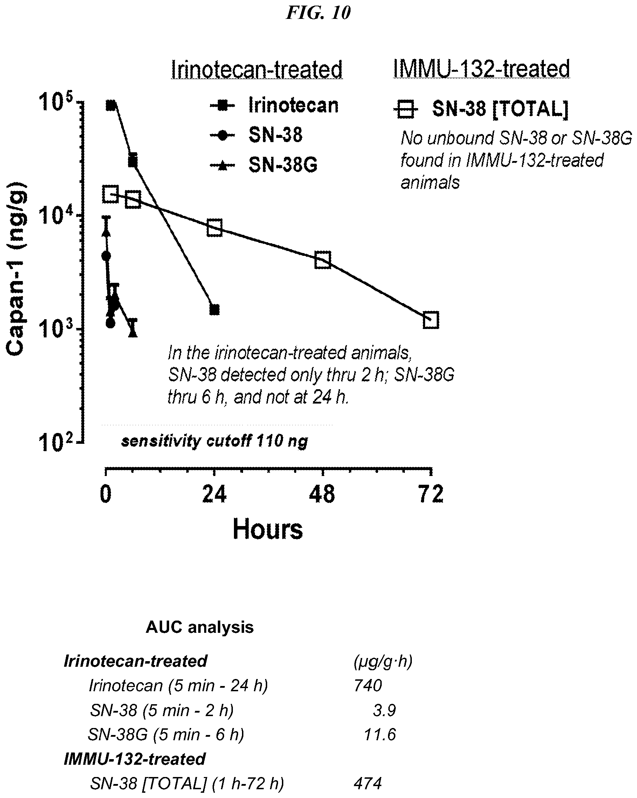

In the irinotecan-treated animals, SN-38 could only be detected in the tumors at 5 min, 1, and 2 h), yet irinotecan and SN-38G were detected in these same samples and in the 6-h sample (**FIG. 10**); none of these products was detected in the 24-h sample. Despite having high levels of irinotecan in the tumors, concentrations of SN-38 or SN-38G were very small. For example, at 2 h, with an average of 101.6 ± 58.6 µg/g of irinotecan in the tumors, there were only 1.6 ± 1.0 (1.6%) and 1.3 ± 1.2 µg/g (1.3%) of SN-38 and SN-38G in the tumors, respectively. Thus, while there was a sizeable amount of irinotecan taken into the Capan-1 tumors very quickly, the irinotecan within the tumor was very poorly converted to SN-38.

For animals receiving IMMU-132, there were no detectable levels of unbound SN-38 or SN-38G in the tumor; however, high levels of SN-38 [TOTAL] were detected. Since there was no unbound SN-38 or SN-38G detected, this means all of the SN-38 in the tumor of the IMMU-132-treated animals was bound to the IgG, illustrating that the product integrity is retained, with SN-38 being delivered to the tumor exclusively by the binding of the conjugate to the tumor. It is important to keep in mind that the mole equivalents of SN-38 injected with IMMU-132 is only a fraction of the mole equivalents of SN-38 contained in the irinotecan injected animals (e.g., 448 µg SN-38, or if based on a 25% conversion, ∼115 µg vs 20 µg for IMMU-132).

Comparing the AUC for SN-38 delivered to the tumors for irinotecan (3.9 µg/g•h) to that of the SN-38 [TOTAL] in the tumors for IMMU-132 (474 µg/g•h), one finds IMMU-132 has the ability to deliver nearly 120-fold more SN-38 to the Capan-1 tumor than irinotecan, even though as in this study, animals were given 22-fold less SN-38 equivalents with IMMU-132 than irinotecan (448 vs 20 µg).

A similar analysis was performed in animals bearing Capan-1 tumors that were given an irrelevant, non-targeting antibody conjugate. Tumor/blood concentrations of the IMMU-132 were 4-fold higher than the ratios found in the animals given the non-targeting SN-38 antibody conjugate, confirming the benefits of using a conjugate that binds specifically to the tumor.

Analysis of liver and feces: Unbound SN-38 levels in the liver of animals given IMMU-132 were very low, averaging 130 ± 58 ng/g at 1 h, with no detectable levels found at later time. Even SN-38[TOTAL] concentrations were relatively low, averaging 1023 ± 202, 909 ± 186, and 203 ± 29 ng/g at 1, 6, and 24 h, respectively, with no detectable levels found in the liver at 48 and 72 h. This is in good agreement with relatively low concentrations of unbound SN-38 in the feces isolated from the small intestine (442 ± 103 ng and 912 ± 373 ng in the entire contents at 1 and 6 h). SN-38 concentrations in the TOTAL-processed samples (Bound + Unbound) were only slightly higher than in the FREE-processed samples (Unbound), confirming that the intact conjugate does not traverse the hepatobiliary elimination pathway into the intestine.

In the irinotecan-treated animals, SN-38 concentrations were much higher, peaking in the liver at 5 min (3,511 ± 476 ng/g), decreasing to 1296 ± 505 ng/g at 1 h (at this time, it is nearly 10-fold higher than Unbound SN-38 in animals given IMMU-132), and finally measuring 638 ng/g at 6 h before becoming undetectable at 24 h. Surprisingly, SN-38 could be detected in the small intestinal contents even as early as 5 min (799 ± 550 ng/g), and maintaining a level of ∼10,000 ng/g over 6 h before becoming undetectable at 24 h. This illustrates how quickly irinotecan is converted to SN-38 and SN-38G. As mentioned earlier, blood clearance data had shown more than 98% of the product had been eliminated from the blood within just 5 min.

### Example 10. Anti-CD74-CL2A-SN-38 Conjugates for Treatment of CD74+ Human Cancers (not claimed)

CD74 is an attractive target for antibody-drug conjugates (ADC), because it internalizes and recycles after antibody binding. CD74 mostly is associated with hematological cancers, but is expressed also in solid cancers. Therefore, the utility of ADCs prepared with the humanized anti-CD74 antibody, milatuzumab, for the therapy CD74-expressing solid tumors was examined. Milatuzumab-doxorubicin and two milatuzumab-SN-38 conjugates were prepared with cleavable linkers (CL2A and CL2E), differing in their stability in serum and how they release SN-38 in the lysosome. CD74 expression was determined by flow cytometry and immunohistology. *In vitro* cytotoxicity and *in vivo* therapeutic studies were performed in the human cancer cell lines A-375 (melanoma), HuH-7 and Hep-G2 (hepatoma), Capan-1 (pancreatic), and NCI-N87 (gastric), and Raji Burkitt lymphoma. The milatuzumab-SN-38 ADC was compared to SN-38 ADCs prepared with anti-TROP-2 and anti-CEACAM6 antibodies in xenografts expressing their target antigens.

Milatuzumab-doxorubicin was most effective in the lymphoma model, while in A-375 and Capan-1, only the milatuzumab-CL2A-SN-38 showed a therapeutic benefit. Despite much lower surface expression of CD74 than TROP-2 or CEACAM6, milatuzumab-CL2A-SN-38 had similar efficacy in Capan-1 as anti-TROP-2-CL2A-SN-38, but in NCI-N87, the anti-CEACAM6 and anti-TROP-2 conjugates were superior. Studies in 2 hepatoma cell lines at a single dose level showed significant benefit over saline-treated animals, but not against an irrelevant IgG conjugate. CD74 is a suitable target for ADCs in some solid tumor xenografts, with efficacy largely influenced by uniformity of CD74 expression, and with CL2A-linked SN-38 conjugates providing the best therapeutic responses.

### Materials and Methods

Human tumor cell lines. Raji Burkitt lymphoma, A-375 (melanoma), Capan-1 (pancreatic adenocarcinoma), NCI-N87 (gastric carcinoma), Hep-G2 hepatoma and MC/CAR myeloma cell lines were purchased from American Tissue Culture Collection (Manassas, VA). HuH-7 hepatoma cell line was purchased from Japan Health Science Research Resources Bank (Osaka, Japan). All cell lines were cultured in a humidified CO₂ incubator (5%) at 37 °C in recommended media containing 10% to 20% fetal-calf serum and supplements. Cells were passaged <50 times and checked regularly for mycoplasma.

Antibodies and conjugation methods. Milatuzumab (anti-CD74 MAb), epratuzumab (anti-CD22), veltuzumab (anti-CD20), labetuzumab (anti-CEACAM5), hMN15 (anti-CEACAM6), and hRS7 (anti-TROP-2) are humanized IgG₁ monoclonal antibodies. CL2A and CL2E linkers and their SN-38 derivatives were prepared and conjugated to antibodies as described in the Examples above. The milatuzumab-doxorubicin conjugates were prepared as previously described (Griffiths et al., 2003, Clin Cancer Res 9:6567-71). All conjugates were prepared by disulfide reduction of the IgG, followed by reaction with the corresponding maleimide derivatives of these linkers. Spectrophotometric analyses estimated the drug:IgG molar substitution ratio was 5-7 (1.0 mg of the protein contains -16 µg of SN-38 or 25 µg of doxorubicin equivalent).

*In vitro* cell binding and cytotoxicity. Assays to compare cell binding of the unconjugated and conjugated milatuzumab to antigen-positive cells and cytotoxicity testing used the MTS dye reduction method (Promega, Madison, WI).

Flow cytometry and immunohistology. Flow cytometry was performed in a manner that provided an assessment of only membrane-bound or membrane and cytoplasmic antigen. Immunohistology was performed on formalin-fixed, paraffin-embedded sections of subcutaneous tumor xenografts, staining without antigen retrieval methods, using antibodies at 10 µg/mL that were revealed with an anti-human IgG conjugate.

*In vivo* studies. Female nude mice (4-8 weeks old) or female SCID mice (7 weeks old) were purchased from Taconic (Germantown, NY) and used after a 1-week quarantine. All agents, including saline controls, were administered intraperitoneally twice-weekly for 4 weeks. Specific doses are given in Results. Toxicity was assessed by weekly weight measurements. For the Raji Burkitt lymphoma model, SCID mice were injected intravenously with 2.5×10⁶ Raji cells in 0.1 mL media. Five days later, animals received a single intravenous injection (0.1 mL) of the conjugate or saline (N = 10/group). Mice were observed daily for signs of distress and paralysis, and were euthanized when either hind-limb paralysis developed, >15% loss of initial weight, or if otherwise moribund (surrogate survival endpoints).

Subcutaneous tumors were measure by caliper in two dimensions, and the tumor volume (TV) calculated as *L×w²*/*2*, where *L* is the longest diameter and *w* is the shortest. Measurements were made at least once weekly, with animals terminated when tumors grew to 1.0 cm³ (i.e., surrogate survival end-point). The A-375 melanoma cell line (6 x 10⁶ cells in 0.2 mL) was implanted in nude mice and therapy was initiated when tumors averaged 0.23 ± 0.06 cm³ (N = 8/group). Capan-1 was implanted subcutaneously in nude mice using a combination of tumor suspension from serially-passaged tumors (0.3 mL of a 15% w/v tumor suspension) combined with 8×10⁶ cells from tissue culture. Treatments were initiated when TV averaged 0.27 ± 0.05 cm³ (N = 10/group). NCI-N87 gastric tumor xenografts were initiated by injecting 0.2 mL of a 1:1 (v/v) mixture of matrigel and 1×10⁷ cells from terminal culture subcutaneously. Therapy was started when the TV averaged 0.249 ± 0.045 cm³ (N = 7/group). The same procedure was followed for developing the Hep-G2 and HuH-7 hepatoma xenografts in nude mice. Therapy was started when Hep-G2 averaged 0.364 ± 0.062 cm³ (N = 5/group) and HuH-7 averaged 0.298 ± 0.055 cm³ (N = 5/group).

Efficacy is expressed in Kaplan-Meier survival curves, using the surrogate end-points mentioned above for determining the median survival times. Analysis was performed by a log-rank (Mantel-Cox) test using Prism GraphPad software (LaJolla, CA), with significance at *P* <0.05.

### Results

CD74 expression in human tumor cell lines and xenografts. Six cell lines derived from 4 different solid tumor types were identified as CD74-positive based primarily on the analysis of permeabilized cells (**Table 4**), since the MFI of membrane-only CD74 in the solid tumor cell lines very often was <2-fold higher than the background MFI (except A-375 melanoma cell line). Surface CD74 expression in Raji was >5-fold higher than the solid tumor cell lines, but total CD74 in permeabilized Raji cells was similar to most of the solid tumor cell lines.

**Table 4. CD74 expression by flow cytometry expressed as mean fluorescent intensity (MFI) of milatuzumab-positive gated cells.**

| **Cell line** | | **Surface** | | **Surface and cytoplasmic** | |
|---|---|---|---|---|---|
| | | **hLL1 (bkgd)^{a}** | **MFI Ratio hLL1:bkgd** | **hLL1 (bkgd)^{b}** | **MFI Ratio hLL1:bkgd** |
| Panc CA^{c} | Capan-1 | 22 (12) | 1.8 | 248 (5) | 49.6 |
| Gastric | Hs746T | 17 (8) | 2.1 | 144 (5) | 28.8 |
| | NCI-N87 | 5 (4) | 1.3 | 220 (6) | 36.7 |
| Melanoma | A-375 | 16 (3) | 5.3 | 185 (6) | 30.8 |
| Hepatoma | Hep-G2 | 9 (6) | 1.5 | 156 (5) | 31.2 |
| | HuH-7 | 8 (5) | 1.6 | 114 (4) | 28.5 |
| Lymphoma | Raji | 59 (3) | 19.6 | 143 (5) | 28.6 |

| | | | | | |
|---|---|---|---|---|---|
| ND, not done ^{a}Background MFI of cells incubated with GAH-FITC only. | | | | | |

Immunohistology showed Raji subcutaneous xenografts had a largely uniform and intense staining, with prominent cell surface labeling (not shown). The Hep-G2 hepatoma cell line had the most uniform uptake of the solid tumors, with moderately strong, but predominantly cytoplasmic, staining (not shown), followed by the A-375 melanoma cell line that had somewhat less uniform staining with more intense, yet mostly cytoplasmic, expression (not shown). The Capan-1 pancreatic (not shown) and NCI-N87 (not shown) gastric carcinoma cell lines had moderate (Capan-1) to intense (NCI-N87) CD74 staining, but it was not uniformly distributed. The HuH-7 hepatoma cell line (not shown) had the least uniform and the weakest staining.

Immunoreactivity of the conjugates. *K*_{d} values for unconjugated milatuzumab, milatuzumab-CL2A-SN-38 and milatuzumab-CL2E-SN-38 conjugates were not significantly different, averaging 0.77 nM, 0.59 nM, and 0.80 nM, respectively. *K*_{d} values for the unconjugated and doxorubicin-conjugated milatuzumab measured in the MC/CAR multiple myeloma cell line were 0.5 ± 0.02 nM and 0.8 ± 0.2 nM, respectively (Sapra et al., 2008, Clin Cancer Res 14:1888-96).

*In vitro* drug release and serum stabilities of conjugates. The release mechanisms of SN-38 from the mercaptoethanol-capped CL2A and CL2E linkers were determined in an environment partially simulating lysosomal conditions, namely, low pH (pH 5.0), and in the presence or absence of cathepsin B. The CL2E-SN-38 substrate was inert at pH 5 in the absence of the enzyme (not shown), but in the presence of cathepsin B, cleavage at the Phe-Lys site proceeded quickly, with a half-life of 34 min (not shown). The formation of active SN-38 requires intramolecular cyclization of the carbamate bond at the 10^{th} position of SN-38, which occurred more slowly, with a half-life of 10.7 h (not shown).

As expected, cathepsin B had no effect on the release of active SN-38 in the CL2A linker. However, CL2A has a cleavable benzyl carbonate bond, releasing active SN-38 at a rate similar to the CL2E linker at pH 5.0, with a half-life of ∼ 10.2 h (not shown). The milatuzumab-doxorubicin conjugate, which has a pH-sensitive acylhydrazone bond, had a half-life of 7 to 8 h at pH 5.0 (not shown).

While all of these linkers release the drug at relatively similar rates under lysosomally-relevant conditions, they have very different stabilities in serum. Milatuzumab-CL2A-SN-38 released 50% of free SN-38 in 21.55 ± 0.17 h (not shown), consistent with other CL2A-SN-38 conjugates. The CL2E-SN-38 conjugate, however, was highly inert, with a half-life extrapolated to ∼2100 h. The milatuzumab-doxorubicin conjugate released 50% of the doxorubicin in 98 h, which was similar to 2 other antibody-doxorubicin conjugates (not shown).

Cytotoxicity. A significant issue related to the evaluation of these conjugates was the relative potency of free doxorubicin and SN-38 in hematopoietic and solid tumor cell lines. Our group previously reported that SN-38 was active in several B-cell lymphoma and acute leukemia cell lines, with potencies ranging from 0.13 to 2.28 nM (Sharkey et al., 2011, Mol Cancer Ther 11:224-34). SN-38 potency in 4 of the solid tumor cell lines that were later used for *in vivo* therapy studies ranged from 2.0 to 6 nM (not shown). Doxorubicin had a mixed response, with 3-4 nM potency in the Raji lymphoma and the A-375 melanoma cell lines, but it was nearly 10 times less potent against Capan-1, NCI-N87, and Hep G2 cell lines. Other studies comparing the potency of SN-38 to doxorubicin found: LS174T colon cancer, 18 vs. 18 (nM potency of SN-38 vs. doxorubicin, respectively); MDA-MB-231 breast cancer, 2 vs. 2 nM; SK-OV-4 ovarian cancer, 18 vs. 90 nM; Calu-3 lung adenocarcinoma, 32 vs. 582 nM; Capan-2 pancreatic cancer, 37 vs. 221 nM; and NCI-H466 small cell lung cancer, 0.1 vs. 2 nM. Thus, SN-38 was 5- to 20-fold more potent than doxorubicin in 4 of these 6 cell lines, with similar potency in LS174T and MDA-MB-231. Collectively, these data indicate that doxorubicin is less effective against solid tumors than SN-38, while SN-38 appears to be equally effective in solid and hematopoietic tumors.

As expected, the 3 conjugate forms were often some order of magnitude less potent than the free drug *in vitro,* since both drugs are expected to be transported readily into the cells, while drug conjugates require antibody binding to transport drug inside the cell (not shown). The CL2A-linked SN-38 conjugate is an exception, since more than 90% of the SN-38 is released from the conjugate into the media over the 4-day assay period (Cardillo et al., 2011, Clin Cancer Res 17:3157-69; Sharkey et al., 2011, Mol Cancer Ther 11:224-34). Thus, even if the conjugate was internalized rapidly, it would be difficult to discern differences between the free drug and the CL2A-linked drug.

The stable CL2E-linked SN-38 performed comparatively well in the Raji cell line, compared to free SN-38, but it had substantially (7- to 16-fold) lower potency in the 4 solid tumor cell lines, suggesting the relatively low surface expression of CD74 may be playing a role in minimizing drug transport in these solid tumors. The milatuzumab-doxorubicin conjugate had substantial differences in its potency when compared to the free doxorubicin in all cell lines, which was of similar magnitude as the CL2E-SN-38 conjugates to free SN-38 in the solid tumor cell lines.

In the 6 additional cell lines mentioned above, the milatuzumab-CL2A-SN-38 conjugate was 9- to 60-times more potent than the milatuzumab-doxorubicin conjugate (not shown), but again, this result was influenced largely by the fact that the CL2A-linked conjugate releases most of its SN-38 into the media over the 4-day incubation period, whereas the doxorubicin conjugate would at most release 50% of its drug over this same time. The CL2E-linked milatuzumab was not examined in these other cell lines.

*In vivo* therapy of human tumor xenografts. Previous *in vivo* studies with the milatuzumab-doxorubicin or SN-38 conjugates prepared with various antibodies had indicated they were efficacious at doses far lower than their maximum tolerated dose (Griffiths et al., 2003, Clin Cancer Res 9:6567-71; Sapra et al., 2005, Clin Cancer Res 11:5257-64; Govindan et al., 2009, Clin Cancer Res 15:6052-61; Cardillo et al., 2011, Clin Cancer Res 17:3157-69; Sharkey et al., 2011, Mol Cancer Ther 11:224-34), and thus *in vivo* testing focused on comparing similar, but fixed, amounts of each conjugate at levels that were well-tolerated.

Initial studies first examined the doxorubicin and SN-38 conjugates in a disseminated Raji model of lymphoma in order to gauge how the milatuzumab-doxorubicin conjugate compared to the 2 SN-38 conjugates (not shown). All specific conjugates were significantly better than non-targeting labetuzumab-SN-38 conjugate or saline-treated animals, which had a median survival of only 20 days (*P* <0.0001). Despite *in vitro* studies indicating as much as an 8-fold advantage for the SN-38 conjugates in Raji, the best survival was seen with the milatuzumab-doxorubicin conjugates, where all animals given a single 17.5 mg/kg (350 µg) dose and 7/10 animals given 2.0 mg/kg (40 µg) were alive at the conclusion of the study (day 112) (e.g., 17.5 mg/kg dose milatuzumab-doxorubicin vs. milatuzumab-CL2A-SN-38, *P* = 0.0012). Survival was significantly lower for the more stable CL2E-SN-38 conjugates (*P*< 0.0001 and *P* = 0.0197, 17.5 and 2.0 mg/kg doses for the CL2A vs. CL2E, respectively), even though *in vitro* studies suggested that both conjugates would release active SN-38 at similar rates when internalized.

Five solid tumor cell lines were examined, starting with the A-375 melanoma cell line, since it had the best *in vitro* response to both doxorubicin and SN-38. A-375 xenografts grew rapidly, with saline-treated control animals having a median survival of only 10.5 days (not shown). A 12.5 mg/kg (0.25 mg per animal) twice-weekly dose of the milatuzumab-CL2A-SN-38 conjugate extended survival to 28 days (*P* = 0.0006), which was significantly better than the control epratuzumab-CL2A-SN-38 conjugate having a median survival of 17.5 days (*P* = 0.0089), with the latter not being significantly different from the saline-treated animals (*P* = 0.1967). The milatuzumab-CL2A conjugate provided significantly longer survival than the milatuzumab-CL2E-SN-38 conjugate (*P* = 0.0014), which had the same median survival of 14 days as its control epratuzumab-CL2E-SN-38 conjugate. Despite giving a 2-fold higher dose of the milatuzumab-doxorubicin than the SN-38 conjugates, the median survival was no better than the saline-treated animals (10.5 days).

As with the A-375 melanoma model, in Capan-1, only the CL2A-linked SN-38 conjugate was effective, with a median survival of 35 days, significantly different from untreated animals (*P* <0.036) (not shown), even at a lower dose (5 mg/kg;100 µg per animal) (*P*<0.02). Neither the milatuzumab-CL2E nor the non-targeting epratuzumab-CL2A-SN-38 conjugates, or a 2-fold higher dose of the milatuzumab-doxorubicin conjugate, provided any survival advantage (*P* = 0.44 vs. saline). It is noteworthy that in the same study with animals given the same dose of the internalizing anti-TROP-2 CL2A-SN-38 conjugate (hRS7-SN-38; IMMU-132), the median survival was equal to milatuzumab-CL2A-SN-38 (not shown). The hRS7-CL2A-SN-38 conjugate had been identified previously as an ADC of interest for treating a variety of solid tumors (Cardillo et al., 2011, Clin Cancer Res 17:3157-69). The MFI for surface-binding hRS7 on Capan-1 was 237 (not shown), compared to 22 for milatuzumab (see **Table 4**). Thus, despite having a substantially lower surface antigen expression, the milatuzumab-CL2A-SN-38 conjugate performed as well as the hRS7-CL2A-SN-38 conjugate in this model.

With the milatuzumab-doxorubicin conjugate having inferior therapeutic results in 2 of the solid tumor xenografts, the focus shifted to compare the milatuzumab-SN-38 conjugates to SN-38 conjugates prepared with other humanized antibodies against TROP-2 (hRS7) or CEACAM6 (hMN-15), which are more highly expressed on the surface of many solid tumors (Blumenthal et al., 2007, BMC Cancer 7:2; Stein et al., 1993, Int J Cancer 55:938-46). Three additional xenograft models were examined.

In the gastric tumor model, NCI-N87, animals given 17.5 mg/kg/dose (350 µg) of milatuzumab-CL2A-SN-38 provided some improvement in survival, but it failed to meet statistical significance compared to the saline-treated animals (31 vs. 14 days; *P* = 0.0760) or to the non-binding veltuzumab anti-CD20-CL2A-SN39 conjugate (21 days; *P* = 0.3128) (not shown). However, the hRS7- and hMN-15-CL2A conjugates significantly improved the median survival to 66 and 63 days, respectively (*P* = 0.0001). The MFI for surface-expressed TROP-2 and CEACAM6 were 795 and 1123, respectively, much higher than CD74 that was just 5 (see **Table 4**). Immunohistology showed a relatively intense cytoplasmic expression of CD74 in the xenograft of this cell line, but importantly it was scattered, appearing only in defined pockets within the tumor (not shown). CEACAM6 and TROP-2 were more uniformly expressed than CD74 (not shown), with CEACAM6 being more intensely present both cytoplasmically and on the membrane, and TROP-2 primarily found on the membrane. Thus, the improved survival with the anti-CEACAM6 and anti-TROP-2 conjugates most likely reflects both higher antigen density and more uniform expression in NCI-N87.

In the Hep-G2 hepatoma cell line (not shown), immunohistology showed a very uniform expression with moderate cytoplasmic staining of CD74, and flow cytometry indicated a relatively low surface expression (MFI = 9). The MFI with hMN-15 was 175 and immunohistology showed a fairly uniform membrane and cytoplasmic expression of CEACAM6, with isolated pockets of very intense membrane staining (not shown). A study in animals bearing Hep-G2 xenografts found the milatuzumab-CL2A-SN-38 extended survival to 45 days compared to 21 days in the saline-treated group (*P* = 0.0048), while the hMN-15-CL2A-SN-38 conjugate improved survival to 35 days. There was a trend favoring the milatuzumab conjugate over hMN-15-CL2A-SN-38, but it did not achieve statistical significance (46 vs. 35 days; *P* = 0.0802). However, the non-binding veltuzumab-CL2A-SN-38 conjugate provided a similar survival advantage as the milatuzumab conjugate. We previously observed therapeutic results with non-binding conjugates could be similar to the specific CL2A-linked conjugate, particularly at higher protein doses, but titration of the specific and control conjugates usually revealed selectively. Thus, neither of the specific conjugates provided a selective therapeutic advantage at these doses in this cell line.

Another study using the HuH-7 hepatoma cell line (not shown), which had similar surface expression, but slightly lower cytoplasmic levels as Hep-G2 (see **Table 4**), found the hMN-15-SN-38 conjugate providing a longer (35 vs.18 days), albeit not significantly different, survival advantage than the milatuzumab-CL2A conjugate (*P* = 0.2944). While both the hMN-15 and milatuzumab conjugates were significantly better than the saline-treated animals (*P* = 0.008 and 0.009, respectively), again, neither conjugate was significantly different from the non-targeted veltuzumab-SN-38 conjugate at this dose level (*P* = 0.4602 and 0.9033, respectively). CEACAM6 surface expression was relatively low in this cell line (MFI = 81), and immunohistology showed that both CD74 (not shown) and CEACAM6 (not shown) were very faint and highly scattered.

These studies show that neoadjuvant use of SN-38 antibody-drug conjugates is not limited to anti-TROP2 (hRS7) antibody, but may also utilize antibodies against different antigenic targets, such as CD74.

### Example 11. Use of hRS7-SN-38 (IMMU-132) to treat therapy-refractive metastatic colonic cancer (mCRC)

The patient was a 62-year-old woman with mCRC who originally presented with metastatic disease in January 2012. She had laparoscopic ileal transverse colectomy as the first therapy a couple of weeks after diagnosis, and then received 4 cycles of FOLFOX (leucovorin, 5-fluorouracil, oxaliplatin) chemotherapy in a neoadjuvant setting prior to right hepatectomy in March 2012 for removal of metastatic lesions in the right lobe of the liver. This was followed by an adjuvant FOLFOX regimen that resumed in June, 2012, for a total of 12 cycles of FOLFOX. In August, oxaliplatin was dropped from the regimen due to worsening neurotoxicity. Her last cycle of 5-FU was on 09/25/12.

CT done in Jan 2013 showed metastases to liver. She was then assessed as a good candidate for enrollment to IMMU-132 (hRS7-CL2A-SN-38) investigational study. Comorbidities in her medical history include asthma, diabetes mellitus, hypertension, hypercholesteremia, heart murmur, hiatal hernia, hypothyroidism, carpel tunnel syndrome, glaucoma, depression, restless leg syndrome, and neuropathy. Her surgical history includes tubo-ligation (1975), thyroidectomy (1983), cholescystectomy (2001), carpel tunnel release (2008), and glaucoma surgery.

At the time of entry into this trial, her target lesion was a 3.1-cm tumor in the left lobe of the liver. Non-target lesions included several hypo-attenuated masses in the liver. Her baseline CEA was 781 ng/m.

After the patient signed the informed consent, IMMU-132 was given on a once-weekly schedule by infusion for 2 consecutive weeks, then a rest of one week, this constituting a treatment cycle. These cycles were repeated as tolerated. The first infusion of IMMU-132 (8mg/kg) was started on Feb 15, 2013, and completed without notable events. She experienced nausea (Grade 2) and fatigue (Grade 2) during the course of the first cycle and has been continuing the treatment since then without major adverse events. She reported alopecia and constipation in March 2013. The first response assessment done (after 6 doses) on 04/08/2013 showed a shrinkage of target lesion by 29% by computed tomography (CT). Her CEA level decreased to 230 ng/ml on March 25, 2013. In the second response assessment (after 10 doses) on May 23, 2013, the target lesion shrank by 39%, thus constituting a partial response by RECIST criteria. She has been continuing treatment as of 06/14/13, receiving 6 cycles constituting 12 doses of hRS7-CL2A-SN-38 (IMMU-132) at 8 mg/kg. Her overall health and clinical symptoms improved considerably since starting this investigational treatment.

### Example 12. Use of hRS7-SN-38 (IMMU-132) to treat therapy-refractive metastatic breast cancer

The patient was a 57-year-old woman with stage IV, triple-negative, breast cancer (ER/PR negative, HER-neu negative), originally diagnosed in 2005. She underwent a lumpectomy of her left breast in 2005, followed by Dose-Dense ACT in adjuvant setting in September 2005. She then received radiation therapy, which was completed in November. Local recurrence of the disease was identified when the patient palpated a lump in the contralateral (right) breast in early 2012, and was then treated with CMF (cyclophosphamide, methotrexate, 5-fluorouracil) chemotherapy. Her disease recurred in the same year, with metastatic lesions in the skin of the chest wall. She then received a carboplatin + TAXOL® chemotherapy regimen, during which thrombocytopenia resulted. Her disease progressed and she was started on weekly doxorubicin, which was continued for 6 doses. The skin disease also was progressing. An FDG-PET scan on 09/26/12 showed progression of disease on the chest wall and enlarged, solid, axillary nodes. The patient was given oxycodone for pain control.

She was given IXEMPRA® from October 2012 until February 2013 (every 2 weeks for 4 months), when the chest wall lesion opened up and bled. She was then put on XELODA®, which was not tolerated well due to neuropathy in her hands and feet, as well as constipation. The skin lesions were progressive and then she was enrolled in the IMMU-132 trial after giving informed consent. The patient also had a medical history of hyperthyroidism and visual disturbances, with high risk of CNS disease (however, brain MRI was negative for CNS disease). At the time of enrollment to this trial, her cutaneous lesions (target) in the right breast measured 4.4 cm and 2.0 cm in the largest diameter. She had another non-target lesion in the right breast and one enlarged lymph node each in the right and left axilla.

The first IMMU-132 infusion (12 mg/kg) was started on March 12, 2013, which was tolerated well. Her second infusion was delayed due to Grade 3 absolute neutrophil count (ANC) reduction (0.9) on the scheduled day of infusion, one week later. After a week delay and after receiving NEULASTA®, her second IMMU-132 was administered, with a 25% dose reduction at 9 mg/kg. Thereafter she has been receiving IMMU-132 on schedule as per protocol, once weekly for 2 weeks, then one week off. Her first response assessment on May 17, 2013, after 3 therapy cycles, showed a 43% decrease in the sum of the long diameter of the target lesions, constituting a partial response by RECIST criteria. She is continuing treatment at the 9 mg/kg dose level. Her overall health and clinical symptoms improved considerably since she started treatment with IMMU-132.

### Example 13. Neoadjuvant Use of IMMU-132 to treat TNBC

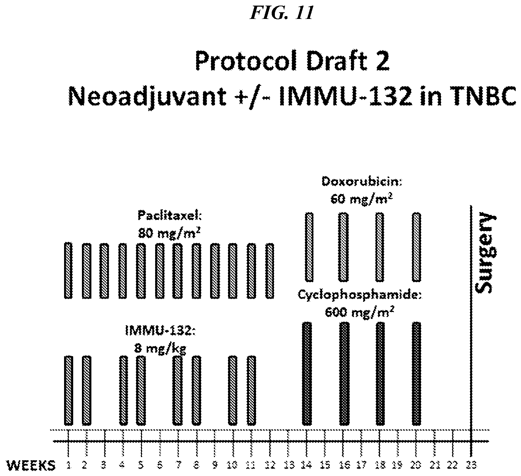

The study is open to 18 years and older females with operable TNBC, Stages II and III. Eligible patients are enrolled and randomized by computer to one of two therapy arms that are identical except for one arm having IMMU-132 added to paclitaxel in the first course of combination therapy, as shown in the following treatment regimen (**FIG. 11**). Dose-reduction and dose-delay rules are established for IMMU-132 if toxicity exceeds that predicted from prior trials, so that paclitaxel is kept at the prescribed dose if possible. Use of myeloid growth factors, such as G-CSF, are at the discretion of the treating physician for this first combination course in the investigational arm, but when patients receive the combination of doxorubicin and cyclophosphamide as the final chemotherapy course prior to surgery, prophylactic administration of G-CSF is mandated in order to reduce the risk of febrile neutropenia.

### Inclusion Criteria

1. Written informed consent by women with diagnosed invasive breast cancer who are ≥18 years old.
2. Histologically-confirmed invasive breast cancer by core needle or incisional (not excisional) biopsy. Clinical stage T2-4 N0-2 or T1 N1-2 by physical exam or radiological studies.
3. Documented Breast Cancer Gene (BRCA) germline mutation testing.
4. Estrogen receptor (ER)-negative, progesterone-receptor-negative, and human epidermal growth factor (HER2)-negative (triple-negative) breast cancer.
5. ECOG performance status 0-1.
6. Normal cardiac function confirmed by ECG and cardiac ultrasound (LVEF or shortening fraction) within 1 months of registration.
7. Adequate bone marrow function as reflected by CBC and Hgb levels.
8. Not of childbrearing potential OR a negative serum pregnancy test prior to randomization.

### Exclusion Criteria

1. Previous systemic or loco-regional anticancer therapy, including investigational agents with therapeutic intent for current breast cancer.
2. Previous treatment with any of the agents included in this trial.
3. Metastatic breast cancer
4. Concurrent treatment with an ovarian replacement therapy or with hormonal agents or any estrogen receptor modulator at the time of randomization.
5. A history of seizure within 12 months of study entry.
6. Pre-existing neuropathy from any cause in excess of Grade 1.
7. Pre-existing abnormally low values of leukocytes (neutrophils), platelets, erythrocytes, or hemoglobin.
8. History within 5 years of a cancer other than cervical cancer *in situ* or basal cell carcinoma of the skin.
9. Medical conditions that indicate intolerant to neoadjuvant therapy (uncontrolled pulmonary disease, diabetes mellitus, severe infection, active peptic ulcer, coagulation disorder, connective tissue disease, myelosuppressive disease).
10. Inadequate liver or renal function.
11. Contraindication for using dexamethasone or high-dose corticosteroids.
12. History of congestive heart failure or other serious cardiac conditions, including untrolled hypertension.
13. Pregnancy or breast-feeding.
14. Treatment with any investigational drug within 30 days before commencing this study treatment.

*Safety and Tolerability* - Safety and tolerability are assessed from adverse events, standard safety laboratories (CBC, differential and platelet counts, serum chemistries and urinalysis), physical examination, vital signs, and EKG. Additional cardiac monitoring as required by standard of care for doxorubicin therapy is performed. Adverse events are classified by the MedDRA system, and all adverse events and abnormal laboratories are classified for severity using NCI CTCAEv4.0 toxicity grades. Pharmacokinetics (PK) for IMMU-132 and also for paclitaxel in the investigational group are evaluated in a representative group of patients. Results are characterized by standard PK parameters, including peak and trough values, area-under-the-curve (AUC), Cmax, and T1/2. Immunogenicity of IMMU-132 is assessed by ELISA test measuring anti-RS7 and anti-SN-38 titers in the blood of the patients.

*Statistical Plan and Data Analysis* - We aim to achieve an increase in the pCR rate with the addition of IMMU-132 of at least 20 percentage points (57% increase) to be clinically meaningful. For power calculations, we assume a pCR rate of 35% in the control arm without IMMU-132 and 55% in the arm with IMMU-132. With 1:1 randomization, and standard statistics comparing proportions, a minimum of 94 patients in each arm will provide 80% power to detect with 95% confidence whether this advantage exists between the two arms. Thus, the minimum sample size required for this endpoint would be approximately 200 patients.

In addition, patients are followed for recurrence and survival for up to 5 years. A 10%-point improvement in event-free survival (EFS) or overall survival (OS) with the addition of IMMU-132 is accepted as providing a clinically significant benefit. Several studies of TNBC show 3 year EFS rates with standard neoadjuvant chemotherapy of 60-80%. For calculations, we assume 70% in the control arm, since this is determined from the large meta-analysis of 1157 TNBC patients (43), and therefore 80% in the arm with IMMU-132. Assuming EFS is approximately an exponentially decaying function, the median EFS is 5.8 years in the control arm and 9.4 years in the arm with IMMU-132, with a 0.62 hazard ratio (HR) between treatment arms. Assuming accrual over no sooner than 3 years with analysis no sooner than after 4 years of follow-up, about 200 patients in each arm will provide 85% power to detect with 95% confidence whether this EFS difference exists between the two arms. Thus, using EFS to demonstrate a clinically significant benefit, the required sample size required for this endpoint is approximately 400 patients, but with an overage of 10% to provide for drop-outs, we estimate that 440 patients are enrolled for this trial.

Most studies of neoadjuvant therapy in TNBC consider recurrence endpoints, not survival. While this study has not been powered for OS, an MD Anderson study (Liedtke et al., J Clin Oncol. 2008 Mar 10;26(8):1275-1281) reported that OS rates at 5 years were close to their PFS rates at 3 years. This suggests that sufficient events would have occurred after approximately 2 additional years of follow-up to have similar power to demonstrate the same 10%-point improvement measure of clinically significant benefit based on OS.

### Example 14. Use of hRS7-SN-38 (IMMU-132) to treat refractory, metastatic, non-small cell lung cancer

A 60-year-old man is diagnosed with non-small cell lung cancer. The patient then presents with a left mediastinal mass measuring 6.5 x 4 cm and pleural effusion. After signing informed consent, the patient is given IMMU-132 at a dose of 18 mg/kg every other week. During the first two injections, brief periods of neutropenia and diarrhea are experienced, with 4 bowel movements within 4 hours, but these resolve or respond to symptomatic medications within 2 days. After a total of 6 infusions of IMMU-132, CT evaluation of the index lesion shows a 22% reduction, just below a partial response but definite tumor shrinkage. The patient continues with this therapy for another two months, when a partial response of 45% tumor shrinkage of the sum of the diameters of the index lesion is noted by CT, thus constituting a partial response by RECIST criteria.

The patient is then given chemotherapy regimens of carboplatin, bevacizumab for 6 months and shows a response, and then after progressing, receives further courses of chemotherapy with carboplatin, etoposide, TAXOTERE®, and gemcitabine. The mediastinal tumor is eliminated with the neoadjuvant use of ADC combined whith chemotherapy.

### Example 15. Treatment of Metastatic Pancreatic Cancer with Anti-MUC5ac-CL2A-SN-38 Immunoconjugate

This 44-year-old patient has a history of metastatic pancreatic carcinoma, with pancreas ductal adenocarcinoma in the pancreas head, and showing metastases to left and right lobes of the liver, the former measuring 3 x 4 cm and the latter measuring 2x3 cm. The patient is given a course of gemcitabine but shows no objective response. Four weeks later, he is given hPAM4-CL2A-SN-38 i.v. at a dose of 8 mg/kg twice-weekly for 2 weeks, with one week off, and then repeated for another 2 cycles. CT studies are done one week later and elimination of the metastases and a reduction in the primary tumor mass of 32% (partial response), alongside a drop in his blood CA19-9 titer from 220 at baseline to 75 at the time of radiological evaluation. The patient shows only grade 1 nausea and vomiting after each treatment with the antibody-drug conjugate, and a grade 2 neutropenia at the end of the last treatment cycle, which resolves 4 weeks later. The elimination of metastatic lesions and size reduction of the primary tumor allows surgical removal of the previously inoperable tumor mass. Six months after surgical treatment, the patient shows no signs of recurrence of the pancreatic carcinoma.

### Example 16. Production and Use of Pro-2-Pyrrolinodoxorubicin (P2PDox) (not claimed)

Synthesis - Structures of intermediates in the synthetic pathway of P2PDox, as well as a maleimide derivative of P2PDox suitable for conjugation to antibodies or other proteins or sulfhydryl-containing peptides, are disclosed herein. A general scheme for producing an exemplary P2PDox is shown in Scheme 1 below. We have performed 1-g scale reactions to generate > 1 g of 4,4-diacetoxybutyraldehyde in an yield of ∼40%. To avoid using sodium cyanoborohydride that can potentially contaminate products with cyanide, the reducing agent was changed to sodium triacetoxyborohydride in reductive alkylation. On an exploratory scale, >80% conversion of doxorubicin to P2PDox was recorded. This was increased to 2-g scale to generate > 1g of P2PDox. (Scheme 1). The 4,4-diacetoxybutyraldehyde was prepared by a modification of the reported method (Nagy et al., 1998, Proc Natl Acad Sci U S A 95:1794-9), which was necessary to avoid a hazardous ozonolysis step. Diacetoxylation of commercially available 4-pentene-1-al with acetic anhydride and indium chloride catalysis, followed by oxidative cleavage of olefin by ruthenium chloride and sodium periodate combination (Yang & Zhang, 2001, 66:4814-8) furnished the 4,4-diacetoxybutyraldehyde, which was reductively coupled to doxorubicin to obtain P2PDox.

The following steps were involved: (i) To a mixture of acetic anhydride (7.45 mL) and indium chloride (0.56 g) in dichloromethane (20 mL) was added 5.05 g of 4-penten-1-al. After 10 to 30 min, the reaction mixture was treated with 25% aqueous sodium acetate (20 mL), and the organic layer was washed with brine and dried. Solvent removal gave 15.3 g of the liquid prouct, which was taken to the next step; (ii) 3.5 mM ruthenium chloride stock solution in water (69.4 mL) was added to the solution of the step (i) product in dichloromethane in 6:1 acetonitrile-water (350 mL). Sodium periodate (29.7 g) was added in portions. After completion of reaction, as judged by TLC analysis, the reaction mixture was treated with 30 mL of saturated sodium thiosulfate, filtered through a pad of celite, and acetonitrile was evaporated off. The remaining aqueous layer was extracted with ethyl acetate, washed with 25% sodium acetate, water, and brine, and dried. The crude material was purified by chromatography on silica gel using ethyl acetate-hexane mixture for elution. The pure product was used for reductive alkylation of doxorubicin in the next step; (iii) 1.5 grams of doxorubicin hydrochloride was dissolved in 1,1,1,3,3,3,-hexafluoroisopropanol (195 mL) and diisopropylethylamine (2.7 mL), and reacted with 3.4 g (7-fold molar excess) of the aldehyde from step (ii) and 0.66 g of sodium triacetoxyborohydride. The reaction was complete in 10 min, and the product was purified on silica gel using methylene chloride-isopropanol mixtures for elution, resulting in 0.96 g of pure product. Electrospray mass spectrum showed the mass at m/z 716.2570 (M+H) consistent with the structure of the product. The structure was also confirmed by proton and C-13 NMR spectra. (iv) P2PDox from step iii was converted to MCC hydrazone using SMCC hydrazide as follows: To 0.6 g of P2PDox dissolved in 75 mL of anhydrous methanol, and treated with 0.34 g of SMCC hydrazide, calculated to be 1.8-fold excess based on the spectrophotometric quantification of the amount of P2PDox used. The percent conversion was judged to be 88% by HPLC. LC-MS analysis the showed the product peak at m/z of 949.3734 (M+H), consistent with the calculated mass (m/z) of 949.3713 (M+H). The material, after solvent removal, was used as such for conjugation since underivatized starting material did not conjugate and was removed during conjugate purification process.

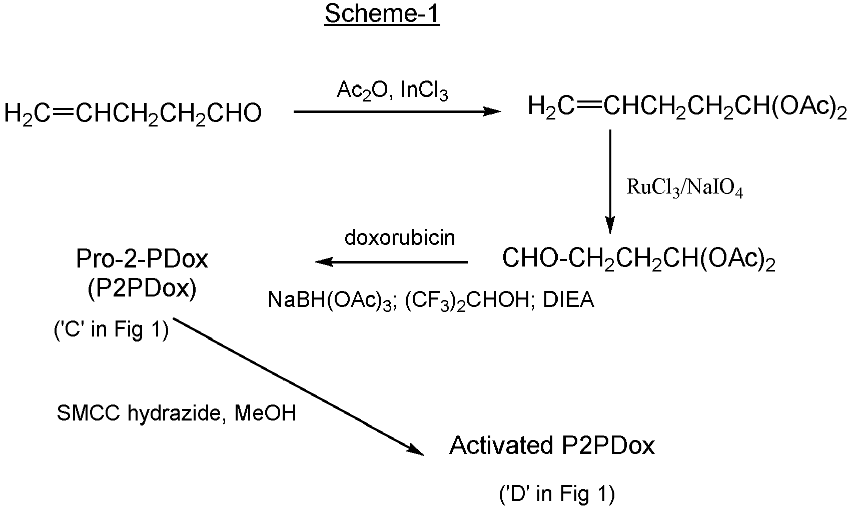

Small-scale conjugate preparation - Conjugate preparation followed a general methodology of mildly reducing interchain disulfides of IgG with TCEP in PBS, followed by coupling to a 10-fold excess of activated P2PDox. The conjugates were purified on centrifuged size exclusion chromatography (SEC) on SEPHADEX® equilibrated in 25 mM 3-(N-morpholino)propanesulfonic acid (MOPS), pH 6.8, followed by passage over a hydrophobic column. The products were formulated with trehalose and polysorbate 80, and lyophilized. The conjugated product, with a substitution in the range of 4-7 drug/IgG, eluted as a single peak by size-exclusion HPLC, and contained typically <1% of unconjugated free drug by reversed-phase HPLC.

Scaled-up conjugate preparation - Conjugate of humanized anti-TROP-2 antibody, hRS7, was prepared, on 5-g and 10-g scale, by TCEP reduction of an antibody, followed by *in situ* conjugation using a 12-fold excess of activated P2PDox, with DMSO as co-solvent (5% v/v). The product was purified by tangential flow filtration using 25 mM MOPS buffer, pH 6.8, with 20-diafiltration volumes for purification. The product was formulated with 25 mM trehalose and 0.01% TWEEN® 80, aliquotted in 20-mg or 100-mg lots, and lyophilized.

### Representative Conjugates

| | Conjugate | Lot | % Protein recover | P2PDo x/IgG | HPLC | |
|---|---|---|---|---|---|---|
| | | | | | Aggr. | Free drug |
| 1 | hIMMU-31-P2PDox | II22-138 | 75.0% | 7.39 | 1.9% | 0.26% |
| 2 | hA20-P2PDox | II22-135 | 85.7% | 6.79 | <2% | <0.1% |
| 3 | hLL1-P2PDox | II22-145 | 88.6% | 7.10 | 2.8% | 0.2% |
| 4 | hRS7-P2PDox | II22-142 | 80.1% | 7.17 | 1.8% | 0.12% |
| 5 | hMN15-P2PDox | II22-180 | 74.9% | 6.87 | 1.1% | 0.46% |
| 6 | hMN-14-P2PDox | II22-183 | 80.2% | 6.78 | 2.1% | 0.53% |

Conjugates have also been prepared for hPAM4-P2PDox, hLL2-P2PDox and RFB4-P2PDox, with similar protein recovery and purity (not shown).

### Example 17. In Vitro Preclinical Studies With P2PDox (not claimed)

*In vitro* cell-binding studies - Retention of antibody binding was confirmed by cell binding assays comparing binding of the conjugate to unconjugated antibody (Chari, 2008, Acc Chem Res 41:98-107). The potency of the conjugate was tested in a 4-day MTS assay using appropriate target cells. The hRS7-P2PDox conjugate exhibited IC₅₀ values of 0.35-1.09 nM in gastric (NCI-N87), pancreatic (Capan-1), and breast (MDA-MB-468) human cancer cell lines, with free drug exhibiting 0.02-0.07 nM potency in the same cell lines.

Serum stability - Serum stability of prototypical P2PDox conjugate, hRS7-P2PDox, was determined by incubating in human serum at a concentration of 0.2 mg/mL at 37 ⁰C. The incubate was analyzed by HPLC using butyl hydrophobic interaction chromatography (HIC) column in which there was good retention time separation between the peak due to free drug and that due to conjugate or higher molecular weight species. This analysis showed that there was no release of free drug from the conjugate, suggesting high serum stability of the conjugate. When the same experiment was repeated with hRS7-doxorubicin conjugate, containing the same cleavable linker as hRS7-P2PDox, and where the free drug was independently verified to be released with a half-life of 96 h, clear formation of free drug peak, namely doxorubicin peak, was seen on HIC HPLC.

Surprisingly, it was determined that the P2PDox conjugate was held tightly to the antibody because it cross-linked the peptide chains of the antibody together. The cross-linking stabilizes the attachment of the drug to the antibody so that the drug is only released intracellularly after the antibody is metabolized. The cross-linking assists in minimizing toxicity, for example cardiotoxicity, that would result from release of free drug in circulation. Previous use of 2-PDox peptide conjugates failed because the drug cross-linked the peptide to other proteins or peptides *in vivo.* With the present conjugates, the P2PDox is attached to interchain disulfide thiol groups while in the prodrug form. The prodrug protection is rapidly removed *in vivo* soon after injection and the resulting 2-PDox portion of the conjugate crosslinks the peptide chains of the antibody, forming intramolecular cross-linking within the antibody molecule. This both stabilizes the ADC and prevents cross-linking to other molecules in circulation.

### Example 18: In Vivo Preclinical Studies With P2PDox (not claimed)

General - Tumor size was determined by caliper measurements of length (L) and width (W) with tumor volume calculated as (LxW²)/2. Tumors were measured and mice weighed twice a week. Mice were euthanized if their tumors reached >1 cm³ in size, lost greater than 15% of their starting body weight, or otherwise became moribund. Statistical analysis for the tumor growth data was based on area under the curve (AUC) and survival time. Profiles of individual tumor growth were obtained through linear curve modeling. An *f-*test was employed to determine equality of variance between groups prior to statistical analysis of growth curves. A two-tailed *t*-test was used to assess statistical significance between all the various treatment groups and non-specific controls. For the saline control analysis a one-tailed *t*-test was used to assess significance. Survival studies were analyzed using Kaplan-Meier plots (log-rank analysis), using the Prism GraphPad Software (v4.03) software package (Advanced Graphics Software, Inc.; Encinitas, CA). All doses in preclinical experiments are expressed in antibody amounts. In terms of drug, 100 µg of antibody (5 mg/kg) in a 20-g mouse, for example, carries 1.4 µg-2.8 µg (0.14-0.17 mg/kg) of P2PDox equivalent dose when using an ADC with 3-6 drugs/IgG.

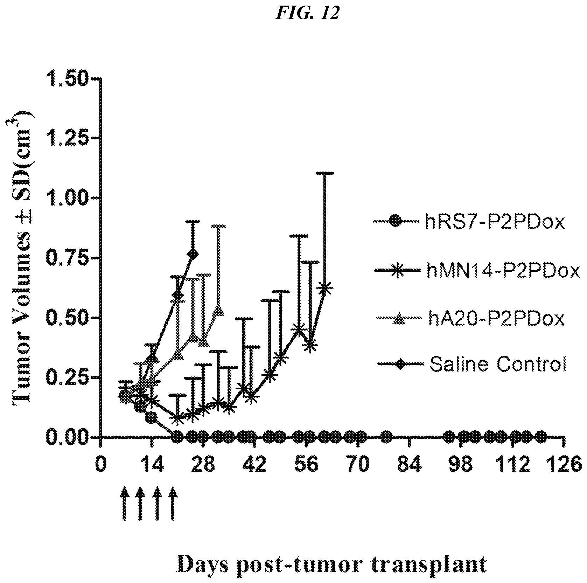

A single i.v. dose of ≥ 300 µg [∼10 µg of P2PDox] of the conjugate was lethal, but 4 doses of 45 µg given in 2 weeks were tolerated by all animals. Using this dosing regimen, we examined the therapeutic effect of hRS7-P2PDox in 2 human tumor xenograft models, Capan-1 (pancreatic cancer) and NCI-N87 (gastric cancer). Therapy began 7 days after tumor transplantation in nude mice. In the established, 7-day-old, Capan-1 model, 100% of established tumors quickly regressed, with no evidence of re-growth (**FIG. 12**). This result was reproduced in a repeat experiment (not shown). Similar findings were made in the established NCI-N87 model (not shown), where a 2^{nd} course of therapy, administered after day 70, was safely tolerated and led to further regressions of residual tumor (not shown). The internalizing hRS7-SN-38 conjugate, targeting Trop-2, provided better therapeutic responses than a conjugate of a poorly internalizing anti-CEACAM5 antibody, hMN-14 (**FIG. 2**). A non-targeted anti-CD20 ADC, hA20-P2PDox, was ineffective, indicating selective therapeutic efficacy (**FIG. 2**). Data from a breast cancer xenograft (MDA-MB-468) and a second pancreatic cancer xenograft (not shown) reiterate the same trend of the conjugate's specific and significant antitumor effects.

PK and toxicity of hRS7-P2PDox with substitutions of 6.8 or 3.7 drug/IgG - Antibody-drug conjugates (ADCs) carrying as much as 8 ultratoxic drugs/MAb are known to clear faster than unmodified MAb and to increase off-target toxicity, a finding that has led to the current trends to use drug substitutions of ≤ 4 (Hamblett et al., 2004, Clin Cancer Res 10:7063-70). Conjugates were prepared and evaluated with mean drug/MAb substitution ratios (MSRs) of ∼6:1 and ∼3:1. Groups of normal mice (n = 5) were administered, i.v., single doses of unmodified hRS7 or hRS7-P2PDox with drug substitution of 6.8 or 3.7 (same protein dose), and serum samples were collected at 30 min, 4 h, 24 h, 72 h, and 168 h post-injection. These were analyzed by ELISA for antibody concentration. There were no significant differences in serum concentrations at various times, indicating that these cleared similarly. The PK parameters (Cmax, AUC, etc.) were similar. Conjugates with either higher or lower drug substitution had similar tolerability in nude mice, when the administered at the same dose of conjugated drug.

Therapeutic Efficacy at Minimum Effective Dose (MED) - Anti-TROP-2 antibody conjugate, hRS7-P2PDox, was evaluated in nude mice bearing NCI-N87 human gastric cancer xenografts by administering a single bolus protein dose of 9 mg/kg, 6.75 mg/kg, 4.5 mg/kg, 2.25 mg/kg, or 1 mg/kg. The therapy was started when the mean tumor volume (mTV) was 0.256 cm³. On day 21, mTV in the saline control group (non-treatment group) was 0.801 ± 0.181 cm³ which was significantly larger than that in mice treated with 9, 6.75, 4.5, or 2.25 mg/kg dose with mTV of 0.211 ± 0.042 cm³, 0.239 ± 0.0.054 cm³, 0.264 ± 0.087 cm³, and 0.567 ± 0.179 cm³, respectively (P<0.0047, one tailed t-test). From these, the minimum effective dose was judged to be 2.25 mg/kg, while 9 mg/kg represented MTD.

### Example 19. MTD of Antibody-P2PDox (not claimed)

An MTD study comparing 2-PDox and P2PDox conjugates of prototype antibody, hLL1, in mice demonstrated that the P2PDox conjugate was much more potent (not shown). The MTD of a single i.v. injection was between 100 and 300 µg. The MTD of multiple injections, at a schedule of every four days for a total of four injections (q4dx4) was then determined, using protein doses between 25 µg to 150 µg per injection. At these doses, a cumulative dose of between 100 and 600 µg was given to the animals. **Table 5** below summarizes the various groups.

**Table 5. Dosage and Schedule for MTD of antibody-P2PDox**

| **12 Female Athymic Nude Mice** | | | |
|---|---|---|---|
| **Group** | **N** | **Treatment** | **Total Amount** |
| **1** | **3** | 25 µg i.v. q4dx4 | 100 µg |
| **2** | **3** | 50 µg i.v. q4dx4 | 200 µg |
| **3** | **3** | 100 µg i.v. q4dx4 | 400 µg |
| **4** | **3** | 150 µg i.v. q4dx4 | 600 µg |

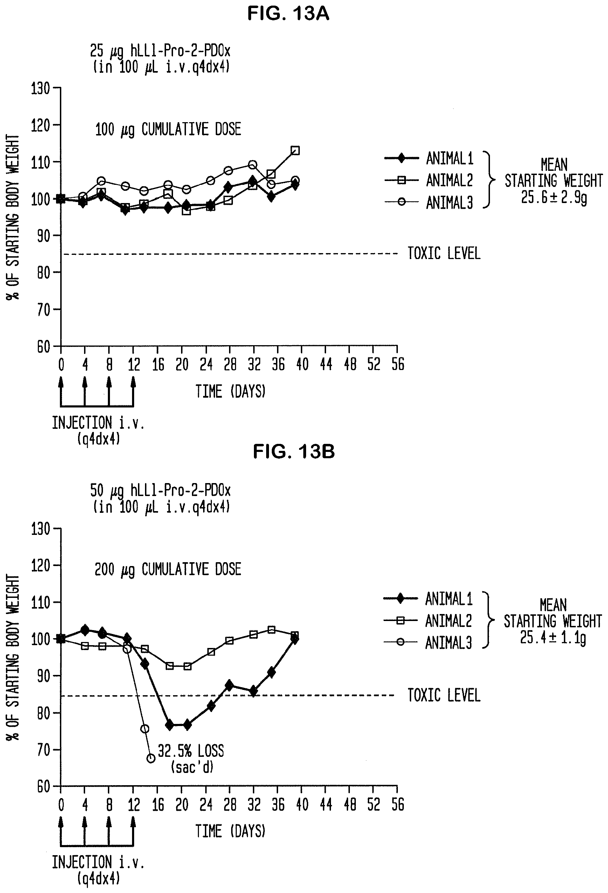

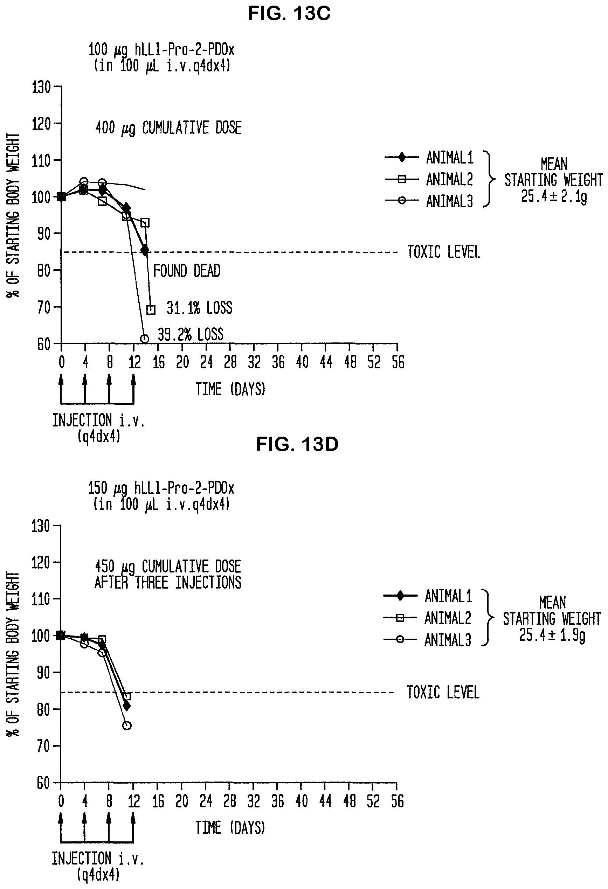

Graphs showing weight loss are shown in **FIG. 13A-D**. Only those mice treated with 25 µg P2PDox-ADC continue to show no signs of toxicity. This is a cumulative dose of 100 µg which was also the dose tolerated when administered as a single injection (not shown). Therefore, the MTD for multiple injections of a P2PDox-ADC in mice is 25 µg q4dx4 from this experiment. A careful analysis of data and repetition of the experiment established the MTD for fractionated dosing to be 45 µg of protein dose of the conjugate, administered every 4 days for 2 weeks (45 µg, q4dx4 schedule).

### Example 20. Additional Studies With P2PDox Conjugates (not claimed)

No significant difference in binding of the antibody moiety to NCI-N87 gastric carcinoma cells was observed between unconjugated hRS7 and P2PDox-hRS7 conjugated to 6 molecules of P2PDox per antibody (not shown). The lack of effect of conjugation on antibody binding to target antigen was confirmed for P2PDox-hMN-15 (anti-CEACAM6), P2PDox-hLL2 (anti-CD22) and P2PDox-hMN-24 (anti-CEACAM5) conjugates. It is concluded that conjugation of P2PDox to antibodies does not affect antibody-antigen binding activity.

The cytotoxicity of P2PDox-mAb conjugates to target cells was examined. hRS7-P2PDox and hMN-15-P2PDox were cytotoxic to MDA-MB-468, AG S, NCI-N87 and Capan-1 solid tumor cell lines (not shown). hMN-14-P2PDox was cytotoxic to Capan-1, BxPC-3 and AsPC-1 human pancreatic tumor lines and AGS, NCI-N87 and LS147T human gastric and colonic tumor lines (not shown). hLL2-P2PDOx was cytotoxic to Daudi, Raji, Ramos and JVM-3 hematopoietic tumor lines (not shown). IC₅₀ values for the conjugates were in the nanomolar concentration range (not shown).

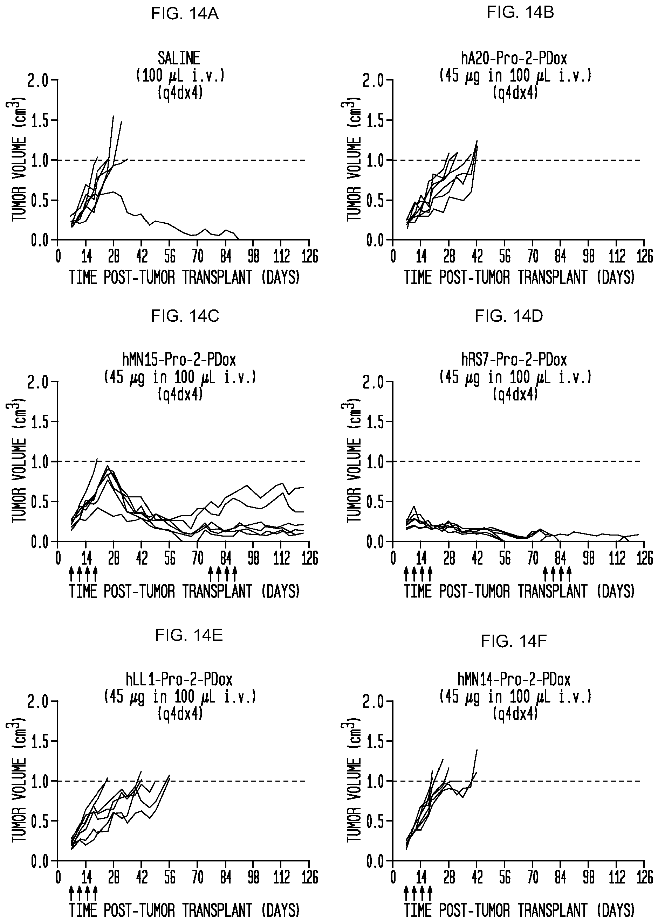

Further *in vivo* efficacy studies were performed in nude mice implanted with NCI-N87 human gastric cancer xenografts (**FIG. 14A-F**). One treatment cycle with 4 x 45 µg of hRS7-P2PDox rapidly regressed all tumors (**FIG. 14D**). A second treatment cycle was initiated about 2 months after the end of the first cycle, resulting in complete regression of all but one of the hRS7-P2PDox treated animals. The hA20, hLL1 and hMN-14 conjugates had little effect on tumor progression (**FIG. 14A, 14B, 14E** and **14F**). Administration of P2PDox-hMN-15 resulted in a delayed regression of gastric cancer, which was less effective than the hRS7 conjugate.

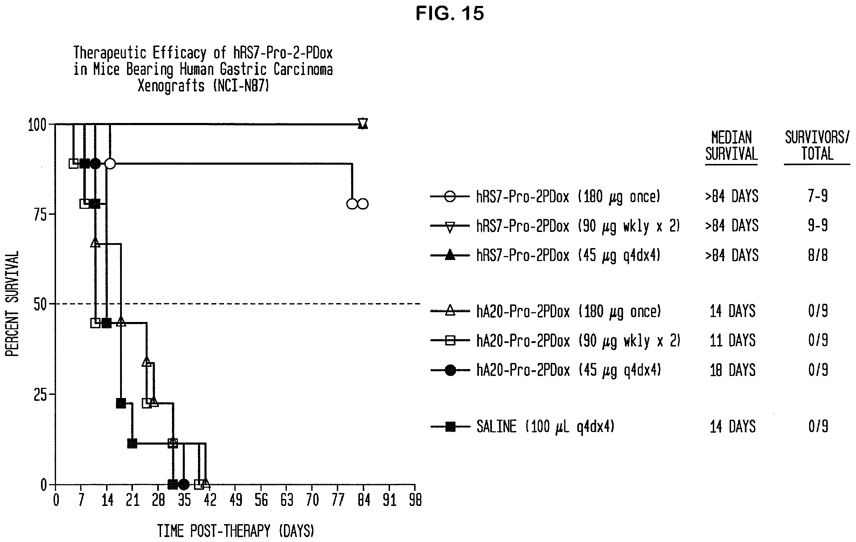

The effect of varying dosage schedule on anti-tumor efficacy was examined (**FIG. 15**). The experiment began 9 days after tumor implantation when mean tumor volume for all groups was 0.383 cm³, and ended on day 93 (84 days after initiation of therapy). In this study, a single dose of 180 µg, two weekly doses of 90 µg, and q4dx4 of 45 µg all resulted in significantly enhanced survival (**FIG. 15**). For the saline control, 0 of 9 mice survived (not shown). For mice receiving 45 µg q4dx4 of hRS7-P2PDox, 8 of 9 mice were alive at day 94 (not shown). For mice receiving 90 µg weekly x 2 of hRS7-P2PDox, 9 of 9 mice were alive at day 94 (not shown). For mice receiving a single dose of 180 µg of hRS7-P2PDox,78 of 9 mice were alive at day 94 (not shown).

At the same dosage schedule, the control hA20 conjugate had no effect on survival (**FIG. 15**). A toxicity study showed that the three dosage schedules of hRS7-P2PDox resulted in similarly low levels of toxicity (not shown).

The hRS7-P2PDox conjugate was also effective in Capan-1 pancreatic cancer (not shown) and was more effective at inhibiting tumor growth than a hRS7-SN-38 conjugate (not shown). The hPAM4-P2PDox conjugate was also more effective at inhibiting growth of Capan-1 human pancreatic cancer than an hPAM4-SN-38 conjugate (not shown). At 63 days after Capan-1 tumor injection (with therapy starting at 1 days post-innoculation), 0 of 10 mice were alive in the saline control, 10 of 10 mice were alive in mice treated twice weekly x 2 weeks with 45 µg of hPAM4-P2PDox, 2 of 10 mice were alive in mice treated twice weekly x 2 weeks with 45 µg of hA20-P2PDox, 0 of 10 mice were alive in mice treated twice weekly x 4 weeks with 250 µg of hPAM4-SN-38, and 0 of 10 mice were alive in mice treated twice weekly x 4 weeks with 250 µg of h20-SN-38.

hRS7-P2PDox was substantially more effective than hRS7-SN-38 at inhibiting growth of PxPC-3 pancreatic cancer (not shown) and was slightly more effective than hRS7-SN-38 at inhibiting growth of MDA-MB-468 breast cancer (not shown).

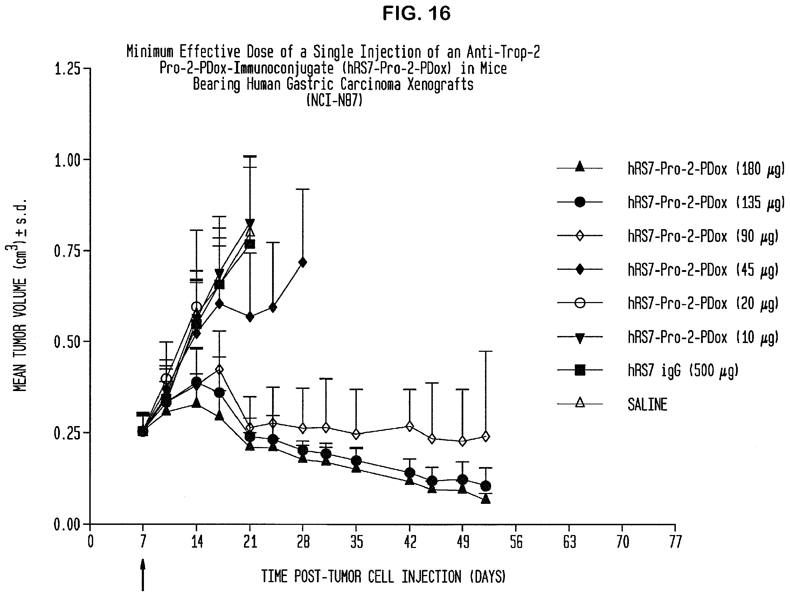

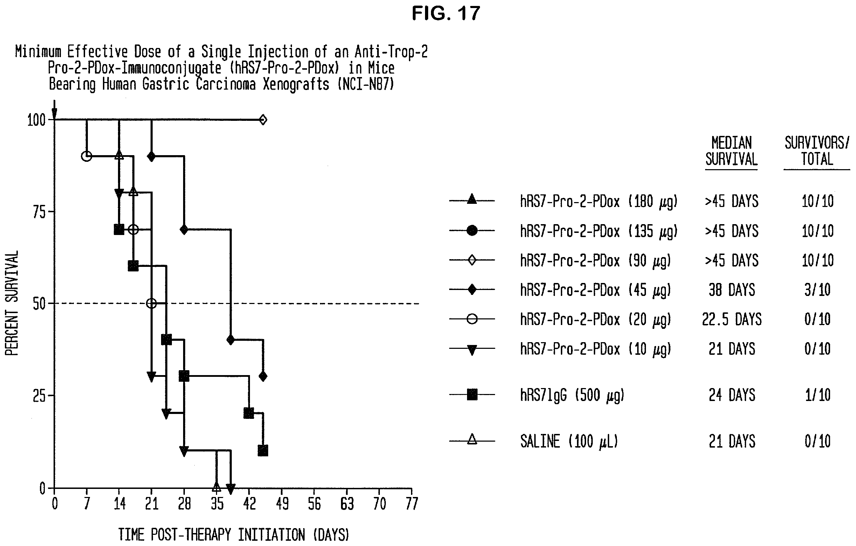

The effect of different single doses of hRS7-P2PDox on growth of NCI-N87 gastric carcinoma xenografts is shown in **FIG. 16****.** Using a single dose, the maximum effect on tumor growth was observed at 90 µg or higher **(****FIG. 16****).** A single dose of 45 µg was the minimum required to see a significant survival benefit compared to saline control **(****FIG. 17****).**

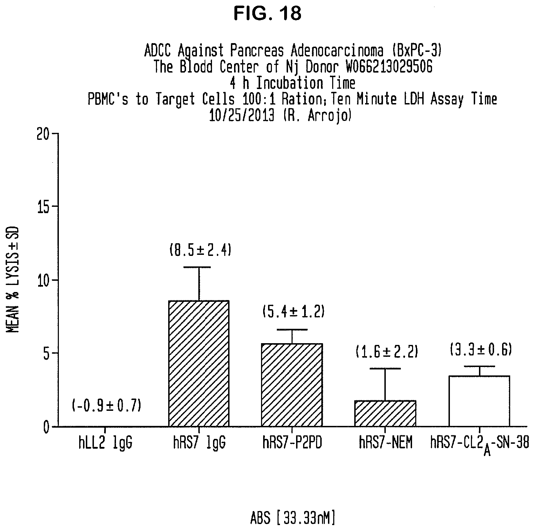

The ADCC activity of various hRS7-ADC conjugates was determined in comparison to hRS7 IgG **(****FIG. 18****).** PBMCs were purified from blood purchased from the Blood Center of New Jersey. A Trop-2-positive human pancreatic adenocarcinoma cell line (BxPC-3) was used as the target cell line with an effector to target ratio of 100:1. ADCC mediated by hRS7 IgG was compared to hRS7-Pro-2-PDox, hRS7-CL2A-SN-38, and the reduced and capped hRS7-NEM. All were used at 33.3 nM.

Results are shown in **FIG. 18****.** Overall activity was low, but significant. There was 8.5% specific lysis for the hRS7 IgG which was not significantly different from hRS7-Pro-2-PDox. Both were significantly better than hLL2 control and hRS7-NEM and hRS7-SN-38 (P<0.02, two-tailed *t*-test). There was no difference between hRS7-NEM and hRS7-SN-38.

### Example 21. Treatment of Triple Negative Breast Cancer With P2PDox-hRS7 ADC (not claimed)

P2PDox-hRS7 ADC is prepared as described in the Examples above. Patients with triple-negative breast cancer who had failed at least two standard therapies receive 3 cycles of 70 mg P2PDox-hRS7 injected i.v. every 3 weeks. Objective responses are observed at this dose level of P2PDox- hRS7, with an average decrease in tumor volume of 35%, after two cycles of therapy. All serum samples evaluated for human anti-hRS7 antibody (HAHA) are negative. Reduction of tumor mass is followed by surgical removal of the tumor.

## Claims

1. An antibody-drug conjugate (ADC) for use in a method for neoadjuvant treatment of cancer comprising:
a) administering the antibody-drug conjugate (ADC) comprising an antibody moiety conjugated to a drug, to a subject with cancer, wherein the antibody moiety is an anti-Trop-2 hRS7 antibody and the drug is SN-38; and
b) treating the subject with a standard anti-cancer therapy selected from the group consisting of surgery, and radiation therapy, and
wherein the ADC is administered before the standard anti-cancer therapy.

2. A standard anti-cancer therapy for use in a method for neoadjuvant treatment of cancer comprising:
a) administering an antibody-drug conjugate (ADC) comprising an antibody moiety conjugated to a drug, to a subject with cancer, wherein the antibody moiety is an anti-Trop-2 hRS7 antibody and the drug is SN-38; and
b) treating the subject with the standard anti-cancer therapy, wherein the standard anti-cancer therapy is selected from the group consisting of surgery, and radiation therapy, and
wherein the ADC is administered before the standard anti-cancer therapy.

3. The antibody-drug conjugate (ADC) for use of claim 1 and the standard anti-cancer therapy for use of claim 2, wherein the antibody moiety comprises human constant regions selected from the group consisting of IgG1, IgG2, IgG3 and IgG4.

4. The antibody-drug conjugate (ADC) for use of claim 1 and the standard anti-cancer therapy for use of claim 2, wherein the antibody moiety has an allotype selected from the group consisting ofnon-G1m1 (nG1m1), G1m3, and Km3.

5. The antibody-drug conjugate (ADC) for use of claim 1 and the standard anti-cancer therapy for use of claim 2, wherein the ADC comprises a linker that attaches the drug to the antibody moiety.

6. The antibody-drug conjugate (ADC) for use of claim 5 and the standard anti-cancer therapy for use of claim 5, wherein the drug is SN-38 and the linker is CL2A.

7. The antibody-drug conjugate (ADC) for use of claim 1 and the standard anti-cancer therapy for use of claim 2, further comprising administering at least one therapeutic agent to said subject.

8. The antibody-drug conjugate (ADC) for use of claim 7 and the standard anti-cancer therapy for use of claim 7, wherein the therapeutic agent is selected from the group consisting of a toxin, an enzyme, a hormone, a second antibody, a second antibody fragment, an immunoconjugate, a radionuclide, an antisense oligonucleotide, and an RNAi.

9. The antibody-drug conjugate (ADC) for use of claim 8 and the standard anti-cancer therapy for use of claim 8, wherein the radionuclide is attached to an antibody or antigen-binding antibody fragment and is selected from the group consisting of ¹¹¹In, ¹¹¹At, ¹⁷⁷Lu, ²¹¹Bi, ²¹²Bi, ²¹³Bi, ²¹¹At, ⁶²Cu, ⁶⁷Cu, ⁹⁰Y, ¹²⁵I, ¹³¹I ¹³³I ³²P, ³³P, ⁴⁷Sc, ¹¹¹Ag, ⁶⁷Ga, ¹⁵³Sm, ¹⁶¹Tb, ¹⁵²Dy, ¹⁶⁶Dy, ¹⁶¹Ho, ¹⁶⁶Ho, ¹⁸⁶Re, ¹⁸⁸Re, ¹⁸⁹Re, ²¹¹Pb, ²¹²Pb, ²²³Ra, ²²⁵AC, ⁷⁷As, ⁸⁹Sr, ⁹⁹Mo, ¹⁰⁵Rh, ¹⁴⁹Pm, ¹⁶⁹Er, ¹⁹⁴Ir, ⁵⁸Co , ^{80m}Br, ^{99m}Tc, ^{103m}Rh , ¹⁰⁹Pt, ¹¹⁹Sb, ^{189m}Os, ¹⁹²Ir ²¹⁹Rn, ²¹⁵Po, ²²¹Fr, ²⁵⁵Fm, ¹¹C, ¹³N, ¹⁵O, ⁷⁵Br, ¹⁹⁸Au, ¹⁹⁹Au, ²²⁴Ac, ⁷⁷Br, ^{113m}In, ⁹⁵Ru, ⁹⁷Ru, ¹⁰³Ru, ¹⁰⁵Ru, ¹⁰⁷Hg, ²⁰³Hg, ^{121m}Te, ^{122m}Te, ^{125m}Te, ²²⁷Th, ¹⁶⁵Tm, ¹⁶⁷Tm, ¹⁶⁸Tm, ¹⁹⁷Pt, ¹⁰⁹Pd, ¹⁴²Pr, ¹⁴³Pr, ¹⁶¹Tb, ⁵⁷Co , ⁵⁸Co, ⁵¹Cr, ⁵⁹Fe, ⁷⁵Se, ²⁰¹T1, ⁷⁶Br and ¹⁶⁹Yb.

10. The antibody-drug conjugate (ADC) for use of claim 1 and the standard anti-cancer therapy for use of claim 2, wherein the cancer is selected from the group consisting of colon cancer, stomach cancer, esophageal cancer, medullary thyroid cancer, kidney cancer, breast cancer, lung cancer, pancreatic cancer, urinary bladder cancer, ovarian cancer, uterine cancer, cervical cancer, testicular cancer, prostate cancer, liver cancer, skin cancer, bone cancer, brain cancer, rectal cancer, and melanoma.

11. The antibody-drug conjugate (ADC) for use of claim 1 and the standard anti-cancer therapy for use of claim 2, wherein the cancer is selected from the group consisting of triple negative breast cancer (TNBC), metastatic colon cancer, metastatic non-small-cell lung cancer (NSCLC), metastatic pancreatic cancer, metastatic renal cell carcinoma, metastatic gastric cancer, metastatic prostate cancer, and metastatic small-cell lung cancer.

12. The antibody-drug conjugate (ADC) for use of claim 1 and the standard anti-cancer therapy for use of claim 2, wherein the cancer is refractory to other therapies but responds to therapy with neoadjuvant ADC.

## Patentansprüche

1. Antikörper-Wirkstoff-Konjugat (ADC) zur Verwendung in einem Verfahren zur neoadjuvanten Behandlung von Krebs, umfassend
a) Verabreichen des Antikörper-Wirkstoff-Konjugates (ADC) umfassend einen an einen Wirkstoff konjugierten Antikörperteil an ein Lebewesen mit Krebs, wobei der Antikörperteil an anti-Trop-2 hRS7-Antikörper und der Wirkstoff SN-38 ist; und
b) Behandeln des Lebewesens mit einer standardmäßigen Krebstherapie, die ausgewählt ist aus der Gruppe bestehend aus Operation und Strahlentherapie, und
wobei das ADC vor der standardmäßigen Krebstherapie verabreicht wird.

2. Standardmäßige Krebstherapie zur Verwendung in einem Verfahren zur neoadjuvanten Behandlung von Krebs, umfassend:
a) Verabreichen eines Antikörper-Wirkstoff-Konjugates (ADC) umfassend einen an einen Wirkstoff konjugierten Antikörperteil an ein Lebewesen mit Krebs, wobei der Antikörperteil ein anti-Trop-2 hRS7-Antikörper und der Wirkstoff SN-38 ist; und
b) Behandeln des Lebewesens mit der standardmäßigen Krebstherapie, wobei die standardmäßige Krebstherapie ausgewählt ist aus der Gruppe bestehend aus Operation und Strahlentherapie, und
wobei das ADC vor der standardmäßigen Krebstherapie verabreicht wird.

3. Antikörper-Wirkstoff-Konjugat (ADC) zur Verwendung nach Anspruch 1 und standardmäßige Krebstherapie zur Verwendung nach Anspruch 2, wobei der Antikörperteil humane konstante Regionen umfasst, die ausgewählt sind aus der Gruppe bestehend aus IgG1, IgG2, IgG3 and IgG4.

4. Antikörper-Wirkstoff-Konjugat (ADC) zur Verwendung nach Anspruch 1 und standardmäßige Krebstherapie zur Verwendung nach Anspruch 2, wobei der Antikörperteil einen Allotyp aufweist, der ausgewählt ist aus der Gruppe bestehend aus nicht-G1m1 (nG1m1), G1m3 und Km3.

5. Antikörper-Wirkstoff-Konjugat (ADC) zur Verwendung nach Anspruch 1 und standardmäßige Krebstherapie zur Verwendung nach Anspruch 2, wobei das ADC einen Linker umfasst, der den Wirkstoff an den Antikörperteil bindet.

6. Antikörper-Wirkstoff-Konjugat (ADC) zur Verwendung nach Anspruch 5 und standardmäßige Krebstherapie zur Verwendung nach Anspruch 5, wobei der Wirkstoff SN-38 und der Linker CL2A ist.

7. Antikörper-Wirkstoff-Konjugat (ADC) zur Verwendung nach Anspruch 1 und standardmäßige Krebstherapie zur Verwendung nach Anspruch 2, weiter umfassend Verabreichen von wenigstens einem therapeutischen Agens an das Lebewesen.

8. Antikörper-Wirkstoff-Konjugat (ADC) zur Verwendung nach Anspruch 7 und standardmäßige Krebstherapie zur Verwendung nach Anspruch 7, wobei das therapeutische Agens ausgewählt ist aus der Gruppe bestehend aus einem Toxin, einem Enzym, einem Hormon, einem zweiten Antikörper, einem zweiten Antikörperfragment, einem Immunkonjugat, einem Radionuklid, einem Antisense-Oligonukleotid und einer RNAi.

9. Antikörper-Wirkstoff-Konjugat (ADC) zur Verwendung nach Anspruch 8 und standardmäßige Krebstherapie zur Verwendung nach Anspruch 8, wobei das Radionuklid an einen Antikörper oder ein Antigen-bindendes Antikörperfragment gebunden und ausgewählt ist aus der Gruppe bestehend aus ¹¹¹In, ¹¹¹At, ¹⁷⁷Lu, ²¹¹Bi, ²¹²Bi, ²¹³Bi, ²¹¹At, ⁶²Cu, ⁶⁷Cu, ⁹⁰Y, ¹²⁵I, ¹³¹I, ¹³³I, ³²P, ³³P, ⁴⁷Sc, ¹¹¹Ag, ⁶⁷Ga, ¹⁵³Sm, ¹⁶¹Tb, ¹⁵²Dy, ¹⁶⁶Dy, ¹⁶¹Ho, ¹⁶⁶Ho, ¹⁸⁶Re, ¹⁸⁸Re, ¹⁸⁹Re, ²¹¹Pb, ²¹²Pb, ²²³Ra, ²²⁵Ac, ⁷⁷As, ⁸⁹Sr, ⁹⁹Mo, ¹⁰⁵Rh, ¹⁴⁹Pm , ¹⁶⁹Er, ¹⁹⁴Ir , ⁵⁸Co, ^{80m}Br, ^{99m}Tc, ^{103m}Rh, ¹⁰⁹Pt, ¹¹⁹Sb, ^{189m}OS, ¹⁹²Ir ²¹⁹Rn, ²¹⁵Po, ²²¹Fr, ²⁵⁵Fm, ¹¹C, ¹³N, ¹⁵O, ⁷⁵Br, ¹⁹⁸Au, ¹⁹⁹Au, ²²⁴Ac, ⁷⁷Br, ^{113m}In, ⁹⁵Ru, ⁹⁷Ru, ¹⁰³Ru, ¹⁰⁵Ru, ¹⁰⁷Hg, ²⁰³Hg, ¹²¹mTe, ^{122m}Te, ^{125m}Te, ²²⁷Th, ¹⁶⁵Tm, ¹⁶⁷Tm, ¹⁶⁸Tm, ¹⁹⁷Pt, ¹⁰⁹Pd, ¹⁴²Pr, ¹⁴³Pr, ¹⁶¹Tb, ⁵⁷Co, ⁵⁸Co, ⁵¹Cr, ⁵⁹Fe, ⁷⁵Se, ²⁰¹T1, ⁷⁶Br und ¹⁶⁹Yb.

10. Antikörper-Wirkstoff-Konjugat (ADC) zur Verwendung nach Anspruch 1 und standardmäßige Krebstherapie zur Verwendung nach Anspruch 2, wobei der Krebs ausgewählt ist aus der Gruppe bestehend aus Kolonkrebs, Magenkrebs, Speiseröhrenkrebs, medullärem Schilddrüsenkrebs, Nierenkarzinom, Brustkrebs, Lungenkrebs, Pankreaskarzinom, Harnblasenkarzinom, Ovarialkarzinom, Uteruskarzinom, Zervixkarzinom, Hodenkrebs, Prostatakrebs, Leberkrebs, Hautkrebs, Knochenkrebs, Hirntumor, Rektumkarzinom und Melanom.

11. Antikörper-Wirkstoff-Konjugat (ADC) zur Verwendung nach Anspruch 1 und standardmäßige Krebstherapie zur Verwendung nach Anspruch 2, wobei der Krebs ausgewählt ist aus der Gruppe bestehend aus dreifach-negativem Brustkrebs (TNBC), metastatischem Kolonkrebs, metastatischem nichtkleinzelligen Lungenkarzinom (NSCLC), metastatischem Pankreaskarzinom, metastatischem Grawitz-Tumor, metastatischem Magenkrebs, metastatischem Prostatkrebs und metastatischem kleinzelligen Lungenkarzinom.

12. Antikörper-Wirkstoff-Konjugat (ADC) zur Verwendung nach Anspruch 1 und standardmäßige Krebstherapie zur Verwendung nach Anspruch 2, wobei der Krebs refraktär gegenüber anderen Therapien ist, aber auf Therapie mit neoadjuvantem ADC anspricht.

## Revendications

1. Conjugué anticorps-médicament (ADC) à utiliser dans un procédé pour le traitement néoadjuvant du cancer comprenant :
a) l'administration du conjugué anticorps-médicament (ADC) comprenant un fragment d'anticorps conjugué à un médicament, à un patient atteint d'un cancer, dans lequel le fragment d'anticorps est un anticorps anti-Trop-2 hRS7 et le médicament est SN-38 ; et
b) le traitement du patient avec une thérapie anti-cancer classique sélectionnée parmi le groupe composé de la chirurgie et de la radiothérapie, et
dans lequel l'ADC est administré avant la thérapie anti-cancer classique.

2. Thérapie anti-cancer classique à utiliser dans un procédé pour le traitement néoadjuvant du cancer comprenant :
a) l'administration du conjugué anticorps-médicament (ADC) comprenant un fragment d'anticorps conjugué à un médicament, à un patient atteint d'un cancer, dans lequel le fragment d'anticorps est un anticorps anti-Trop-2 hRS7 et le médicament est SN-38 ; et
b) le traitement du patient avec la thérapie anti-cancer classique, dans lequel la thérapie anti-cancer classique est sélectionnée parmi le groupe composé de la chirurgie et de la radiothérapie, et
dans lequel l'ADC est administré avant la thérapie anti-cancer classique.

3. Conjugué anticorps-médicament (ADC) à utiliser selon la revendication 1 et thérapie anti-cancer classique à utiliser selon la revendication 2, dans lesquels le fragment d'anticorps comprend des régions constantes de l'homme sélectionnées parmi le groupe composé d'IgG1, d'IgG2, d'IgG3 et d'IgG4.

4. Conjugué anticorps-médicament (ADC) à utiliser selon la revendication 1 et thérapie anti-cancer classique à utiliser selon la revendication 2, dans lesquels le fragment d'anticorps a un allotype sélectionné parmi le groupe composé de non-Glml (nG1m1), G1m3 et Km3.

5. Conjugué anticorps-médicament (ADC) à utiliser selon la revendication 1 et thérapie anti-cancer classique à utiliser selon la revendication 2, dans lesquels l'ADC comprend un lieur qui fixe le médicament sur le fragment d'anticorps.

6. Conjugué anticorps-médicament (ADC) à utiliser selon la revendication 5 et thérapie anti-cancer classique à utiliser selon la revendication 5, dans lesquels le médicament est SN-38 et le lieur est CL2A.

7. Conjugué anticorps-médicament (ADC) à utiliser selon la revendication 1 et thérapie anti-cancer classique à utiliser selon la revendication 2, comprenant en outre l'administration d'au moins un agent thérapeutique audit patient.

8. Conjugué anticorps-médicament (ADC) à utiliser selon la revendication 7 et thérapie anti-cancer classique à utiliser selon la revendication 7, dans lesquels l'agent thérapeutique est sélectionné parmi le groupe composé d'une toxine, d'un enzyme, d'une hormone, d'un second anticorps, d'un second fragment d'anticorps, d'un immunoconjugué, d'un radionucléide, d'un oligunucléotide antisens et d'un ARNi.

9. Conjugué anticorps-médicament (ADC) à utiliser selon la revendication 8 et thérapie anti-cancer classique à utiliser selon la revendication 8, dans lesquels le radionucléide est fixé sur un anticorps ou un fragment d'anticorps de liaison à l'antigène et est sélectionné parmi le groupe composé de ¹¹¹In, ¹¹¹At, ¹⁷⁷Lu, ²¹¹Bi, ²¹²Bi, ²¹³Bi, ²¹¹At, ⁶²Cu, ⁶⁷Cu, ⁹⁰Y, ¹²⁵I, ¹³¹I, ¹³³I, ³²P, ³³P, ⁴⁷Sc, ¹¹¹Ag, ⁶⁷Ga, ¹⁵³Sm, ¹⁶¹Tb, ¹⁵²Dy, ¹⁶⁶Dy, ¹⁶¹Ho, ¹⁶⁶Ho, ¹⁸⁶Re, ¹⁸⁸Re, ¹⁸⁹Re, ²¹¹Pb, ²¹²Pb, ²²³Ra, ²²⁵Ac, ⁷⁷As, ⁸⁹Sr, ⁹⁹Mo, ¹⁰⁵Rh, ¹⁴⁹Pm, ¹⁶⁹Er, ¹⁹⁴Ir, ⁵⁸Co, ^{80m}Br, ^{99m}Tc, ^{103m}Rh, ¹⁰⁹Pt, ¹¹⁹Sb, ^{189m}Os, ¹⁹²Ir, ²¹⁹Rn, ²¹⁵Po, ²²¹Fr, ²⁵⁵Fm, ¹¹C, ¹³N, ¹⁵O, ⁷⁵Br, ¹⁹⁸Au, ¹⁹⁹Au, ²²⁴Ac, ⁷⁷Br, ^{113m}In, ⁹⁵Ru, ⁹⁷Ru, ¹⁰³Ru, ¹⁰⁵Ru, ¹⁰⁷Hg, ²⁰³Hg, ¹²¹mTe, ^{122m}Te, ^{125m}Te, ²²⁷Th, ¹⁶⁵Tm, ¹⁶⁷Tm, ¹⁶⁸Tm, ¹⁹⁷Pt, ¹⁰⁹Pd, ¹⁴²Pr, ¹⁴³Pr, ¹⁶¹Tb, ⁵⁷Co, ⁵⁸Co, ⁵¹Cr, ⁵⁹Fe, ⁷⁵Se, ²⁰¹T1, ⁷⁶Br et ¹⁶⁹Yb.

10. Conjugué anticorps-médicament (ADC) à utiliser selon la revendication 1 et thérapie anti-cancer classique à utiliser selon la revendication 2, dans lesquels le cancer est sélectionné parmi le groupe composé du cancer du côlon, du cancer de l'estomac, du cancer de l'œsophage, du cancer médullaire de la thyroïde, du cancer du rein, du cancer du sein, du cancer du poumon, du cancer du pancréas, du cancer de la vessie, du cancer de l'ovaire, du cancer de l'utérus, du cancer de l'utérus, du cancer du col de l'utérus, du cancer des testicules, du cancer de la prostate, du cancer du foie, du cancer de la peau, du cancer des os, du cancer du cerveau, du cancer du rectum et du mélanome.

11. Conjugué anticorps-médicament (ADC) à utiliser selon la revendication 1 et thérapie anti-cancer classique à utiliser selon la revendication 2, dans lesquels le cancer est sélectionné parmi le groupe composé du cancer du sein triple négatif (TNBC), du cancer métastatique du côlon, du cancer du poumon métastatique non à petites cellules (NSCLC), du cancer métastatique du pancréas, du carcinome métastatique des cellules rénales, du cancer métastatique de l'estomac, du cancer métastatique de la prostate et du cancer du poumon métastatique à petites cellules.

12. Conjugué anticorps-médicament (ADC) à utiliser selon la revendication 1 et thérapie anti-cancer classique à utiliser selon la revendication 2, dans lesquels le cancer est réfractaire à d'autres thérapies mais répond à une thérapie avec un ADC néoadjuvant.
